# EUROPEAN PATENT SPECIFICATION

(11) **EP 1 646 620 B1**
(45) Date of publication and mention of the grant of the patent: **15.08.2007**
(21) Application number: 04763339.1
(22) Date of filing: 16.07.2004
(51) Int. Cl.: C07D 401/04, C07D 295/14, C07D 413/10, C07D 413/14, C07D 403/02, A61K 31/4545, A61P 25/00

(54) **SUBSTITUTED PIPERIDINES AS HISTAMINE H3 RECEPTOR LIGANDS**
SUBSTITUIERTE PIPERIDINE ALS HISTAMIN-H3-REZEPTORLIGANDEN
PIPERIDINES SUBSTITUEES UTILISEES EN TANT QUE LIGANDS DU RECEPTEUR H3 DE L'HISTAMINE

(30) Priority: 18.07.2003 GB 0316893; 19.05.2004 GB 0411167
(43) Date of publication of application: 19.04.2006
(73) Proprietor: GLAXO GROUP LIMITED, Greenford, Middlesex UB6 0NN (GB)
(72) Inventor: BAILEY, J. M.; c/o GlaxoSmithKline, Harlow Essex CM19 5AW (GB); BRUTON, Gordon; c/o GlaxoSmithKline, Harlow Essex CM19 5AW (GB); HUXLEY, Anthony; c/o GlaxoSmithKline, Harlow Essex CM19 5AW (GB); MILNER, Peter Henry; c/o GlaxoSmithKline, Harlow Essex CM19 5AW (GB); ORLEK, B. S.; c/o GlaxoSmithKline, Harlow Essex CM19 5AW (GB)
(74) Representative: Goddard, Carolyn Janice
(86) International application number: PCT/EP2004/008061
(87) International publication number: WO 2005/014571

(56) References cited:
- WO-A-02/32893
- WO-A-02/072093
- WO-A-03/031432

## Description

The present invention relates to novel piperidine ether derivatives having pharmacological activity, processes for their preparation, to compositions containing them and to their use in the treatment of neurological and psychiatric disorders.

WO 03/24450 (Eisai Co. Ltd) describes a series of heterocyclic cholinesterase inhibitors which are claimed to be useful in the treatment of prion diseases. WO 03/24456 (Eisai Co. Ltd) describes a series of heterocyclic cholinesterase inhibitors which are claimed to be useful in the treatment and prevention of migraine. WO 03/84948 (Eisai Co. Ltd) describe a series of nitrogenous heterocyclic compounds as sodium channel blockers which are claimed to be useful in the treatment of pain. WO 99/37304 (Rhone-Poulenc Rorer Pharmaceuticals Inc) and WO 01/07436 (Aventis Pharmaceuticals Products Inc) both describe a series of substituted oxoazaheterocyclyl Factor Xa inhibitors. WO 03/103669 and WO 03/088967 (both Schering Corp) describe a series of piperidinyl benzimidazolone compounds as histamine H3 antagonists. WO 02/32893 and WO 02/72570 (both Schering Corp) describe a series of non-imidazole compounds as histamine H3 antagonists. WO 99/24422 (Neurosearch AS) describe a series of aza ring ether derivatives as nicotinic acetylcholine receptor modulators which are claimed to be useful in the treatment of pain, inflammatory disease, disease caused by smooth muscle contractions or substance abuse. WO 97/38665 (Merck & Co Inc) describe a series of piperidine derivatives as famesyl Ras-protein transferase inhibitors which are claimed to be useful in the treatment of cancer, vascularisation, hepatitis, restenosis and kidney disease. WO 03/031432 discloses substituted piperidines useful in the treatment of disorders related to histamine H3 receptor.

The histamine H3 receptor is predominantly expressed in the mammalian central nervous system (CNS), with minimal expression in peripheral tissues except on some sympathetic nerves (Leurs et al., (1998), Trends Pharmacol. Sci. 19, 177-183). Activation of H3 receptors by selective agonists or histamine results in the inhibition of neurotransmitter release from a variety of different nerve populations, including histaminergic and cholinergic neurons (Schlicker et al., (1994), Fundam. Clin. Pharmacol. 8, 128-137). Additionally, *in vitro* and *in vivo* studies have shown that H3 antagonists can facilitate neurotransmitter release in brain areas such as the cerebral cortex and hippocampus, relevant to cognition (Onodera et al., (1998), In: The Histamine H3 receptor, ed Leurs and Timmerman, pp255-267, Elsevier Science B.V.). Moreover, a number of reports in the literature have demonstrated the cognitive enhancing properties of H3 antagonists (e.g. thioperamide, clobenpropit, ciproxifan and GT-2331) in rodent models including the five choice task, object recognition, elevated plus maze, acquisition of novel task and passive avoidance (Giovanni et al., (1999), Behav. Brain Res. 104, 147-155). These data suggest that novel H3 antagonists such as the current series could be useful for the treatment of cognitive impairments in diseases such as Alzheimer's disease and related neurodegenerative disorders.

The present invention provides, in a first aspect, a compound of formula (I) or a pharmaceutically acceptable salt thereof: 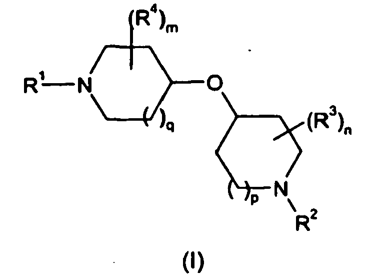 wherein:
R¹ represents aryl, heteroaryl,-aryl-X-aryl, -aryl-X-heteroaryl, -aryl-X-heterocyclyl,-heteroaryl-X-heteroaryl, -heteroaryl-X-aryl or -heteroaryl-X-heterocyclyl;
wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -COR¹⁵, -COOR¹⁵, NR¹⁵R¹⁶, -CONR¹⁵R¹⁶, -NR¹⁵COR¹⁶, -NR¹⁵SO₂R¹⁶ or -SO₂NR¹⁵R¹⁶, wherein R¹⁵ and R¹⁶ independently represent hydrogen, C₁₋₆ alkyl, haloC₁₋₆ alkyl, polyhaloC₁₋₆ alkyl or C₃₋₆ cycloalkyl or together form a heterocyclic ring;
X represents a bond, O, CO, SO₂, OCH₂ or CH₂O;
R² represents C₃₋₈ alkyl, C₃₋₆ alkenyl, C₃₋₆ alkynyl, C₃₋₆ cycloalkyl, C₅₋₆ cycloalkenyl or -C₁₋₄alkyl-C₃₋₆ cycloalkyl;
wherein said C₃₋₆ cycloalkyl groups of R² may be optionally substituted by one or more (e.g. 1, 2 or 3) substituents which may be the same or different, and which are selected from the group consisting of halogen, C₁₋₄ alkyl or trifluoromethyl groups;
each R³ and R⁴ group independently represents C₁₋₄ alkyl;
m and n independently represents 0, 1 or 2;
p and q independently represents 1 or 2;
or a pharmaceutically acceptable salt thereof.

Specific compounds of formula (I) which may be mentioned are those wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, arylsulfonyl, arylsulfonyloxy, aryloxy, arylsulfonamido, arylcarboxamido, aroyl, or a group NR¹⁵R¹⁶, -CONR¹⁵R¹⁶, -NR¹⁵COR¹⁶, -NR¹⁵SO₂R¹⁶ or -SO₂NR¹⁵R¹⁶, wherein R¹⁵ and R¹⁶ independently represent hydrogen, C₁₋₆ alkyl or together form a heterocyclic ring.

Specific compounds of formula (I) which may be mentioned are those wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group NR¹⁵R¹⁶, -CONR¹⁵R¹⁶,-NR¹⁵COR¹⁶, -NR¹⁵SO₂R¹⁶ or -SO₂NR¹⁵R¹⁶, wherein R¹⁵ and R¹⁶ independently represent hydrogen, C₁₋₆ alkyl or C₃₋₆ cycloalkyl or together form a heterocyclic ring.

In one particular aspect of the present invention, there is provided a compound of formula (I) as defined above wherein R¹ represents heteroaryl,-aryl-X-aryl, -aryl-X-heteroaryl, -aryl-X-heterocyclyl, -heteroaryl-X-heteroaryl, -heteroaryl-X-aryl or-heteroaryl-X-heterocyclyl.

Alkyl groups, whether alone or as part of another group, may be straight chain or branched and the groups alkoxy and alkanoyl shall be interpreted similarly. The term 'halogen' is used herein to describe, unless otherwise stated, a group selected from fluorine, chlorine, bromine or iodine and the term 'polyhalo' is used herein to refer to a moiety containing more than one (e.g. 2-5) of said halogen atoms.

The term "aryl" includes single and fused rings wherein at least one ring is aromatic, for example, phenyl, naphthyl and tetrahydronaphthalenyl.

The term "heterocyclyl" is intended to mean a 4-7 membered monocyclic saturated or partially unsaturated aliphatic ring or a 4-7 membered saturated or partially unsaturated aliphatic ring fused to a benzene ring containing 1 to 3 heteroatoms selected from oxygen, nitrogen or sulphur. Suitable examples of such monocyclic rings include pyrrolidinyl, azetidinyl, piperidinyl, piperazinyl, morpholinyl, tetrahydrofuranyl, tetrahydropyranyl, diazepanyl and azepanyl. Suitable examples of benzofused heterocyclic rings include indolinyl, isoindolinyl, 2,3,4,5-tetrahydro-1*H*-3-benzazepine or tetrahydroisoquinolinyl.

The term "heteroaryl" is intended to mean a 5-6 membered monocyclic aromatic or a fused 8-10 membered bicyclic aromatic ring containing 1 to 3 heteroatoms selected from oxygen, nitrogen and sulphur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include benzofused aromatic rings such as quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like.

Preferably, R¹ represents
- aryl (e.g. phenyl) optionally substituted by a cyano, -CONR¹⁵R¹⁶ (e.g.-CON(H)(Me), -CONMe₂ or -CON(H)(chloropropyl)), -COR¹⁵ (e.g. -COMe, -COEt, -CO-cyclopropyl or -CO-cyclobutyl), halogen (e.g. fluorine) or -NR¹⁵COR¹⁶ (e.g. -NHCOMe) group;
- heteroaryl (e.g. pyrid-2-yl, pyrid-3-yl, pyrid-4-yl, pyrazin-2-yl, pyridazin-3-yl, pyrimidin-5-yl or quinolin-6-yl) optionally substituted by a cyano, C₁₋₆ alkyl (e.g. methyl), polyhaloC₁₋₆ alkyl (e.g. -CF₃), -CONR¹⁵R¹⁶ (e.g. -CON(H)(Me), -CONMe₂, -CON(H)(Et), -CON(H)(Pr), -CON(H)(chloropropyl), -CON(H)(i-Pr), -CON(H)(cyclobutyl) or-CON(H)(cyclopentyl), -COR¹⁵ (e.g. -COMe) or -COOR¹⁵ (e.g. -COOt-Bu) group;
- aryl-X-heterocyclyl (e.g. -phenyl-CO-morpholinyl, -phenyl-CO-piperidinyl or-phenyl-CO-pyrrolidinyl);
- aryl-X-heteroaryl (e.g. -phenyl-oxazolyl, -phenyl-isoxazolyl or -phenyl-oxadiazolyl) optionally substituted by a halogen (e.g. fluorine), C₁₋₆ alkyl (e.g. methyl) or aryl (e.g. phenyl) group; or
- heteroaryl-X-heterocyclyl (e.g. -pyrid-2-yl-CO-pyrrolidinyl, -pyrid-2-yl-CO-piperidinyl, -pyrid-2-yl-CO-morpholinyl, -pyrid-3-yl-CO-pyrrolidinyl, -pyrid-3-yl-CO-piperidinyl or -pyrid-3-yl-CO-morpholinyl).

More preferably, R¹ represents
- aryl (e.g. phenyl) optionally substituted by a cyano, -CONR¹⁵R¹⁶ (e.g.-CON(H)(Me), -COR¹⁵ (e.g. -COMe, -COEt, -CO-cyclopropyl or -CO-cyclobutyl), halogen (e.g. fluorine) or -NR¹⁵COR¹⁶ (e.g. -NHCOMe) group;
- heteroaryl (e.g. pyrid-2-yl, pyrid-3-yl, pyrazin-2-yl, pyridazin-3-yl, pyrimidin-5-yl or quinolin-6-yl) optionally substituted by a cyano (e.g. 5-cyano-2-pyridyl or 6-cyano-3-pyridyl), C₁₋₆ alkyl (e.g. methyl), polyhaloC₁₋₆ alkyl (e.g. -CF₃), -CONR¹⁵R¹⁶ (e.g.-CON(H)(Me), -CONMe₂, -CON(H)(Et), -CON(H)(Pr), -CON(H)(i-Pr) or-CON(H)(cyclobutyl)) or -COR¹⁵ (e.g. -COMe) group;
- aryl-X-heterocyclyl (e.g. -phenyl-CO-morpholinyl);
- aryl-X-heteroaryl (e.g. -phenyl-oxazolyl, -phenyl-isoxazol-5-yl or -phenyl-1,2,4-oxadiazol-5-yl) optionally substituted by a halogen (e.g. fluorine), C₁₋₆ alkyl (e.g. methyl) or aryl (e.g. phenyl) group; or
- heteroaryl-X-heterocyclyl (e.g. -pyrid-3-yl-CO-piperidinyl or-pyrid-3-yl-CO-morpholinyl).

Most preferably, R¹ represents pyrid-3-yl optionally substituted by a -CONR¹⁵R¹⁶ group (e.g. 6-CON(H)(Me) or 6-CON(H)(Et)), -phenyl-1,2,4-oxadiazol-5-yl optionally substituted by a C₁₋₆ alkyl (e.g. methyl) group (e.g. 3-methyl-1,2,4-oxadiazol-5-yl), phenyl optionally substituted by a -COR¹⁵ (e.g. 4-COMe) group, pyridazin-3-yl optionally substituted by a polyhaloC₁₋₆ alkyl (e.g. 6-CF₃) group, pyrazin-2-yl optionally substituted by a polyhaloC₁₋₆ alkyl (e.g. 5-CF₃) or pyrimidin-5-yl optionally substituted by a polyhaloC₁₋₆ alkyl (e.g. 2-CF₃) group.

Especially preferably, R¹ represents pyrid-3-yl optionally substituted by a -CONR¹⁵R¹⁶ group (e.g. 6-CON(H)(Me) or 6-CON(H)(Et)), -phenyl-1,2,4-oxadiazol-5-yl optionally substituted by a C₁₋₆ alkyl (e.g. methyl) group (e.g. 3-methyl-1,2,4-oxadiazol-5-yl), phenyl optionally substituted by a -COR¹⁵ (e.g. 4-COMe) group, pyridazin-3-yl optionally substituted by a polyhaloC₁₋₆ alkyl (e.g. 6-CF₃) group or pyrimidin-5-yl optionally substituted by a polyhaloC₁₋₆ alkyl (e.g. 2-CF₃) group.

Preferably, m and n represent 0.

Preferably, p and q represent 1.

Preferably, R² represents C₃₋₈ alkyl (e.g. 1-methylpropyl or isopropyl), C₃₋₆ cycloalkyl (e.g. cyclobutyl) or -C₁₋₄alkyl-C₃₋₆ cycloalkyl (e.g. -CH₂-cyclopropyl), more preferably R² represents C₃₋₈ alkyl (e.g. 1-methylpropyl or isopropyl) or C₃₋₆ cycloalkyl (e.g. cyclobutyl), especially isopropyl or cyclobutyl.

Preferred compounds according to the invention include examples E1-E120 as shown below, or a pharmaceutically acceptable salt thereof.

Most preferred compounds according to the invention include:
1-(1-Methylethyl)-4-({1-[4-(3-methyl-1,2,4-oxadiazol-5-yl)phenyl]-4-piperidinyl}oxy)piperidine (E17);
5-{4-[(1-Cyclobutyl-4-piperidinyl)oxy]-1-piperidinyl}-*N*-methyl-2-pyridinecarboxamide (E38);
1-(4-{4-[(1-Cyclobutyl-4-piperidinyl)oxy]-1-piperidinyl}phenyl)ethanone (E48);
3-{4-[(1-Cyclobutyl-4-piperidinyl)oxy]-1-piperidinyl}-6-(trifluoromethyl)pyridazine (E82); or
5-{4-[(1-Cyclobutyl-4-piperidinyl)oxy]-1-piperidinyl}-2-(trifluoromethyl)pyrimidine (E88) or a pharmaceutically acceptable salt thereof.

A pharmaceutically acceptable acid addition salt can be formed by reaction of a compound of formula (I) with a suitable inorganic or organic acid (such as hydrobromic, hydrochloric, sulfuric, nitric, phosphoric, succinic, maleic, formic, acetic, propionic, fumaric, citric, tartaric, lactic, benzoic, salicylic, glutamaic, aspartic, p-toluenesulfonic, benzenesulfonic, methanesulfonic, ethanesulfonic, naphthalenesulfonic such as 2-naphthalenesulfonic, or hexanoic acid), optionally in a suitable solvent such as an organic solvent, to give the salt which is usually isolated for example by crystallisation and filtration. A pharmaceutically acceptable acid addition salt of a compound of formula (I) can comprise or be for example a hydrobromide, hydrochloride, sulfate, nitrate, phosphate, succinate, maleate, formate, acetate, propionate, fumarate, citrate, tartrate, lactate, benzoate, salicylate, glutamate, aspartate, p-toluenesulfonate, benzenesulfonate, methanesulfonate, ethanesulfonate, naphthalenesulfonate (e.g. 2-naphthalenesulfonate) or hexanoate salt.

Certain compounds of formula (I) are capable of existing in stereoisomeric forms. It will be understood that the invention encompasses all geometric and optical isomers of these compounds and the mixtures thereof including racemates. Tautomers also form an aspect of the invention.

The present invention also provides a process for the preparation of a compound of formula (1) or a pharmaceutically acceptable salt thereof, which process comprises:
(a) reacting a compound of formula (II) 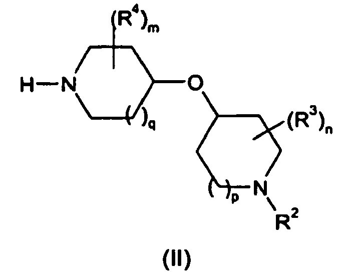 wherein R², R³, R⁴, m, n, p and q are as defined above, with a compound of formula R¹-L¹, wherein R¹ is as defined above and L¹ represents a suitable leaving group, such as a halogen atom (e.g. fluorine, chlorine, bromine or iodine); or
(b) reacting a compound of formula (III) 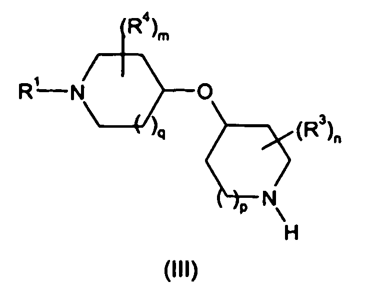 wherein R¹, R³, R⁴, m, n, p and q are as defined above, with a compound of formula R²-L² where R² is as defined above and L² represents a suitable leaving group, such as a halogen atom or a sulfonate such as methanesulfonate; or
(c) reacting a compound of formula (III) as defined above with a compound of formula H-R^{2'}=O under reductive conditions, wherein R^{2'} is as defined above for R² or a group convertible thereto; or
(d) preparing a compound of formula (I) wherein p represents 1 which comprises reduction of a compound of formula (IV) 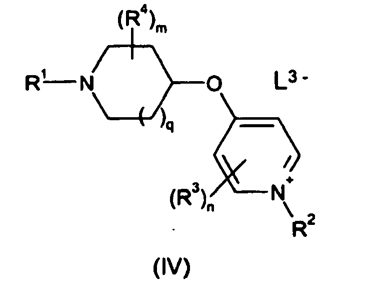 wherein R¹, R², R³, R⁴, m, n and q are as defined above and L³⁻ represents a suitable counter ion such as a halogen atom; or
(e) deprotecting a compound of formula (I) or converting groups which are protected; and optionally thereafter
(f) interconversion to other compounds of formula (I).

Process (a) typically comprises the use of a suitable base, such as potassium carbonate in a suitable solvent such as dimethylsulfoxide, 1-methyl-2-pyrrolidinone or N,N-dimethylformamide at elevated temperature. Alternatively, process (a) may be carried out with a suitable catalyst system such as tris(dibenzylideneacetone)dipalladium(0) and 2-dicyclohexylphosphino-2'-(N,N-dimethylamino)biphenyl; or bis(dibenzylideneacetone)palladium and 2-dicyclohexylphosphino-2'-(N,N-dimethylamino)biphenyl; or tris(dibenzylideneacetone)dipalladium(0) and xantphos; or acetato(2'-di-t-butylphosphin-1,1'-biphenyl-2-yl)palladium (II); or palladium(II) acetate and BINAP, or palladium(II) acetate and 2,8,9-triisobutyl-2,5,8,9-tetraaza-1-phosphabicyclo[3.3.3]undecane, in the presence of a suitable base such as sodium t-butoxide, caesium carbonate or potassium phosphate in a solvent such as o-xylene, dioxane or toluene, under an inert atmosphere, optionally at an elevated temperature.

Process (b) typically comprises the use of a suitable base such as potassium carbonate in a solvent such as N,N-dimethylformamide or acetonitrile.

Process (c) typically comprises the use of standard reductive amination conditions with a reducing agent such as sodium triacetoxy borohydride in a suitable solvent such as dichloromethane or hydrogenation in the presence of a suitable catalyst such as palladium.

Process (d) is typically carried out under suitable reductive conditions e.g. using lithium borohydride in combination with ammonium formate and a palladium catalyst in a solvent such as methanol. The process may also be carried out in a stepwise manner by reduction with sodium borohydride in a suitable solvent such as methanol followed by transfer hydrogenation, e.g. using palladium in the presence of ammonium formate in a solvent such as methanol. Alternatively, the reduction may be carried out by hydrogenation over a catalyst such as platinum oxide in a solvent such as ethanol optionally at elevated temperature and pressure.

In process (e), examples of protecting groups and the means for their removal can be found in T. W. Greene 'Protective Groups in Organic Synthesis' (J. Wiley and Sons, 1991). Suitable amine protecting groups include sulfonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (f) may be performed using conventional interconversion procedures such as epimerisation, oxidation, reduction, alkylation, nucleophilic or electrophilic aromatic substitution, ester hydrolysis or amide bond formation.

Compounds of formula (II) wherein p represents 1 may be prepared in accordance with the following procedure: 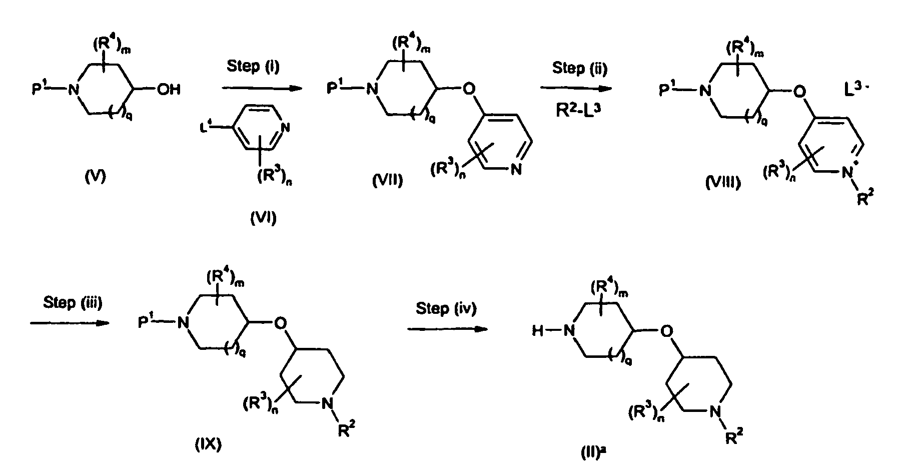 wherein R², R³, R⁴ , m, n and q are as defined above, L³ represents a suitable leaving group and L³⁻ represents the resultant counter ion, L⁴ represents a suitable leaving group such as a halogen atom, and P¹ represents a suitable protecting group such as t-butoxycarbonyl.

When L⁴ represents a suitable leaving group such as a halogen atom (e.g. chlorine), step (i) typically comprises the use of a suitable base such as potassium carbonate or sodium hydride in a solvent such as dimethylsulfoxide optionally at elevated temperature.

When L³ represents a suitable leaving group such as a halogen atom (e.g. bromine, iodine), step (ii) is typically carried out in a suitable solvent such as dichloromethane or acetonitrile optionally at elevated temperature.

Step (iii) is carried out under reductive conditions e.g. using lithium borohydride in combination with ammonium formate and a palladium catalyst in a solvent such as methanol. Step (iii) may also be carried out in a stepwise manner by reduction with sodium borohydride in a suitable solvent such as methanol followed by transfer hydrogenation, e.g. using palladium in the presence of ammonium formate in a solvent such as methanol. Alternatively, the reduction may be carried out by hydrogenation over a catalyst such as platinum oxide in a solvent such as ethanol optionally at elevated temperature and pressure.

Step (iv) is a deprotection reaction where the conditions are dependent upon the nature of the group P¹. Removal of a P¹ tert-butoxycarbonyl group can be performed under acidic conditions, e.g. using trifluoroacetic acid in a suitable solvent such as ethyl acetate, or HCl in dioxane.

Compounds of formula (II) wherein p represents 1 may also be prepared in accordance with the following procedure: 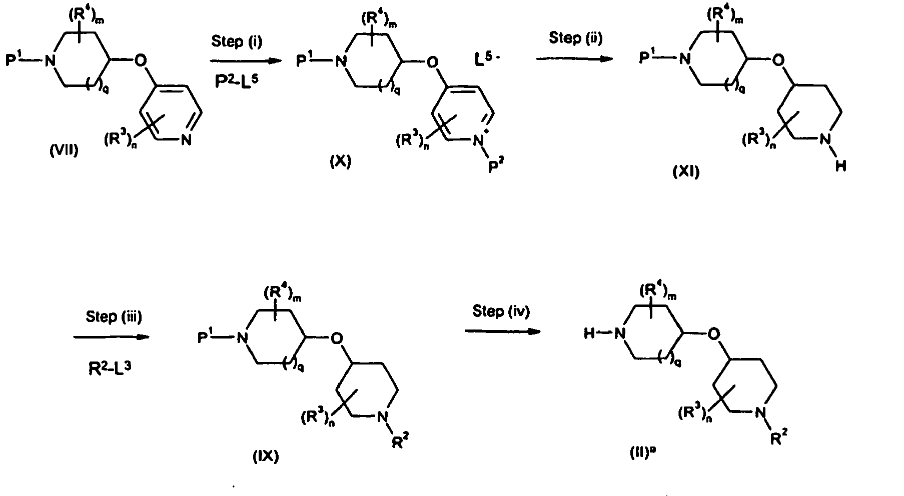 wherein R², R³, R⁴, m, n, q, P¹ and L³ are as defined above, L⁵ represents a suitable leaving group such as a halogen atom and P² represents a suitable protecting group such as benzyl or p-methoxybenzyl.

When L⁵ represents a suitable leaving group such as a halogen atom (e.g. bromine), step (i) is typically carried out in a solvent such as dichloromethane optionally at elevated temperature.

Step (ii) is carried out under suitable reductive conditions. The reduction may be carried out in a stepwise manner by reduction with sodium borohydride in a suitable solvent such as methanol followed by hydrogenation in the presence of a palladium catalyst and then transfer hydrogenation, e.g. using palladium in the presence of ammonium formate in a solvent such as methanol. The reduction may also be carried out with sodium borohydride in a suitable solvent such as methanol followed by transfer hydrogenation, e.g. using palladium in the presence of ammonium formate in a solvent such as methanol. The reduction may also be carried out using lithium borohydride in combination with ammonium formate and a palladium catalyst in a solvent such as methanol, followed by hydrogenation in the presence of a suitable catalyst such as palladium.

When L³ represents a suitable leaving group such as a halogen atom (e.g. bromine, iodine), step (iii) typically involves reaction with R²-L³ in a suitable solvent such as dichloromethane or acetonitrile optionally at elevated temperature. Alternatively, the reaction may be carried out with a compound R²=O using reductive amination conditions such as sodium triacetoxyborohydride in a suitable solvent such as dichloromethane, or hydrogenation in the presence of a suitable catalyst such as palladium.

Step (iv) is a deprotection reaction where the conditions are dependent upon the nature of the group P¹. Removal of a P¹ tert-butoxycarbonyl group can be performed under acidic conditions, e.g. using trifluoroacetic acid in a suitable solvent such as ethyl acetate, or HCl in dioxane.

Alternatively, a compound of formula (VII) may be reduced directly to give a compound of formula (XI) by hydrogenation over a catalyst such as platinum oxide in a solvent such as ethanol optionally in the presence of an acid such as acetic acid and optionally at elevated temperature and pressure. The reduction may also be carried out in the presence of a compound of formula R²=O to give a compound of formula (IX).

Compounds of formula (III) wherein q represents 1 may be prepared in accordance with the following procedure: 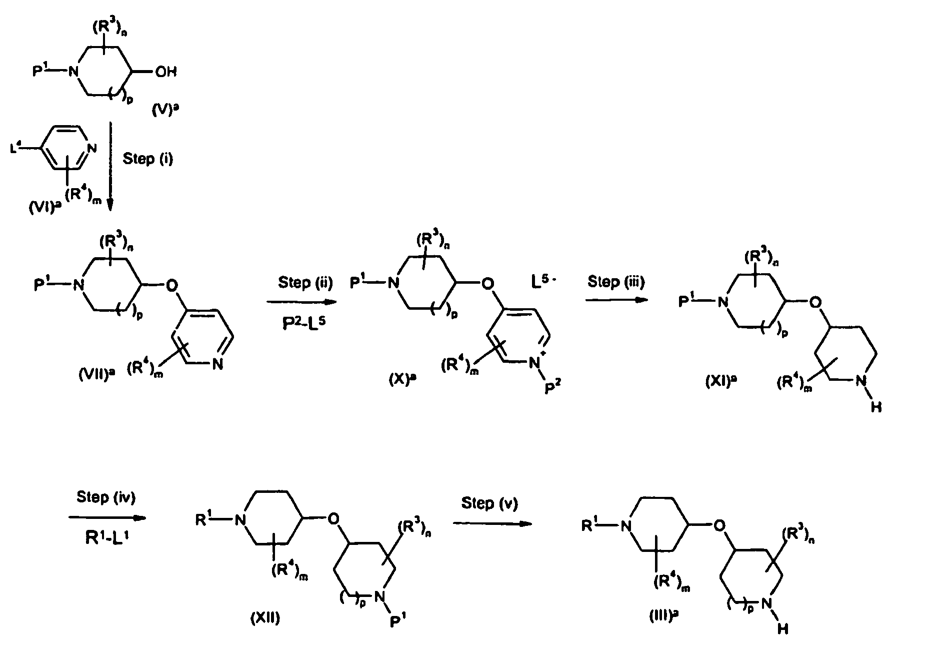 wherein R¹, R³, R⁴, m, n, p, L¹, L⁴, L⁵, P¹ and P² are as defined above.

When L⁴ represents a suitable leaving group such as a halogen atom (e.g. chlorine), step (i) typically comprises the use of a suitable base such as potassium carbonate or sodium hydride in a solvent such as dimethylsulfoxide optionally at elevated temperature.

When L⁵ represents a suitable leaving group such as a halogen atom (e.g. bromine), step (ii) is typically carried out in a solvent such as dichloromethane optionally at elevated temperature.

Step (iii) is carried out under reductive conditions e.g. using lithium borohydride in combination with ammonium formate and a palladium catalyst in a solvent such as methanol, followed by hydrogenation in the presence of a suitable catalyst such as palladium. The reduction may also be carried out in a stepwise manner by reduction with sodium borohydride in a suitable solvent such as methanol followed by hydrogenation in the presence of a palladium catalyst and then transfer hydrogenation, e.g. using palladium in the presence of ammonium formate in a solvent such as methanol. The reduction may also be carried out with sodium borohydride in a suitable solvent such as methanol followed by transfer hydrogenation, e.g. using palladium in the presence of ammonium formate in a solvent such as methanol.

Alternatively, a compound of formula (VII)^{a} may be reduced directly to give a compound of formula (XI)^{a} by hydrogenation over a catalyst such as platinum oxide in a solvent such as ethanol optionally in the presence of an acid such as acetic acid and optionally at elevated temperature and pressure.

When L¹ represents a suitable leaving group such as a halogen atom (e.g. fluorine or chlorine), step (iv) typically comprises the use of a suitable base, such as potassium carbonate in a suitable solvent such as dimethylsulfoxide or N,N-dimethylformamide at elevated temperature. Alternatively, step (iv) may be carried out with a suitable catalyst system such as tris(dibenrylideneacetone)dipalladium(0) and 2-dicyclohexylphosphino-2'-(N,N-dimethylamino)biphenyl; bis(dibenzylideneacetone)palladium and 2-dicyclohexylphosphino-2'-(N,N-dimethylamino)biphenyl; or tris(dibenzylideneacetone)dipalladium(0) and xantphos; or acetato(2'-di-t-butylphosphino-1,1'-biphenyt-2-y1)palladium II; or palladium(II) acetate and BINAP; or palladium(II) acetate and 2,8,9-triisobutyl-2,5,8,9-tetraaza-1-phosphabicyclo[3.3.3]undecane, in the presence of a suitable base such as sodium t-butoxide, caesium carbonate or potassium phosphate in a solvent such as o-xylene, dioxane or toluene, under an inert atmosphere, optionally at an elevated temperature.

Step (v) is a deprotection reaction where the conditions are dependent upon the nature of the group P¹. Removal of a P¹ tert-butoxycarbonyl group can be performed under acidic conditions e.g. using trifluoroacetic acid in a suitable solvent such as ethyl acetate, or HCl in dioxane.

Compounds of formula (IV) may be prepared in accordance with the following procedure: 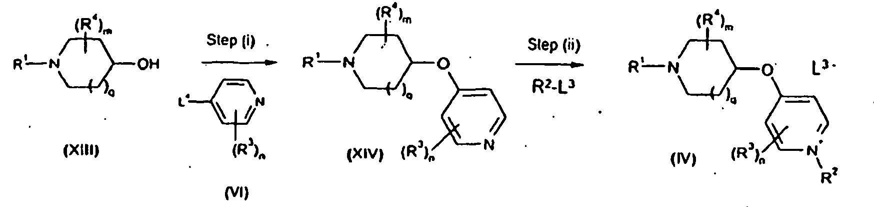 wherein R¹, R², R³, R⁴, m, n, q, L³ and L⁴ are as defined above.

When L⁴ represents a suitable leaving group such as a halogen atom (e.g. chlorine), step (i) typically comprises the use of suitable base such as potassium carbonate or sodium hydride in a solvent such as dimethylsulfoxide optionally at elevated temperature.

When L³ represents a suitable leaving group such as a halogen atom (e.g. bromine or iodine), step (ii) is typically carried out in a suitable solvent such as dichloromethane optionally at elevated temperature.

Compounds of formula (V), (V)^{a}, (VI), (VI)^{a}, (XIII) and R¹-L¹, R²-L² and R²-L³ are either known in the literature or can be prepared by analogous methods.

Compounds of formula (I) and their pharmaceutically acceptable salts have affinity for the histamine H3 receptor and are believed to be of potential use in the treatment of neurological diseases including Alzheimer's disease, dementia, age-related memory dysfunction, mild cognitive impairment, cognitive deficit, epilepsy, neuropathic pain, inflammatory pain, Parkinson's disease, multiple sclerosis, stroke and sleep disorders including narcolepsy; psychiatric disorders including schizophrenia (particularly cognitive deficit of schizophrenia), attention deficit hypereactivity disorder, depression (particularly bipolar disorder) and addiction; and other diseases including obesity, asthma, allergic rhinitis, nasal congestion, chronic obstructive pulmonary disease and gastro-intestinal disorders.

Thus the invention also provides a compound of formula (I) or a pharmaceutically acceptable salt thereof, for use as a therapeutic substance in the treatment or prophylaxis of the above disorders, in particular neurodegenerative disorders including Alzheimer's disease.

In another aspect, the invention provides the use of a compound of formula (I) or a pharmaceutically acceptable salt thereof in the manufacture of a medicament for use in the treatment of the above disorders.

When used in therapy, the compounds of formula (I) are usually formulated in a standard pharmaceutical composition. Such compositions can be prepared using standard procedures.

Thus, the present invention further provides a pharmaceutical composition for use in the treatment of the above disorders which comprises the compound of formula (I) or a pharmaceutically acceptable salt thereof and a pharmaceutically acceptable carrier.

The present invention further provides a pharmaceutical composition which comprises the compound of formula (I) or a pharmaceutically acceptable salt thereof and a pharmaceutically acceptable carrier.

Compounds of formula (I) may be used in combination with other therapeutic agents, for example histamine H1 antagonists or medicaments claimed to be useful as either disease modifying or symptomatic treatments of Alzheimer's disease. Suitable examples of such other therapeutic agents may be agents known to modify cholinergic transmission such as 5-HT₆ antagonists, M1 muscarinic agonists, M2 muscarinic antagonists or acetylcholinesterase inhibitors. When the compounds are used in combination with other therapeutic agents, the compounds may be administered either sequentially or simultaneously by any convenient route.

The invention thus provides, in a further aspect, a combination comprising a compound of formula (I) or a pharmaceutically acceptable derivative thereof together with a further therapeutic agent or agents.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three a day. Such therapy may extend for a number of weeks or months.

The following Descriptions and Examples illustrate the preparation of compounds of the invention. An Emrys^{™} Optimizer microwave reactor was employed for reactions carried out with microwave heating. Commercial scavenger resins used were obtained from Argonaut Technologies. The resins, and molarity of the solid phase reagents were used as supplied. Varian Mega BE (10g) SCX columns or Isolute Flash SCX-2 (20g) columns were used for the work-up of reactions. Crude mixtures were applied to the column, nonpolar materials were washed off with methanol, and the desired amines were eluted with ammonia in methanol.

### Description 1

### tert-Butyl 4-(4-pyridinyloxy)-1-piperidinecarboxylate (D1)

### Method A

To 1-*tert*-butoxycarbonyl-4-hydroxypiperidine (2g) in DMSO (20ml) was added potassium carbonate (2g) followed by 4-chloropyridine (1.3g). The reaction was heated to 70°C for 3h, then cooled and diluted with EtOAc (50ml). The mixture was washed with saturated brine (4x) then evaporated and chromatographed (silica gel; eluting with EtOAc/MeOH, 0-50% MeOH) to give the title compound (D1) as a gum (1.5g).

### Method B

Sodium hydride (20.88g) was suspended in DMSO (600ml) under argon and 4-chloropyridine hydrochloride (31.0g), suspended in DMSO (150ml), was added slowly over 45min. The reaction was then stirred for 10min. 1-*tert*-Butoxycarbonyl-4-hydroxypiperidine (35g), dissolved in DMSO (150ml), was added over 15min and the reaction was stirred at rt overnight. Saturated sodium hydrogen carbonate solution (150ml) was then added, slowly, and the reaction stirred for 20min. The mixture was evaporated to a minimum, redissolved in ethyl acetate (600ml) and washed with saturated sodium hydrogen carbonate (150ml)/water (150ml), followed by water (5 x 250ml). The organic layer was treated with decolourising charcoal powder (15g) and dried (MgSO₄) for 45 min. The solution was filtered and evaporated to give a yellow solid which was triturated with hexane and then dried at 50°C overnight to give the title compound (D1) as a pale yellow solid (38.0g). MS electrospray (+ve ion) 279 (MH⁺). ¹H NMR δ (CDCl₃): 8.43 (2H, d, J=4.8Hz), 6.87 (2H, d, J=4.8Hz), 4.57 (1H, m), 3.68 (2H, m), 3.37 (2H, m), 1.90 (2H, m), 1.78 (2H, m), 1.47 (9H, s).

### Description 2

### 1-Isopropyl-4-(4-piperidinyloxy)piperidine dihydrochloride (D2)

### Method A

*tert*-Butyl 4-(4-pyridinyloxy)-1-piperidinecarboxylate (D1) (0.5g) in DCM (5ml) was treated with isopropyl iodide (2ml). After 2 days the reaction was evaporated from toluene (2x) and then triturated with diethyl ether. The residue was dissolved in MeOH (10ml) containing solid ammonium formate (0.2g), and lithium borohydride (2ml, 1M solution in THF) was added slowly, under an argon stream, with rapid stirring. Then palladium on carbon (0.2g, 10% Pd/C) was added as a slurry in water (2ml), and further lithium borohydride (2ml, 1M solution in THF) was added dropwise. After 2h the reaction was diluted with EtOAc and saturated sodium hydrogen carbonate, and filtered through celite. The EtOAc layer was separated and evaporated to a gum which was dissolved in a small volume of EtOAc and treated with an excess of 95% TFA/water. After 2h toluene was added and the reaction evaporated and then re-evaporated from toluene. The residue was dissolved in EtOAc and treated with HCl (1ml, 1M in diethyl ether). Filtration of the precipitate gave the title compound (D2) (0.5g).

### Method B

### Step 1:4-[(1-{tert-Butoxycarbonyl}-4-piperidinyl)oxy]-1-(1-methylethyl)pyridinium iodide

*tert*-Butyl 4-(4-pyridinyloxy)-1-piperidinecarboxylate (D1) (5.2g) was treated with isopropyl iodide (21.6ml) and the resulting solution was heated in an oil bath at 90°C for 2.5h. The mixture was cooled and then evaporated to dryness from toluene (2× 30ml). The residue was triturated with diethyl ether (2× 150ml). The residue was dissolved in DCM and precipitated out of solution using diethyl ether. Drying the residue under vacuum provided the subtitle compound as a foam (8.4g). MS electrospray (+ve ion) 322 (MH⁺). ¹H NMR δ CDCl₃: 1.47 (9H, s), 1.70 and 1.72 (6H, 2s), 1.78 (2H, m), 2.07 (2H, m), 3.40 (2H, m), 3.80 (2H, m), 5.07 (1H, m), 5.16 (1H. m), 7.68 (2H, d J=7.6 Hz) and 9.02 (2H, d, J=7.6 Hz).

### Step 2: tert-Butyl 4-{[1-(1-methylethyl)-4-piperidinyl]oxy}-1-piperidinecarboxylate Procedure A

The product of D2, Method B, Step 1 (0.50g) was dissolved in ethanol (20ml) and hydrogenated over PtO₂ at 50psi for 6 days. The catalyst was filtered and the filtrate was evaporated to dryness. The residue was partitioned between saturated potassium carbonate solution and ethyl acetate. The organic extract was separated, washed with saturated potassium carbonate solution and brine, then dried (MgSO₄) and concentrated to leave the subtitle compound as a colourless oil (0.198g). MS electrospray (+ve ion) 327 (MH⁺). ¹H NMR δ CDCl₃: 1.03 and 1.04 (6H, 2s), 1.45 (9H, s), 1.52-1.92 (8H, m), 2.25 (2H, m), 2.75 (3H, m), 3.05 (2H, m), 3.40 (1H, m), 3.54 (1H, m), and 3.80 (2H, m).

### Procedure B

The product of D2, Method B, Step 1 (8.4g) was dissolved in methanol (100ml) and treated portionwise with granular sodium borohydride (3.2g) under an argon atmosphere. The reaction solution was stirred at rt for 1h and then acetone (20ml) was added and stirring was continued for a further 15min. The reaction solution was evaporated to dryness and the residue was partitioned between ethyl acetate and saturated potassium carbonate solution. The organic layer was separated and washed with saturated potassium carbonate solution and brine. After drying (MgSO₄) the solution was evaporated, re-dissolved in MeOH (100ml) and treated with ammonium formate (12g). The mixture was briefly degassed then 10% Pd/C (60% wet paste; 4g) was added and the mixture was heated at gentle reflux under an atmosphere of argon for 3h. The mixture was cooled and filtered through celite. The filtrate was evaporated to dryness and the residue was partitioned between ethyl acetate and saturated potassium carbonate solution. The organic layer was separated and washed with saturated potassium carbonate solution and brine and then dried (MgSO₄) and concentrated. The residue was purified by column chromatography (silica gel, step gradient eluting with 10% NH₃ in MeOH/DCM, 0-20%] which afforded the title compound as a gum (4g). MS electrospray (+ve ion) 327 (MH⁺).

### Step 3: 1-Isopropyl-4-(4-piperidinyloxy)piperidine dihydrochloride

The product of D2, Method B, Step 2 (3.8g) was dissolved in a small quantity of MeOH (15ml) and treated with 4N HCl in dioxane (35ml). After 30min the reaction was warmed to 50°C for 2h, then cooled and evaporated from toluene (2× 50ml). The residue was triturated with diethyl ether and then dried under vacuum to give the title di-hydrochloride salt (D2). This material was dissolved in the minimum amount of distilled water (*circa* 15ml) and excess solid potassium carbonate was carefully added portionwise. Ethyl acetate (50ml) was added and after warming and agitation excess magnesium sulfate was also added until there was no visible aqueous phase. The mixture was filtered and the solids washed with EtOAc (20ml) and DCM (30ml). The combined organics were treated with further magnesium sulfate to remove residual water, filtered and evaporated to provide 1-isopropyl-4-(4-piperidinyloxy) piperidine (free base compound) as a viscous oil (1.3g). MS electrospray (+ve ion) 227 (MH⁺). ¹H NMR δ CDCl₃: 1.03 (6H, d), 1.42 (2H, m), 1.59 (3H, m), 1.87 (4H, m), 2.22 (2H, m), 2.59 (2H, m), 2.68 (1H, m), 2.76 (2H, m), 3.08 (2H, m) and 3.41 (2H, m).

### Description 3

### 1-Benzyl-4-[(1-tert-butoxycarbonyl-4-piperidinyl)oxy]pyridinium bromide (D3) Method A

To *tert*-butyl 4-(4-pyridinyloxy)-1-piperidinecarboxylate (D1) (25.47g) in DCM (200ml) was added benzyl bromide (21.91ml). After 4 days the reaction was evaporated and a small volume of DCM added until all solids had dissolved. Diethyl ether was then added and the resultant precipitate was filtered off to give the title compound (D3) as a solid (32.68g).

### Method B

*tert-*Butyl 4-(4-pyridinyloxy)-1-piperidinecarboxylate (D1) (37.5g) was dissolved in DCM (400ml). Benzyl bromide (32.26 ml) was added and the reaction was stirred at rt for 4h. The reaction mixture was evaporated to a minimum and the crude residue was redissolved in a minimum quantity of DCM. Diethyl ether was added to the stirred DCM solution until the product precipitated. The pale pink solid was isolated by filtration and dried at 50°C under high vacuum overnight to give the title compound (D3) (60.0g). MS electrospray (+ve ion) 369 (M⁺). ¹H NMR δ (CDCl₃): 9.20 (2H, d, J=7.5Hz), 7.58 (2H, m), 7.50 (2H, d, J=7.5Hz), 7.41 (3H, m), 6.04 (2H, s), 5.00 (1H, m), 3.71 (2H, m), 3.38 (2H, m), 2.03 (2H, m), 1.74 (2H, m), 1.47 (9H, s).

### Description 4

### tert-Butyl 4-(4-piperidinyloxy)-1-piperidinecarboxylate (D4)

### Method A

To 1-benzyl-4-[(1-*tert*-butoxycarbonyl-4-piperidinyl)oxy]pyridinium bromide (D3) (15g) in MeOH (500ml) was slowly added lithium borohydride (100ml, 2M solution in THF) under a stream of argon whilst the temperature was kept below 30°C. After 2h formic acid was added (30ml) until pH-4. Ammonium formate (50g) in MeOH (100ml) was added as a slurry followed by palladium on carbon (2g, 10% Pd/C). After 2 days the reaction was filtered and evaporated, redissolved in EtOAc (400ml) and washed with saturated sodium hydrogen carbonate solution and brine. The organic layer was dried (MgSO₄), evaporated, and redissolved in MeOH (200ml). Acetic acid (20ml) was added followed by Pd on carbon (2g, 10% Pd/C), and the reaction hydrogenated at rt for 16h followed by 80°C for 2h. The reaction mixture was filtered, evaporated, redissolved in EtOAc (300ml) and washed with saturated sodium hydrogen carbonate solution, followed by brine, before being dried (MgSO₄) and evaporated to give the title compound (D4) as an oil (1.75g).

### Method B

1-Benzyl-4-[(1-*tert-*butoxycarbonyl-4-piperidinyl)oxy]pyridinium bromide (D3) (60g) was stirred in methanol (500ml) under argon and sodium borohydride (pellets, 20.2g) was added portionwise over 40min. The reaction was stirred for a further 45min and then acetone (65ml) was added and the reaction stirred for 1h 20min. The resulting solution was then treated using either Step 1 or Step 2 below.
**Step 1:** 10%Pd/C (paste, 20g) was added to the solution above and the mixture was hydrogenated at atmospheric pressure for 2h. The reaction was then filtered, evaporated, redissolved in ethyl acetate (500ml) and washed with saturated sodium hydrogen carbonate solution (3 x 300ml) and brine (300ml). The organic layer was dried (MgSO₄) and evaporated to give a yellow oil (41g). The oil was dissolved in methanol (700ml) and ammonium formate (69.4g) was added followed by 10%Pd/C (paste, 20g). The reaction was heated to 55°C (bath temperature) (when internal temperature achieved 30°C effervescence was observed), maintained at 55°C for 1 h and then at 60°C for 30min. The reaction was filtered and concentrated. The residue was re-dissolved in ethyl acetate (700ml) and washed with saturated potassium carbonate solution (3 x 300ml), dried (MgSO₄) and evaporated to give an oil which crystallised on standing to give the title compound (D4) as a white solid (26.0g). MS electrospray (+ve ion) 285 (MH⁺). ¹H NMR δ (COCl₃): 3.79 (2H, m), 3.65-3.38 (2H, m), 3.06 (4H, m), 2.60 (2H, m), 1.91-1.67 (4H, m), 1.59-1.31 (13H, m).
**Step 2:** An aliquot of the above solution (20ml; approx 2.1 g enol ether) was treated with decolourising charcoal powder (2g) for 2h. The mixture was filtered and evaporated. The residue was redissolved in ethyl acetate (30ml) and washed with saturated sodium hydrogen carbonate solution (3 x 20ml) and brine (20ml). The organic layer was dried (MgSO₄) and evaporated to give a white solid (1.5g). The solid was dissolved in methanol (25ml) and ammonium formate (2.54g) was added followed by 10%Pd/C (paste, 0.7g). The reaction was heated to 55°C (bath temperature) (when internal temperature achieved 30°C effervescence was observed), maintained at 55°C for 1h and then at 60°C for 30min. The reaction was filtered and concentrated. The residue was re-dissolved in ethyl acetate (50ml) and washed with saturated potassium carbonate solution (3 x 30ml), dried (MgSO₄) and evaporated to give an oil which crystallised on standing to give the title compound (D4) as a white solid (0.83g). MS electrospray (+ve ion) 285 (MH⁺). ¹H NMR δ (CDCl₃): 3.79 (2H, m), 3.65-3.38 (2H, m),3.06 (4H, m), 2.60 (2H, m), 1.91-1.67 (4H, m), 1.59-1.31 (13H, m).

### Description 5

### tert-Butyl 4-[(1-cyclobutyl-4-piperidinyl)oxy]-1-piperidinecarboxylate (D5)

To *tert*-butyl 4-(4-piperidinyloxy)piperidinecarboxylate (D4) (7.0g) and triethylamine (6.9ml) in DCM (300ml) was added cyclobutyl ketone and after 5min sodium triacetoxyborohydride (10.46g) was added. After 16h the reaction was washed with a solution of saturated potassium carbonate (2x200ml) and brine (200ml). The organic layer was dried (MgSO₄) and evaporated to give the title compound (D5) as a white solid (8.11g).

### Description 6

### 1-Cyclobutyl-4-(4-piperidinyloxy)piperidine (D6)

tert-Butyl 4-[(1-cyclobutyl-4-piperidinyl)oxy]piperidinecarboxylate (D5) (8.11g) was stirred in a solution of HCl (200ml, 4M in dioxan) and MeOH (200ml) for 3h. The solvent was removed by evaporation and the residue treated with saturated potassium carbonate solution (250ml) and extracted into DCM (3x300ml). The combined organic extracts were dried (MgSO₄) and evaporated to give the title compound (D6) as a pale yellow oil which crystallised upon standing (5.31g).

### Description 7

### 4-(4-Fluorobenzoyl)-morpholine (D7)

EDC (8.86g) was added to a solution of 4-fluorobenzoic acid (5.0g), morpholine (3.72ml), HOBT (4.82g) and triethylamine (12.41 ml) in DMF (300ml) and stirred at room temperature for 18h. After removal of the solvent by evaporation, the residue was redissolved in DCM (100ml) and washed with saturated sodium hydrogen carbonate solution (2×50ml) and brine (50ml) before drying over MgSO₄ to give the title compound (D7) (6.63g).

### Descriptions 8-11 (D8-D11)

Descriptions 8-11 were prepared from 4-fluorobenzoic acid and the appropriate amine using the procedure described in Description 7.

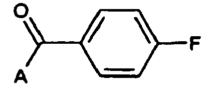

| **Description** | **A** | **Amine** | **Mass Spectrum** |
|---|---|---|---|
| | | | **(ES⁺)** |
| **D8** | MeNH- | MeNH₂ | [MH]⁺ 154 |
| **D9** | Me₂N- | Me₂NH | [MH]⁺ 168 |
| **D10** | 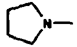 | 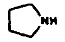 | [MH]⁺ 194 |
| **D11** | 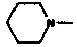 | 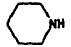 | [MH]⁺ 208 |

### Description 12

### 4-(4-Bromophenyl)-2-methyl-oxazole (D12)

4-Bromophenacyl bromide (21.3g) and acetamide (11.3g) were heated together at 130°C under argon. After 2.5h the reaction mixture was allowed to cool, and partitioned between water (150ml) and Et₂O (150ml). The organic phase was washed with aqueous NaOH (0.5N), aqueous HCl (0.5M) and saturated brine (100ml of each), dried (MgSO₄) and evaporated to give a brown solid which was recrystallised from hexanes to give the title compound (D12) as an orange solid (4.1g). LCMS electrospray (+ve) 239 (MH⁺).

### Description 13

### 5-(4-Bromophenyl)-2-methyl-oxazole (D13)

Trifluoromethanesulfonic acid (6.6ml) was added to a flask containing iodobenzene diacetate (12.2g) and MeCN (200ml) at rt. After 25min a solution of 4'-bromoacetophenone (5g) in MeCN (50ml) was added and the resultant mixture heated at reflux for 6h. The reaction was allowed to cool to rt before the solvent was evaporated and the residue partitioned between saturated aqueous sodium hydrogen carbonate (150ml) and EtOAc (150ml). The organic phase was washed with saturated brine (150ml), dried (MgSO₄) and evaporated to give an orange solid. The crude product was purified by column chromatography (silica gel, 50% EtOAc in hexanes) to give the title compound (D13) as a pate yellow solid (3.5g). LCMS electrospray (+ve) 239 (MH⁺).

### Description 14

### 5-(4-Bromophenyl)-3-methyl isoxazole (D14)

A solution of n-BuLi (81ml of a 1.6M solution in hexanes) was added to a solution of acetone oxime (4.85g) in THF (100ml) at 0°C. The reaction mixture was allowed to warm to rt over 1h. A solution of methyl 4-bromobenzoate (9.4g) in THF (30ml) was then added to the reaction mixture and allowed to stir for 24h. Water (50ml) was added to the reaction, the organics were separated and evaporated to give a brown oil, which was further evaporated from toluene (2×25ml). The crude product was purified by column chromatography (silica gel, 10-25% gradient of EtOAc in hexanes) to give the title compound (D14) as a pale yellow solid (5.4g). LCMS electrospray (+ve) 239 (MH⁺).

### Description 15

### 3-(4-Bromophenyl)-5-methyl-1,2,4-oxadiazole (D15)

### Step 1: 4-Bromo-N-hydroxy-benzenecarboximidamide

4-Bromophenylcarbonitrile (10.2g), hydroxylamine hydrochloride (7.8g) and triethylamine (11.3g) were dissolved in EtOH (250ml) and the reaction mixture was heated at reflux for 3h, after which it was evaporated to form a white precipitate of the desired amidoxime, which was filtered and washed with water (25ml). The filtrate was extracted into EtOAc (2×25ml), and the combined organic extracts were dried (Na₂SO₄) and evaporated to give a second crop of the subtitle compound (combined yield = 11.1g). LCMS electrospray (+ve) 216 (MH⁺).

### Step 2: 3-(4-Bromophenyl)-5-methyl-1,2,4-oxadtazole

The product from D15 step 1 was suspended in acetic anhydride and heated to 100°C for 4h, then 120°C for 3h. After cooling the reaction mixture was evaporated to give a brown solid. This was partitioned between saturated aqueous sodium hydrogen carbonate and EtOAc. The organic phase was washed with saturated brine, dried (Na₂SO₄) and evaporated to give a yellow solid. The crude product was purified by column chromatography (silica gel, 10-100% gradient of EtOAc in hexanes) to give the title compound (D15) as a white solid (6.2g). LCMS electrospray (+ve) 240 (MH⁺).

### Description 16

### 5-(4-Bromophenyl)-3-methyl-1,2,4-oxadiazole (D16)

4-Bromobenzamide (5.3g) and *N,N*-dimethylacetamide dimethylacetal (35ml) were heated together at 125°C for 2h. The reaction was allowed to cool to rt and the liquid evaporated to give a pale yellow solid. Hydroxylamine hydrochloride (2.2g) in 1N NaOH solution (36ml) was added, followed by dioxane (36ml) then AcOH (48ml). The reaction mixture was stirred at rt for 30min then heated at 90°C for 3h. The reaction was allowed to cool to rt and saturated aqueous K₂CO₃ solution (100ml) was added followed by DCM (200ml) before filtering. The organic phase was separated from the mixture, then saturated brine (100ml) was added and the aqueous phase was extracted into EtOAc (200ml). The combined organic phases were dried (Na₂SO₄) and evaporated to give a brown solid. The crude product was purified by column chromatography (silica gel, step gradient 10-50% EtOAc in hexanes) to give the title compound (D16) as a white solid (2.9g). LCMS electrospray (+ve) 240 (MH⁺).

### Description 17

### 2-(4-Bromophenyl)-oxazole (D17)

### Step 1: 4-Bromo-N-(2,2-dimethoxyethyl)-benzamide

Potassium carbonate (8.0g) was added to a solution of 2,2-dimethoxyethylamine in water (90ml) and acetone (40ml) at rt. The reaction mixture was cooled in an ice-water bath and 4-bromobenzoyl chloride (16.4g) dissolved in acetone (70ml) was added dropwise over 90min. The stirred reaction mixture was allowed to warm to rt. After a further 2h the reaction mixture was extracted into EtOAc (3×75ml), the combined organics were washed with saturated aqueous sodium hydrogen carbonate, dried (MgSO₄) and evaporated to give the amide as an off white solid (18.5g). LCMS electrospray (+ve) 289 (MH⁺).

### Step 2: 2-(4-Bromophenyl)-oxazole

The product of D17 step 1 was suspended in Eaton's reagent (200ml), the reaction mixture was purged with argon and heated to 240°C for 9h. The reaction mixture was then allowed to cool and stirred for 65h at rt. The crude mixture was poured over ice (1L) and stirred for 1h. The aqueous mixture was extracted into EtOAc (2×250ml), dried (MgSO₄) and evaporated to give a grey powder. This crude solid was dissolved in THF (300ml) and EtOH (300ml), and Hunig's base (21.1ml) was added. MP-carbonate resin (40.1g) and PS-thiophenol resin (69.7g) were suspended in the reaction mixture, which was stirred for 24h. The suspension was filtered and the solid phase resins washed with 1:1 THF:EtOH (3×600ml), and the combined organics evaporated to give the title compound (D17) as a white solid (9.0g). LCMS electrospray (+ve) 225 (MH⁺).

### Description 18

### 5-Bromo-N,N-dimethyl-2-pyridinecarboxamide (D18)

### Step 1: 5-Bromo-2-pyridinecarboxylic acid

5-Bromo-2-pyridinecarbonitrile (5.0g) was heated at reflux in conc. HCl (75ml) for 4.5h. The reaction was allowed to cool to room temperature and the precipitate filtered to give the subtitle compound as a white solid (3.5g). The filtrate was extracted into diethyl ether (3×200ml), and the solvent was evaporated to give a second crop of the subtitle compound (1.30g).

### Step 2: 5-Bromo-N,N-dimethyl-2-pyridinecarboxamide

The product of D18 step 1 was added to a solution of EDC (1.10g), dimethylamine hydrochloride (0.46g), HOBT (0.50g) and triethylamine (2.10ml) in DMF (70ml) and stirred at rt for 18h. After removal of the solvent by evaporation, the residue was redissolved in DCM (50ml) and washed with saturated sodium hydrogen carbonate (2×25ml), brine (25ml) and dried (Na₂SO₄) to give the crude carboxamide. Purification by chromatography [silica gel, eluting with ethyl acetate/hexanes, 0-100%] gave the title compound (D18) (0.58g).

### Descriptions 19-26 (D19-26)

Descriptions 19-26 were prepared from 5-bromo-2-pyridinecarboxylic acid (Description 18, step 1) and the appropriate amine using the procedure of Description 18, step 2.

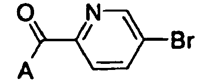

| **Description** | **A** | **Amine** | **Mass Spectrum** |
|---|---|---|---|
| | | | **(ES⁺)** |
| **D19** | MeNH- | MeNH₂ | [MH]⁺ 216 |
| **D20** | EtNH- | EtNH₂ | [MH]⁺ 230 |
| **D21** | n-PrNH- | n-PrNH₂ | [MH]⁺ 244 |
| **D22** | i-PrNH- | i-PrNH₂ | [MH]⁺ 244 |
| **D23** | 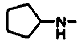 | 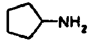 | [MH]⁺ 270 |
| **D24** | 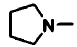 | 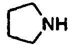 | [MH]⁺ 256 |
| **D25** | 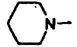 | 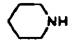 | [MH]⁺ 270 |
| **D26** | 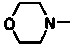 | 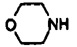 | [MH]⁺ 272 |

### Description 27

### 6-{4-[(1-Cyclobutyl-4-piperidinyl)oxy]-1-piperidinyl}-3-pyridinecarboxylic acid hydrochloride (D27)

### Step 1: Ethyl 6-{4-[(1-{[(1,1-dimethylethyl)oxy]carbonyl}-4-piperidinyl)oxy]-1-piperidinyl}-3-pyridinecarboxylate

*tert-*Butyl 4-(4-piperidinyloxy)-1-piperidinecarboxylate (D4) (1.7g), potassium carbonate (1.5g) and ethyl 6-chloro-3-pyridinecarboxylate (1.0 g) were heated at 60°C under argon overnight. The reaction mixture was evaporated and redissolved in DCM (100 ml) and washed with saturated sodium hydrogen carbonate (3 x 50ml), dried (MgSO₄) and evaporated to give a crude product which was chromatographed [silica gel, eluting with (10%NH₃ in MeOH/DCM, 0-10%] to give the subtitle compound (1.43 g).

### Step 2: Ethyl 6-[4-(4-piperidinyloxy)-1-piperidinyl]-3-pyridinecarboxylate

The product of D27 step 1 (1.43g) was stirred in 30%TFA/DCM (65ml) overnight. The reaction was evaporated, redissolved in DCM (100ml) and washed with saturated sodium hydrogen carbonate (3 x 70ml), dried (Na₂SO₄) and evaporated. After drying under high vacuum the subtitle compound was obtained as a white solid (1.04g).

### Step 3: Ethyl 6-{4-[(1-cyclobutyl-4-piperidinyl)oxy]-1-piperidinyl}-3-pyridinecarboxylate

The product of D27 step 2 (0.52g) was treated with cyclobutyl ketone (0.24ml) and sodium triacetoxyborohydride in DCM in the presence of triethylamine (0.30ml) following the procedure of Description 5 to give the subtitle compound (0.40g)

### Step 4: 6-(4-{[1-(Cyclobutyl)-4-piperidinyl]oxy}-1-piperidinyl)-3-pyridinecarboxylic acid hydrochloride

The product of D27 step 3 (0.2g) was dissolved in dioxane (15ml) and concentrated hydrochloric acid (3.5ml) was added and the reaction heated at 100°C overnight. The reaction mixture was then evaporated (co-evaporated with toluene x 3) to give the title compound (D27) (0.18g).

### Description 28

### 6-(4-{[1-(Isopropyl)-4-piperidinyl]oxy}-1-piperidinyl)-3-pyridinecarboxylic acid hydrochloride (D28)

### Step 1: Ethyl 6-{4-[(1-isopropyl-4-piperidinyl)oxy]-1-piperidinyl}-3-pyridinecarboxylate

The product of D27 step 2 (0.52g) was dissolved in acetonitrile (4ml) with isopropyl iodide (0.3ml) and potassium carbonate (0.22g) and heated at 120°C for 45min. The reaction was evaporated, redissolved in DCM (50ml) and washed with saturated sodium hydrogen carbonate (3x 30ml), brine (30ml) then dried (Na₂SO₄) to give the subtitle compound (0.45g).

### Step 2: 6-(4-{[1-(Isopropyl)-4-piperidinyl]oxy}-1-piperidinyl)-3-pyridinecarboxylic acid hydrochloride

The product of D28 step 1 (0.45g) was dissolved in dioxane (30ml) and concentrated hydrochloric acid (7ml) was added and the reaction heated at 100°C overnight. The reaction mixture was evaporated (co-evaporated with toluene (x 3) to give the title compound (D28) (0.41g).

### Description 29

### 5-{4-[(1-Cyclobutyl-4-piperidinyl)oxy]-1-piperidinyl}-2-pyrazinecarbonyl chloride hydrochloride (D29)

### Step 1: Methyl 5-{4-[(1-cyctobutyl-4-piperidinyl)oxy]-1-piperidinyl}-2-pyrazine carboxylate

1-Cyclobutyl-4-(4-piperidinyloxy)piperidine (D6) (0.5g), methyl 5-chloro-2-pyrazinecarboxylate (0.43g) and potassium carbonate (0.58g) in acetonitrile (4.5ml) was heated at 120°C in the microwave for 5min. The reaction mixture was then loaded onto an SCX column (10g) and washed with methanol (100ml) then eluted with 2M ammonia in methanol (100ml) and evaporated the give the subtitle compound (0.70g).

### Step 2: 5-{4-[(1-Cyclobutyl-4-piperidinyl)oxy]-1-piperidinyl}-2-pyrazinecarboxylic acid hydrochloride

The product of D29 step 1 (0.65g) was dissolved in concentrated hydrochloric acid (30ml) and heated at 100°C for 1.5h. The reaction was then evaporated to a minimum and dried under high vacuum to give the subtitle compound (0.67g).

### Step 3: 5-{4-[(1-Cyclobutyl-4-piperidinyl)oxy]-1-piperidinyl}-2-pyrazinecarbonyl chloride hydrochloride

The product of D29 step 2 (0.67g) was dissolved in thionyl chloride (10ml) and heated under reflux for 2.5h. The reaction mixture was then evaporated (co-evaporated with DCM x 3) to give the title compound (D29) (0.7g).

### Description 30

### 5-{4-[(1-Isopropyl-4-piperidinyl)oxy]-1-piperidinyl}-2-pyrazinecarbonyl chloride hydrochloride (D30)

The title compound (D30) was prepared from 1-isopropyl-4-(4-piperidinyloxy)piperidine (free base from D2) according to the procedures of Description 29 steps 1-3.

### Descriptions 31-34 (D31-34)

Descriptions 31-34 were prepared from 6-chloro-2-pyridinecarboxylic acid and the appropriate amine (2eq.) with EDC (1.3eq.), HOBT (1.0eq.), DIPEA (3eq.) in DCM as solvent using a similar procedure to that of Description 18 step 2

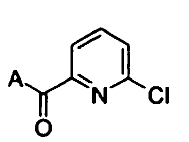

| **Description** | **A** | **Amine** | **Mass Spectrum** |
|---|---|---|---|
| | | | **(ES⁺)** |
| **D31** | MeNH- | MeNH₂ | 171/173 |
| **D32** | Me₂N- | Me₂NH | 185/187 |
| **D33** | 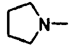 | 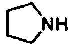 | 211/213 |
| **D34** | 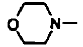 | 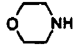 | 227/229 |

### Descriptions 35-36 (D35-36)

Descriptions 35 and 36 were prepared from 4-bromo-2-pyridinecarboxylic acid and isopropylamine and piperidine respectively, with EDC (1.3eq.), HOBT (1.0eq.), triethylamine (4eq.) in DMF as solvent using a similar procedure to that of Description 18 step 2.

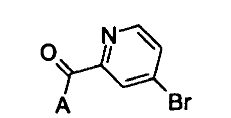

| **Description** | **A** | **Amine** | **Mass Spectrum** |
|---|---|---|---|
| | | | **(ES⁺)** |
| **D35** | 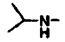 | 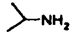 | 243/245 |
| **D36** | 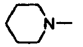 | 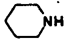 | 268/270 |

### Description 37

### 2-Chloro-5-(trifluoromethyl)pyrazine (D37)

2-Amino-5-trifluoromethylpyrazine (Miesel, US 4 293 552) was converted into 5-trifluoromethylpyrazin-2-one (Fitzjohn, EP 408196). 5-Trifluoromethylpyrazin-2-one (0.5g) was heated at reflux in POCl₃ (3ml) containing 1 drop of conc. H₂SO₄ for 3h. The cooled mixture was poured onto ice and brought to pH 5 by addition of solid NaHCO₃ and extracted (3x) with diethyl ether. The ethereal extracts were washed with water and brine, dried (Na₂SO₄) and evaporated to give the title compound (D37) as a light yellow oil (0.2g) which was sufficiently pure for use without further purification. ¹H NMR δ [CDCl₃]: 8.76 (1H, s), 8.72 (1H, s).

### Description 38

### 5-Bromo-2-(trifluoromethyl)pyrimidine (D38)

A mixture of potassium fluoride (1.77g) and cuprous iodide (5.79g) was stirred and heated together using a heat gun under vacuum (~1 mm) for 20min. After cooling, dimethyl formamide (20ml) and N-methyl pyrrolidinone (20ml) were added followed by (trifluoromethyl)trimethylsilane (4.1 ml) and 5-bromo-2-iodopyrimidine (6.5g). The mixture was stirred at rt for 5h and then the brown solution was poured into 6N ammonia solution. The product was extracted into ethyl acetate and the extracts were washed with saturated aqueous sodium hydrogen solution and brine and then dried (Na₂SO₄) and evaporated. Chromatography on silica gel (elution with 20-50% dichloromethane in pentane) gave the title compound (D38) as a white solid (2.4g). ¹H NMR (CDCl₃): 8.97 (2H, s).

### Description 39

### 5-Bromo-2-pyridinecarboxylic acid (D39)

5-Bromo-2-cyanopyridine (95.0g, 0.519 mol) was added portionwise with stirring over 2min to concentrated hydrochloric acid (650ml) at rt. The solution was stirred at rt for 25min and then it was heated to 110°C for 4.5h under an atmosphere of argon. The solution was then allowed to cool to rt over 4h and the resulting white crystals were filtered and washed with de-ionised water (4 x 200ml). The solid was then suspended in toluene (500ml) and the mixture evaporated to dryness. This was repeated with more toluene (500ml) and the resulting white powder was dried under vacuum at 50°C for 18h to give the title compound (D39) (74.4g). MS electrospray (-ve ion) 200 and 202 (M-H⁻). ¹H NMR δ (DMSO-d6): 13.40 (1H, br.s), 8.82 (1H, d, J=2.5Hz), 8.25 (1H, dd, J=8, 2.5Hz), 7.98 (1H, d, J=8Hz).

### Description 40

### 1,1-Dimethylethyl 5-bromo-2-pyridinecarboxylate (D40)

A suspension of 5-bromo-2-pyridinecarboxylic acid (D39) (68.0g) in *tert*-butanol (680ml) and pyridine (190ml) was stirred vigorously at rt for 0.5h under argon. 4-Toluenesulfonyl chloride (153.7g) was then added portionwise over 10min to give a thick white mixture which gradually dissolved over 2h to give a dark brown solution. After 4.5h at rt the reaction mixture was poured slowly with stirring onto a saturated aqueous solution of sodium hydrogen carbonate (136g) in water (1l). Stirring was continued for 18h at rt. The product was then extracted into diethyl ether (2x1l) and the combined extracts were dried (MgSO₄), filtered and concentrated to give a solid. This was treated with toluene (1l) and the mixture was evaporated to dryness. This was repeated twice more with toluene (2x1l) to give a pink solid which was dried *in vacuo* overnight to give 80.0g of product. Recrystallisation from acetone/ water gave the pure title compound (D40) (66.8g). MS electrospray (+ve ion) 281 (MNa⁺). ¹H NMR δ CDCl₃: 8.79 (1H, s), 7.90 (2H, s), 1.64 (9H, s).

### Description 41

### 1,1-Dimethylethyl 5-{4-[(1-cyclobutyl-4-piperidinyl)oxy]-1-piperidinyl}-2-pyridinecarboxylate (D41)

1,1-Dimethylethyl 5-bromo-2-pyridinecarboxylate (D40) (20.6g), 1-cyclobutyl-4-(4-piperidinyloxy)piperidine (D6) (19.0g), (±)-BINAP (2.98g) and caesium carbonate (130.0g) in dry toluene (325ml) were degassed and placed under an atmosphere of argon. Pd(OAc)₂ (1.08g) was then added and the mixture was heated at 105°C under argon for 18h. Further (±)-BINAP (1.0g) and Pd(OAc)₂ (0.36g) were then added and heating was continued for 3h. The mixture was the cooled, diluted with ethyl acetate (500ml) and filtered through a pad of celite. The filtrate was evaporated to dryness and the residue was purified by chromatography [silica gel, eluting with 0 - 4% (2M NH₃ in methanol) in DCM] to afford the title compound (D41) (21.05g). MS electrospray (+ve ion) 416 (MH⁺). ¹H NMR δ CDCl₃: 8.36 (1H, d, J=3.2Hz), 7.90 (1H, d, J=8.8 Hz), 7.12 (1H, dd, J=8.8, 3.2 Hz), 3.65 (3H, m), 3.46 (1H, m), 3.15 (2H, m), 2.67 (3H, m), 1.81-2.08 (9H, m), 1.54-1.76 (16H, m).

### Description 42

### 5-{4-[(1-Cyclobutyl-4-piperidinyl)oxy]-1-piperidinyl}-2-pyridinecarboxylic acid trifluoroacetate (D42)

1,1-Dimethylethyl 5-{4-[(1-cyclobutyl-4-piperidinyl)oxy]-1-piperidinyl}-2-pyridinecarboxylate (D41) (19.96g) was dissolved in 95:5 TFA:water (200ml) and the resulting solution was stirred at rt for 2h. It was then evaporated to dryness and the residue was treated with toluene and re-evaporated. This process was repeated five more times to ensure removal of TFA. The resulting tan coloured solid was triturated with diethyl ether, filtered and then dried overnight at 40°C to afford the tri-TFA salt (D42) (32.11g, 95%). MS electrospray (+ve ion) 360 (MH⁺). ¹H NMR δ (DMSO-d6): 9.50 (1H, br.s), 8.36 (1H, d, J=1.2Hz), 7.90 (1H, d, J=7.6Hz), 7.46 (1H, dd, J=7.6, 1.2Hz), 3.58-3.90 (5H, m), 3.36 (1H, d, J=11.6 Hz), 3.18 (3H, m), 2.72-2.90 (2H, m), 2.17 (5H, m), 1.91 (3H, m), 1.62-1.81 (3H, m), 1.50 (3H, m).

### Description 43

### 1-[(1S)-1-Methylpropyl]-4-(4-piperidinyloxy)piperidine (D43)

### Step 1: tert-Butyl 4-({1-[(1S)-1-methylpropyl]-4-piperidinyl}oxy)-1-piperidinecarboxylate

*tert*-Butyl 4-(4-piperidinyloxy)-1-piperidinecarboxylate (D4) (3.1g), (1*R*)-1-methylpropyl methanesulfonate (Bums et al., J Am. Chem. Soc., 1997, 119, 2125) (2.0g), and potassium carbonate (1.8g) were dissolved in acetonitrile (20ml)/DMF(15ml) and heated at 95°C overnight. The reaction mixture was then filtered through a plug of potassium carbonate. The filtrate was evaporated, redissolved in ethyl acetate (100ml) and washed with saturated potassium carbonate solution (3 x 70ml), dried (MgSO₄), and then purified by chromatography [silica gel, eluting with (10%NH₃ in MeOH/DCM, 0-10%] to give the subtitle compound (1.73 g).

### Step 2: 1-[(1S)-1-Methylpropyl]-4-(4-piperidinyloxy)piperidine

The product of D43, step 1 (1.73g) was dissolved in methanol (50ml) and 4M HCl in dioxane (50ml) was added and the reaction stirred at rt for 7h. The reaction mixture was then evaporated to a minimum, dissolved in DCM (100ml) and washed with saturated potassium carbonate solution (3 x 50ml). The organic layer was dried (MgSO₄) and concentrated to give the title compound (D43) as a yellow oil (0.97g).

### Description 44

### 1-[(1R)-1-Methylpropyl]-4-(4-piperidinyloxy)piperidine (D44)

The title compound (D44) was prepared from *tert*-butyl 4-(4-piperidinyloxy)-1-piperidinecarboxylate (D4) (3.1g) and (1*S*)-1-methylpropyl methanesulfonate (Bums et al., J Am. Chem. Soc., 1997, 119, 2125) (2.0g) according to the procedures of Description 43 Steps 1 and 2, and was obtained as a yellow oil (1.1g).

### Description 45

### tert-Butyl 4-{[1-(cyclopropylmethyl)-4-piperidinyl]oxy}-1-piperidinecarboxylate (D45)

tert-Butyl 4-(4-pyridinyloxy)-1-piperidinecarboxylate (D1) (5g) was treated with (bromomethyl)cyclopropane (15g) and the resulting solution was heated in an oil bath at 90°C for 2h. The mixture was cooled and then evaporated to dryness from toluene (2x 30ml) and the residue triturated with diethyl ether (2x80ml). The residue was dissolved in methanol (100ml) and treated portionwise with granular sodium borohydride (3g) under an argon atmosphere. The reaction solution was stirred at rt for 1h and then acetone (20ml) was added and stirring was continued for a further 15min. The reaction solution was evaporated to dryness and the residue was partitioned between ethyl acetate and saturated potassium carbonate solution. The organic layer was separated and washed with saturated potassium carbonate solution and brine and then dried (MgSO₄). After evaporation the residue was dissolved in MeOH (100ml) and treated with ammonium formate (12g) and the mixture was briefly degassed. 10% Pd/C (60% wet paste; 4g) was then added and the mixture was heated at gentle reflux under an atmosphere of argon for 3h. The mixture was then cooled and filtered through celite. The filtrate was evaporated to dryness and the residue was partitioned between ethyl acetate and saturated potassium carbonate solution. The organic layer was separated and washed with saturated potassium carbonate solution and brine and then dried (MgSO₄). The solution was filtered and evaporated to afford the crude title compound (D45) as a gum (4g). MS electrospray (+ve ion) 384 (MNa₂⁺), 361 (MNa⁺), 340 (MH₂⁺) and 338 (M⁺).

### Description 46

### 1-(Cyclopropylmethyl)-4-(4-piperidinyloxy)piperidine (D46)

*tert-*Butyl 4-{[1-(cyclopropylmethyl)-4-piperidinyl]oxy}-1-piperidinecarboxylate (D45) (5.3g) was dissolved in methanol (100ml) and 4N HCl in dioxane (100ml) was added. The reaction was stirred at rt for 2.5h. The reaction mixture was then evaporated and partitioned between DCM (100ml) and saturated potassium carbonate (70ml). The DCM layer was washed with saturated potassium carbonate (2 x 70ml), dried (MgSO₄) and evaporated to give the title compound (D46) as a pale yellow oil (3.43g). MS electrospray (+ve ion) 239 (MH⁺)

### Description 47

### N-(4-Bromo-2-fluorophenyl)acetamide (D47)

4-Bromo-2-fluoroaniline (1g) in EtOAc (10ml) was treated with acetic anhydride (1ml). After 30min the reaction was warmed to 50°C for 2h. The reaction was then cooled and diluted with EtOAc, washed with saturated sodium hydrogen carbonate and brine and evaporated. Purification by column chromatography [silica gel, step gradient 0-20% EtOAc/Petroleum ether] afforded the title compound (D47) as a solid (0.8g). MS electrospray (+ve ion) 232 and 234 (MH⁺).

### Description 48

### 1-(4-Pluorobenzoyl)-azetidine (D48)

The title compound (D48) was prepared from 4-fluorobenzoic acid and azetidine in a similar manner to Description 7. MS electrospray (+ve ion) 180 (MH⁺)

### Description 49

### 2-(1-Azetidinylcarbonyl)-5-bromopyridine (D49)

The title compound (D49) was prepared from 5-bromo-2-pyridinecarboxylic acid (product of Description 18, step 1) and azetidine using the procedure of Description 18, step 2. MS electrospray (+ve ion) 240, 242 (MH⁺)

### Description 50

### 5-(4-Bromo-2-fluorophenyl)-3-methyl-1,2,4-oxadiazole (D50)

4-Bromo-2-fluorobenzoic acid (5.27g) was heated at reflux in thionyl chloride (50ml) for 4h and then allowed to cool. The mixture was evaporated *in vacuo* and the residue re-evaporated with DCM (2x) to give the acid chloride as a light brown oil. This was added dropwise to vigorously stirred, ice-cooled concentrated aqueous ammonia (50ml) and when addition was complete the mixture was stirred for 5min and then extracted (3x) with EtOAc. The combined organic extracts were washed with water and brine, dried (Na₂SO₄) and evaporated to give 4-bromo-2-fluorobenzamide as a white solid (4.72g). This material and *N,N*-dimethylacetamide dimethylacetal (17ml) were heated together at 120°C for 2h. The reaction was allowed to cool to rt and the liquid evaporated *in vacuo* to give a brown gum which was partitioned between saturated aqueous sodium hydrogen carbonate and EtOAc. The organic extract was washed with water and brine, dried (Na₂SO₄) and evaporated to a gum. This was purified by chromatography (silica gel, eluant hexane/EtOAc) to give the acylamidine intermediate as a gum which solidified *in vacuo* (4.15g). Hydroxylamine hydrochloride (1.32g) in 1N NaOH solution (23.5ml) was added, followed by dioxane (23.5ml) then AcOH (30ml). The reaction mixture was stirred at rt for 30min then heated at 90°C for 3h. The reaction was allowed to cool to rt and poured into water. The pH was adjusted to ~9 by addition of solid NaHCO₃ and the precipitated product was collected by filtration, washed on the filter with water and dried at 40°C *in vacuo* to give the title compound (D50) as a greyish-brown solid (2.82g). LCMS electrospray (+ve) 257 and 259 (MH⁺).

### Description 51

### 5-(4-Bromo-3-fluorophenyl)-3-methyl-1,2,4-oxadiazole (D51)

4-Bromo-3-fluorobenzoic acid (10.09g) was heated at reflux in thionyl chloride (100ml) for 4h and then allowed to cool. The mixture was evaporated *in vacuo* and the residue re-evaporated with DCM (2x) to give the acid chloride as a light brown oil. This was added dropwise to vigorously stirred, ice-cooled concentrated aqueous ammonia (100ml) and the precipitated product was collected by filtration, washed on the filter with water and dried at 40°C *in vacuo* to give 4-bromo-3-fluorobenzamide as a white solid (9.13g). This material and *N,N*-dimethylacetamide dimethylacetal (27ml) were heated together at 120°C for 2h. The reaction was allowed to cool to rt and the liquid evaporated *in vacuo* to give a brown gum which was partitioned between saturated aqueous sodium hydrogen carbonate and EtOAc. The organic extract was washed with water and brine, dried and evaporated to give the acylamidine intermediate as a gum which solidified *in vacuo,* overnight (12.3 g). This intermediate was treated with a solution of hydroxylamine hydrochloride (4.16g) in 1M aqueous NaOH (74.2ml), dioxane (75ml) and glacial acetic acid (95ml). The reaction mixture was first stirred at rt for 30min then heated at 90°C for 3h. On cooling a first crop of crystals was filtered off and dried *in vacuo* at 50°C to give the title compound (D51) (5.5g). The filtrate afforded a second crop of crystals (2.1g). LCMS electrospray (+ve) 257 and 259 (MH⁺).

### Description 52

### 1,1-Dimethylethyl 5-(4-{[1-(1-methylethyl)-4-piperidinyl]oxy}-1-piperidinyl)-2-pyridinecarboxylate (D52)

The title compound (D52) was prepared in a similar manner to Description 41 from 1-isopropyl-4-(4-piperidinyloxy)piperidine (free base from D2) (0.377g) and 1,1-dimethylethyl 5-bromo-2-pyridinecarboxylate (D40) (0.43g). The compound was isolated as a pale yellow solid (0.39g). MS electrospray (+ion) 426 (M+Na⁺), 404 (MH⁺). ¹H NMR δ (CDCl₃): 8.36 (1H, d, J=2.8Hz), 7.90 (1H, d, J=8.8Hz), 7.12 (1H, dd, J=8.8, 2.8Hz), 3.68-3.62 (3H, m), 3.43 (1H, ddd, J= 12.4, 8.4, 3.8Hz), 3.15 (2H, ddd, J=12.4, 8.8, 3.2Hz), 2.8-2.74 (2H, m), 2.71 (1H, sep, J=6.4Hz), 2.28-2.22 (2H, m), 1.95-1.85 (4H, m), 1.75-1.68 (2H, m), 1.68-1.56 (2H, m), 1.62 (9H, s) and 1.04 (6H, d, J=6.4Hz).

### Description 53

### 5-(4-{[1-(1-Methylethyl)-4-piperidinyl]oxy}-1-piperidinyl)-2-pyridinecarboxylic acid (D53)

The title compound (D53) was prepared in a similar manner to Description 42 from *tert-*butyl 5-(4-{[1-(1-methylethyl)-4-piperidinyl]oxy}-1-piperidinyl)2-pyridinecarboxylate (D52). The compound was isolated as a bright yellow solid (0.33g). MS electrospray (+ion) 348 (MH⁺). ¹H NMR δ (DMSO-d6): 9.38-9.25 (1H, br s, OH), 8.37 (1H, app tr, J=2.8Hz), 7.94 (1H, d, J=9.2Hz), 7.55 (1H, dd, J=9.2, 2.0Hz), 3.78-3.68 (4H, m), 3.51-3.37 (2H, m), 3.29-3.18 (3H, m), 3.09-2.95 (2H, m), 3.12 (1H, br d, J=11.6Hz), 1.98-1.84 (4H, m), 1.66-1.48 (3H, m) and 1.26 (6H, d, J=6.4Hz).

### Description 54

### 5-Bromo-N-cyclobutyl-2-pyridinecarboxamide (D54)

The title compound (D54) was prepared from 5-bromo-2-pyridinecarboxylic acid (product of Description 18, step 1) and cyclobutylamine using the procedure of Description 18, step 2.

### Example 1

### 6-(4-{[1-(1-Methylethyl)-4-piperidinyl]oxy)-1-piperidinyl)-3-pyridinecarbonitrile hydrochloride (E1)

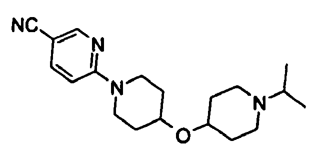

1-Isopropyl-4-(4-piperidinyloxy)piperidine dihydrochloride (D2) (0.25g) in DMSO (3ml) was treated with 2-chloro-5-cyano-pyridine (0.23g) and potassium carbonate (0.23g). The reaction was heated to 100°C for 3h then cooled and diluted with EtOAc and saturated sodium hydrogen carbonate solution. The EtOAc layer was separated, evaporated, and an aliquot processed on a mass directed autoprep HPLC system. The fractions with the correct mass were combined, evaporated from toluene, and dissolved in a small volume of EtOAc before addition of HCl (1ml, 1 M in diethyl ether). The precipitate was filtered and washed with diethyl ether before being dried under vacuum to give the title compound (E1) as a solid (23mg). LCMS electrospray (+ve ion) 329 (MH⁺); ¹H NMR δ(CD₃OD) 1.37 (6H, m), 1.84 (3H, m), 2.06 (4H, m), 2.3 (1H, m), 3.21 (3H, m), 3.5 (2H, m), 3.78 (3H, m), 3.97 (3H, m), 7.52 (1H, br d, J=14.5Hz), 8.09 (1H, m), and 8.48 (1H, d, J= 1.8Hz).

### Example 2

### 6-{4-[(1-Cyclobutyl-4-piperidinyl)oxy]-1-piperidinyl}-3-pyridinecarbonitrile hydrochloride (E2)

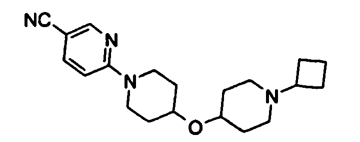

### Step 1: tert-Butyl 4-{[1-(5-cyano-2-pyridinyl)-4-piperidinyl]oxy}-1-piperidinecarboxylate

*tert-*Butyl 4-(4-piperidinyloxy)-1-piperidinecarboxylate (D4) (0.118g) was reacted with 2-chloro-5-cyano-pyridine (0.0573g) in DMSO (5ml) containing potassium carbonate (0.069g) for 4h at 60°C. The reaction was then evaporated to a minimum volume and the residue redissolved in DCM (20ml) and washed with saturated sodium hydrogen carbonate solution. Evaporation of the dried (MgSO₄) organic layer provided the subtitle compound as an oil which crystallised on standing (0.191g).

### Step 2: 6-[4-(4-Piperidinyloxy)-1-piperidinyl]-3-pyridinecarbonitrile hydrochloride

To the product of E2 step 1 (0.191g) in DCM (5ml) was added HCl in dioxan (5ml, 4M) and the mixture was stirred overnight. Evaporation of the solvent from DCM gave the subtitle compound (0.141g).

### Step 3: 6-{4-[(1-Cyclobutyl-4-piperidinyl)oxy]-1-piperidinyl}-3-pyridinecarbonitrile hydrochloride

To the product of E2 step 2 (0.141g) in DCM (5ml) was added triethylamine (0.205ml) and cyclobutyl ketone (0.073ml), and after 5min sodium triacetoxyborohydride (0.208g) was added. After 2 days the reaction was diluted with DCM (10ml) and washed with a solution of potassium carbonate (2x10ml) and brine (10ml). The organic layer was dried (MgSO₄) and evaporated and the residue chromatographed [silica gel, eluting with (10%NH₃ in MeOH)/DCM, 0-10%]. The residue was evaporated from toluene and dissolved in DCM to which was added HCl (0.5ml, 1M in diethyl ether). This was evaporated and co-evaporated from acetone (3x) and then triturated from acetone - diethyl ether to give the title compound (E2) (0.063g). LCMS electrospray (+ve ion) 341 (MH⁺), ¹H NMR δ(CD₃OD) 1.4 (2H, m), 1.6-2 (7H, m), 2.12 (2H, m), 2.3 (2H, m), 2.79 (2H, m), 3.11 (1H,br d, J=2.8 Hz), 3.35 (3H, m), 3.7 (4H, m), 4.0 (2H, m), 6.95 (1H, dd, J=2.8 and 9.2 Hz), 7.82 (1H, m) and 8.46 (1H, br s).

### Example 3

### 4-{4-[(1-Cyclobutyl-4-piperidinyl)oxy]-1-piperidinyl}benzonitrile hydrochloride (E3)

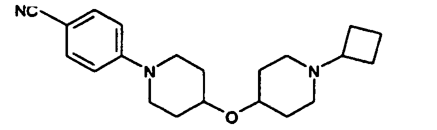

### Step 1: 4-{4-[(1-tert-Butoxycarbonyl-4-piperidinyl)oxy]-1-piperidinyl}benzonitrile

*tert*-Butyl 4-(4-piperidinyloxy)-1-piperidinecarboxylate (D4) (0.340g) was reacted with 4-fluorobenzonitrile (0.218g) in DMSO (10ml) containing potassium carbonate (0.331g) for 5h at 120°C. The reaction was then evaporated to a minimum volume and the residue redissolved in EtOAc (50ml) and washed with saturated sodium hydrogen carbonate (3×30ml) and saturated brine (30ml). Evaporation of the dried (MgSO₄) organic layer provided the subtitle compound as a pale yellow solid (0.422g).

### Step 2: 4-{4-[(4-Piperidinyl)oxy]-1-piperidinyl}benzonitrile hydrochloride

To the product of E3 step 1 (0.422g) in methanol (10ml) was added HCl in dioxan (10ml, 4M). After 3h evaporation of the solvent gave the subtitle compound (0.466g).

### Step 3: 4-{4-[(1-Cyclobutyl-4-piperidinyl)oxy]-1-piperidinyl}benzonitrile hydrochloride

To the product of E3 step 2 (0.150g) in DCM (10ml) was added triethylamine (0.077ml) and cyclobutyl ketone (0.070ml), and after 5min sodium triacetoxyborohydride (0.197g) was added. After 18h the reaction was diluted with DCM (10ml) and washed with K₂CO₃ solution (3x20ml). The organic layer was dried (MgSO₄) and evaporated and the residue chromatographed [silica gel, eluting with (10%NH₃ in MeOH)/DCM, 0-10%]. The free base product was evaporated from toluene and dissolved in DCM (5ml) to which was added HCl (1ml, 1M in diethyl ether). This was evaporated and co-evaporated from acetone (3x) and then recrystallised from acetone to give the title compound (E3) (0.063g). MS electrospray (+ion) 340 (MH⁺). ¹H NMR δ (DMSO-d6): 10.75 (1H, s), 7.56 (2H, d, J=8.8Hz), 7.03 (2H, d, J=8.8Hz), 4.57-3.46 (6H, m, obscured by H2O), 3.38-3.04 (4H, m), 2.75 (2H, m), 2.32 (2H, m), 2.20-1.57 (9H, m), 1.49 (2H, m).

### Example 4

### 4-(4-{[1-(1-Methylethyl)-4-piperidinyl]oxy}-1-piperidinyl)benzonitrile hydrochloride (E4)

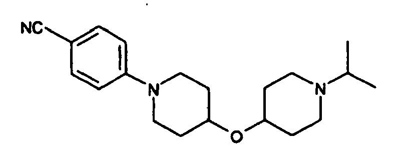

1-Isopropyl-4-(4-piperidinyloxy)piperidine dihydrochloride (D2) (0.25g) in DMSO (3ml) was treated with 4-fluorobenzonitrile (0.2g) and potassium carbonate (0.23g). The reaction was heated to 100°C for 3h then cooled and diluted with EtOAc and saturated sodium hydrogen carbonate solution. The EtOAc layer was separated, evaporated, and an aliquot processed on a mass directed autoprep HPLC system. The fractions with the correct mass were combined, evaporated from toluene, and dissolved in a small volume of EtOAc before addition of HCl (1ml, 1M in diethyl ether). The precipitate was filtered and washed with diethyl ether before being dried under vacuum to give the title compound (E4) as a solid (28mg). LCMS electrospray (+ve ion) 328 (MH⁺); ¹H NMR δ (DMSO-d6) 1.23 (6H, m), 1.46 (2H, m), 1.88 (3H, m), 2.06 (2H, m), 3.05 (2H, m), 3.14 (3H, m), 3.42 (2H, m), 3.72 (3H, m), 4.2 (2H, obscured by H2O), 7.02 (2H, d, J=8.8Hz), 7.55 (2H, d, J=8.8Hz).

### Example 5

### 4-[(4-{4-[(1-Cyclobutyl-4-piperidinyl)oxy]-1-piperidinyl}phenyl)carbonyl]morpholine hydrochloride (E5)

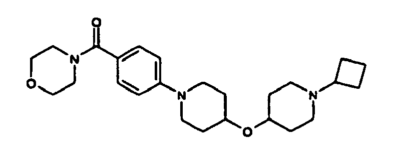

1-Cyclobutyl-4-(4-piperidinyloxy)piperidine (D6) (0.250g), 4-(4-fluorobenzoyl)-morpholine (D7) (0.330g) and anhydrous potassium carbonate (0.290g) were added to a 5ml Personal Chemistry microwave vial, to which DMSO (3ml) was added. The vial was sealed and heated at 230°C for 30min in an Emrys^{™} Optimizer microwave reactor. The crude reaction mixture was passed through an SCX cartridge (20g, MeOH (80ml) then 2N NH₃ in MeOH (80ml). Purification by chromatography [silica gel, eluting with (10%NH₃ in MeOH)/DCM, 0-10%] and treatment of the free base product dissolved in DCM (5ml) with HCl (1ml, 1M in diethyl ether), followed by evaporation and co-evaporation from acetone (3x) gave the title compound (E5) as a crystalline solid (0.125g). MS electrospray (+ion) 428 (MH⁺).¹H NMR δ (DMSO-d6): 9.7 (1H, s), 7.25 (d, 2H, J=8.4), 6.95 (2H, dd, J= 7.2, 1.6), 3.75-3.30 (20H, m, obscured by H₂O), 3.20-3.13 (1H, m), 3.08-2.95 (2H, m), 2.91-2.68 (2H, m), 2.30-2.05 (2H, m), 1.96-1.82 (3H, m), 1.81-1.45 (3H, m)

### Example 6

### 4-{4-[(1-Cyclobutyl-4-piperidinyl)oxy]-1-piperidinyl}-N-methylbenzamide hydrochloride (E6)

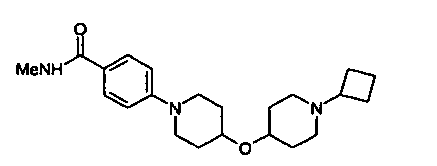

1-Cyclobutyl-4-(4-piperidinyloxy)piperidine (D6) (0.100g), 4-fluoro-*N*-methyl benzamide (D8) (0.088g) and anhydrous potassium carbonate (0.121g) were added to a 5ml Personal Chemistry microwave vial, to which DMSO (2ml) was added. The vial was sealed and heated at 230°C for 30min in an Emrys^{™} Optimizer microwave reactor. The crude reaction mixture was passed through an SCX cartridge [20g, MeOH (80ml) then 2N NH₃ in MeOH (80ml)]. Purification by chromatography [silica gel, eluting with (10%NH₃ in MeOH)/DCM, 0-10%] and treatment of the free base product dissolved in DCM (5ml) with HCl (1ml, 1M in diethyl ether), followed by evaporation and co-evaporation from acetone (3x) gave the title compound (E6) as a crystalline solid (0.074g). MS electrospray (+ion) 372 (MH⁺). ¹H NMR δ (DMSO-d6): 8.17 (1H, s), 7.72 (dd, 2H, J=9.2, 2.8), 7.04 (2H, s), 4.32-2.81 (11H, m, obscured by H₂O). 2.74 (3H, s), 2.34 (2H, m), 2.15 (2H, m), 2.00-1.40 (10H, m)

### Examples 7-14 (E7-E14)

Examples 7-14 were prepared in a similar manner to Example 6 from either 1-isopropyl-4-(4-piperidinyloxy)piperidine (free base from D2) or 1-cyclobutyl-4-(4-piperidinyloxy) piperidine (D6) and the appropriate 4-fluorobenzamide (D7-D11). All compounds displayed ¹H NMR and mass spectral data that were consistent with structure.

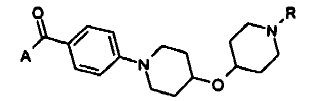

| **Example** | **A** | **R** | **Mass Spectrum** |
|---|---|---|---|
| | | | **(ES⁺)** |
| **E7** | 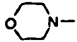 | i-Pr | [MH]⁺ 416 |
| **E8** | 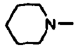 | i-Pr | [MH]⁺ 414 |
| **E9** | 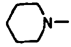 | 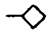 | [MH]⁺ 426 |
| **E10** | 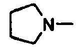 | i-Pr | [MH]⁺ 400 |
| **E11** | 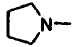 | 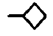 | [MH]⁺ 412 |
| **E12** | Me₂N- | i-Pr | [MH]⁺ 374 |
| **E13** | Me₂N- | 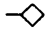 | [MH]⁺ 386 |
| **E14** | MeNH- | i-Pr | [MH]⁺ 360 |

### Example 15

### N-(3-Chloropropyl)-4-{4-[(1-cyclobutyl-4-piperidinyl)oxy]-1-piperidinyl}benzamide hydrochloride (E15)

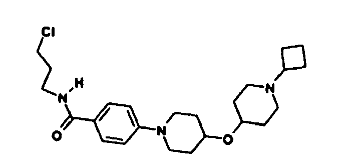

1-Cyclobutyl-4-(4-piperidinyloxy)piperidine (D6) (0.100g), 1-(4-fluorobenzoyl)-azetidine (D48) (0.098g) and anhydrous potassium carbonate (0.121g) were added to a 5ml Personal Chemistry microwave vial, to which DMSO (2ml) was added. The vial was sealed and heated at 230°C for 30min in an Emrys^{™} Optimizer microwave reactor. The crude reaction mixture was passed through an SCX cartridge [20g, MeOH (80ml) then 2N NH₃ in MeOH (80ml)]. Purification by chromatography [silica gel, eluting with (10%NH₃ in MeOH)/DCM, 0-10%] and treatment of the free base azetidine product dissolved in DCM (5ml) with HCl (1ml, 1M in diethyl ether), followed by evaporation and co-evaporation from acetone (3x) gave the title compound (E15) as a crystalline solid (0.090g). ¹H NMR δ (DMSO-d6): 10.8 (1H, m), 8.27 (1H, s), 7.74 (dd, 2H, J= 9.5 ,2.8), 7.05 (2H, s), 4.10-3.44 (6H, m, obscured by H₂O), 3.40-3.25 (3H, m), 3.19-3.00 (3H, m), 2.89-2.64 (2H, m), 2.42-2.26 (2H, m), 2.12-2.10 (2H, m), 2.08-1.81 (7H, m), 1.79-1.60 (4H, m), 1.60-1.40 (2H, m).

### Example 16

### 1-Cyclobutyl-4-({1-[4-(2-methyl-1,3-oxazol-4-yl)phenyl]-4-piperidinyl}oxy)piperidine hydrochloride (E16)

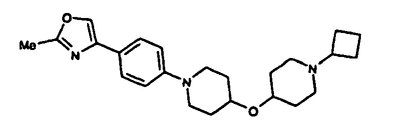

Sodium *tert*-butoxide (0.064g) was added to a solution of 1-cyclobutyl-4-(4-piperidinyloxy)piperidine (D6) (0.105g), 4-(4-bromophenyl)-2-methyl-oxazole (D12) (0.095g) and acetato(2'-di-*tert*-butylphosphino-1,1'-biphenyl-2-yl)palladium (II) (0.004g) in toluene (2ml). The reaction was heated to 60°C for 4h, then at 40°C for 1.5h, then at 55°C for a further 16h. The reaction mixture was diluted with toluene (5ml), and Argonaut MP-NCO resin (1g) was added and the mixture stirred for 1h at 55°C. The reaction mixture was loaded directly onto silica and purified by chromatography [silica gel, eluting with (10% NH₃ in MeOH)/DCM, 0-10%]. The purified residue was evaporated from toluene and dissolved in DCM (5ml) to which was added HCl (1m), 1 M in diethyl ether). Evaporation of the solvent gave the title compound (E16) (0.056g). MS electrospray (+ion) 396 (MH⁺). ¹H NMR δ (DMSO-d6): 10.71 (1H, s), 8.14 (1H, m), 7.75-7.43 (4H, m), 4.05-3.50 (6H, m, obscured by H₂O), 3.38-3.10 (4H, m), 2.91-2.66 (2H, m), 2.45 (3H, s), 2.35 (2H, m), 2.20-1.58 (11H, m).

### Example 17

### 1-(1-Methylethyl)-4-({1-[4-(3-methyl-1,2,4-oxadiazol-5-yl)phenyl]-4-piperidinyl}oxy)piperidine hydrochloride (E17)

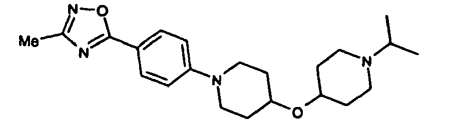

Sodium *tert*-butoxide (0.058g) was added to a solution of 1-isopropyl-4-(4-piperidinyloxy)piperidine (free base from D2) (0.10g), 5-(4-bromophenyl)-3-methyl-1,2,4-oxadiazole (D16) (0.096g) and acetato(2'-di-*tert*-butylphosphino-1,1'-biphenyl-2-yl)palladium(II) (0.004g) in toluene (3ml). The reaction was heated to 60°C for 6h, then a further charge of acetato(2'-di-*tert*-butylphosphino-1,1'-biphenyl-2-yl)palladium(II) (0.004g) was added followed by heating at 70°C for a further 16h. The reaction mixture was diluted with toluene (5ml), and Argonaut MP-NCO resin (1g) was added and the mixture stirred for 1h. After evaporation, the crude residue was diluted with MeOH (5ml) and passed through an SCX cartridge [10g, MeOH (80ml) then 2N NH₃ in MeOH (80ml)]. Purification by chromatography [silica gel, eluting with (10%NH₃ in MeOH)/DCM, 0-10%] and treatment of the free base product dissolved in DCM (5ml) with HCl (1ml, 1M in diethyl ether), followed by evaporation afforded the title compound (E17) (0.054g). MS electrospray (+ion) 385 (MH⁺). ¹H NMR δ (DMSO-d6): 10.05 (1H, m), 7.87 (2H, d, J=8.4Hz), 7.10 (2H, d, J=7.6Hz), 3.90-3.61 (4H, m, obscured by H₂O), 3.47-3.28 (2H, m), 3.18 (3H, m), 3.06-2.87 (2H, m), 2.35 (3H, s), 2.12-1.70 (6H, m), 1.51 (2H, m), 1.25 (6H, m).

### Examples 18-28 (E18-E28)

Examples 18-28 were prepared in a similar manner to Example 17 from either 1-isopropyl-4-(4-piperidinyloxy)piperidine (free base from D2) or 1-cyclobutyl-4-(4-piperidinyloxy) piperidine (D6) and the appropriate 4-bromophenyl precursor (D12-D17 or commercially available). All compounds displayed ¹H NMR and mass spectral data that were consistent with structure.

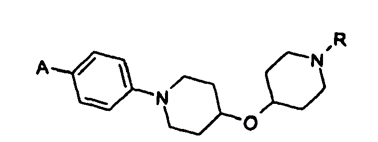

| **Example** | **A** | **R** | **Mass Spectrum** |
|---|---|---|---|
| | | | **(ES⁺)** |
| **E18** | 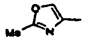 | i-Pr | [MH]⁺ 384 |
| **E19** | 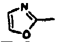 | i-Pr | [MH]⁺ 370 |
| **E20** | 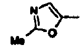 | i-Pr | [MH]⁺ 384 |
| **E21** | 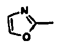 | 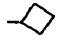 | [MH]⁺ 382 |
| **E22** | 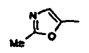 | 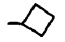 | [MH]⁺ 396 |
| **E23** | 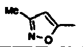 | i-Pr | [MH]⁺ 384 |
| **E24** | 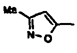 | 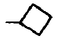 | [MH]⁺ 396 |
| **E25** | 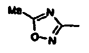 | 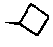 | [MH]⁺ 397 |
| **E26** | 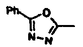 | i-Pr | [MH]⁺ 447 |
| **E27** | 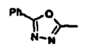 | 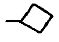 | [MH]⁺ 459 |
| **E28** | 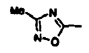 | 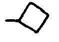 | [MH]⁺ 397 |

### Examples 29-30 (E29-E30)

Examples 29-30 were prepared in a similar manner to Example 6 from either 1-isopropyl-4-(4-piperidinyloxy)piperidine (free base from D2) or 1-cyclobutyl-4-(4-piperidinyloxy) piperidine (D6) and commercially available 5-(4-fluorophenyl)-oxazole. All compounds displayed ¹H NMR and mass spectral data that were consistent with structure.

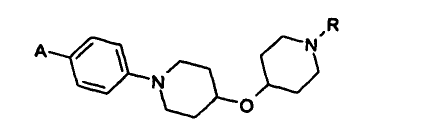

| **Example** | **A** | **R** | **Mass Spectrum** |
|---|---|---|---|
| | | | **(ES⁺)** |
| **E29** | 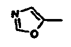 | 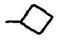 | [MH]⁺ 382 |
| **E30** | 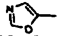 | i-Pr | [MH]⁺ 370 |

### Example 31

### 5-{4-[(1-Cyclobutyl-4-piperidinyl)oxy]-1-piperidinyl}-N,N-dimethyl-2-pyridinecarboxamide hydrochloride (E31)

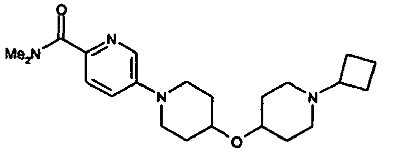

Palladium (II) acetate (0.002g) and sodium *tert*-butoxide (0.050g) were added to an argon-filled round bottom flask, followed by toluene (2ml). 1-Cyclobutyl-4-(4-piperidinyloxy)piperidine (D6) (0.100g), 5-bromo-*N*,*N*-dimethyl-2-pyridinecarboxamide (D18) and 2,8,9-triisobutyl-2,5,8,9-tetraaza-1-phosphabicyclo[3.3.3]undecane (0.005g) were added and the reaction mixture heated at 80°C for 2h. The crude reaction mixture was passed through an SCX cartridge [20g, MeOH (80ml) then 2N NH₃ in MeOH (80ml)]. Chromatography [silica gel, eluting with (10% NH₃ in MeOH)/DCM, 0-10%] and treatment of the free base product dissolved in DCM (5ml) with HCl (1ml, 1M in diethyl ether), followed by evaporation gave the title compound (E31) (0.085g). MS electrospray (+ion) 387 (MH⁺). ¹H NMR δ (DMSO-d6): 10.92 (1H, m), 8.29 (1H, s), 7.53 (2H, m),4.59-3.49 (5H, m, obscured by H₂O), 3.31 (1H, m), 3.15 (3H, m), 3.07 (3H, s), 3.01 (3H, s), 2.78 (2H, m), 2.33 (3H, m), 2.20-1.42 (11H, m).

### Examples 32-37 (E32-37)

Examples 32-37 were prepared in a similar manner to Example 31 from either 1-isopropyl-4-(4-piperidinyloxy)piperidine (free base from D2) or 1-cyclobutyl-4-(4-piperidinyloxy) piperidine (D6) and the appropriate 2-pyridine-carboxamide precursor (D18 and D24-D26). All compounds displayed ¹H NMR and mass spectral data that were consistent with structure.

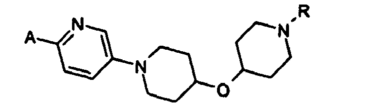

| **Example** | **A** | **R** | **Mass Spectrum** |
|---|---|---|---|
| | | | **(ES⁺)** |
| **E32** | -CONMe₂ | i-Pr | [MH]⁺ 375 |
| **E33** | 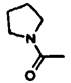 | i-Pr | [MH]⁺ 401 |
| **E34** | 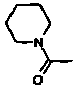 | i-Pr | [MH]⁺ 415 |
| **E35** | 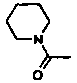 | 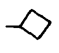 | [MH]⁺ 427 |
| **E36** | 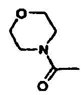 | i-Pr | [MH]⁺ 417 |
| **E37** | 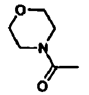 | 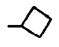 | [MH]⁺ 429 |

### Example 38

### 5-{4-[(1-Cyclobutyl-4-piperidinyl)oxy]-1-piperidinyl}-N-methyl-2-pyridinecarboxamide hydrochloride (E38)

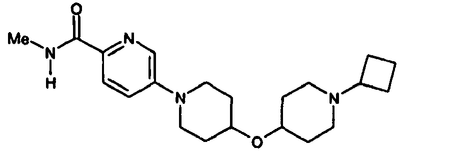

1-Cyclobutyl-4-(4-piperidinyloxy)piperidine (D6) (0.150g), 5-bromo-*N*-methyl-2-pyridinecarboxamide (D19) (0.203g) and anhydrous potassium carbonate (0.174g) were added to a 5ml Personal Chemistry microwave vial, to which DMSO (2ml) was added. The vial was sealed and heated at 250°C for 30min in an Emrys^{™} Optimizer microwave reactor. The crude mixture was diluted with MeOH (5ml) and passed through an SCX cartridge [10g, MeOH (80ml) then 2N NH₃ in MeOH (80ml)]. The eluted mixture was evaporated, then chromatography [silica gel, eluting with (10%NH₃ in MeOH)/DCM, 0-10%] and treatment of the free base product dissolved in DCM (5ml) with HCl (1ml, 1M in diethyl ether), followed by evaporation gave the title compound (E38) (0.015g). MS electrospray (+ion) 373 (MH⁺). ¹H NMR δ (CDCl₃): 8.16 (1H, d, J=2.8Hz), 8.02 (1H, d, J=8.8Hz), 7.76 (1H, d, J=5.6Hz), 7.21 (1H, dd, J=8.8Hz, J=2.8Hz), 3.64 (3H, m), 3.46 (1H, m), 3.13 (2H, m), 2.99 (3H, d, J=5.2Hz), 2.68 (3H, m), 2.09-1.78 (10H, m), 1.74-1.52 (6H, m, obscured by H₂O).

### Example 38A

### 5-{4-[(1-Cyclobutyl-4-piperidinyl)oxy]-1-piperidinyl}-N-methyl-2-pyridinecarboxamide (E38A)

Oxalyl chloride (29.0g) was added dropwise with stirring to a suspension of 5-{4-[(1-cyclobutyl-4-piperidinyl)oxy]-1-piperidinyl}-2-pyridinecarboxylic acid trifluoroacetate (D42) (32.0g) in DCM (800ml) containing DMF (3 drops) at rt. The resulting solution was stirred at rt for 1.5h and then evaporated to dryness. The residue was then re-evaporated twice from DCM to afford a dark green oily solid which was then re-suspended in DCM (300ml) and added dropwise over 30min to a 2M solution of methylamine in THF (137ml) at 0-5°C. The resulting mixture was stirred at 0-5°C for 1.5h and then evaporated to dryness. The residue was partitioned between saturated aqueous sodium hydrogen carbonate solution (100ml) and DCM (500ml). The organic layer was separated and the aqueous layer re-extracted with DCM (4x100ml). The combined organic extracts were dried (MgSO₄) filtered and evaporated to leave the crude product (21g). This was purified by silica chromatography eluting with 0-4% (2M NH₃ in methanol) in DCM. This purified material (14.15g) was dissolved in DCM (300ml) and washed with saturated aqueous potassium carbonate (50ml). The aqueous phase was re-extracted with DCM (2×50ml) and the combined organic extracts were dried (MgSO₄) and evaporated. Crystallisation from hot diethyl ether/ ethyl acetate afforded the title compound (E38A) as white crystals (8.35g). mp = 109.7°C. MS electrospray (+ve ion) 373 (MH⁺). ¹H NMR δ CDCl₃: 8.16 (1H, d, J=3.2Hz), 8.01 (1H, d, J=8.8Hz), 7.75 (1H, m), 7.20 (1H, dd, J=8.8, 3.2Hz), 3.63 (3H, m), 3.47 (1H, m), 3.12 (2H, m), 3.00 (3H, d, J=4.8Hz), 2.67 (3H, m), 1.80-2.06 (9H, m), 1.67 (7H, m),

### Examples 39-46 (E39-46)

Examples 39-46 were prepared in a similar manner to Example 38 from either 1-isopropyl-4-(4-piperidinyloxy)piperidine (free base from D2) or 1-cyclobutyl-4-(4-piperidinyloxy) piperidine (D6) and the appropriate 2-pyridine-carboxamide precursor (D20-23). The free base compounds were converted into hydrochloride salts and displayed ¹H NMR and mass spectral data that were consistent with structure.

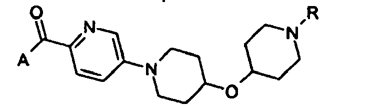

| **Example** | **A** | **R** | **Mass Spectrum** |
|---|---|---|---|
| | | | **(ES⁺)** |
| **E39** | EtNH- | i-Pr | [MH]⁺ 375 |
| **E40** | EtNH- | 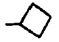 | [MH]⁺ 387 |
| **E41** | n-PrNH- | i-Pr | [MH]⁺ 389 |
| **E42** | n-PrNH- | 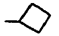 | [MH]⁺ 401 |
| **E43** | i-PrNH- | i-Pr | [MH]⁺ 389 |
| **E44** | i-PrNH- | 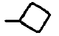 | [MH]⁺ 401 |
| **E45** | 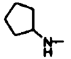 | i-Pr | [MH]⁺ 415 |
| **E46** | 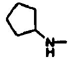 | 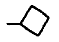 | [MH]⁺ 427 |

### Example 40A

### 5-{4-[(1-Cyclobutyl-4-piperidinyl)oxy]-1-piperidinyl}-N-ethyl-2-pyridinecarboxamide (E40A)

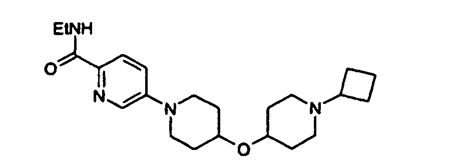

Oxalyl chloride (1.87g) was added dropwise with stirring to a suspension of 5-{4-[(1-cyclobutyl-4-piperidinyl)oxy]-1-piperidinyl}-2-pyridinecarboxylic acid trifluoroacetate (D42) (2.5g) in DCM (100ml) containing DMF (1 drop) at rt. The resulting solution was stirred at rt for 1.5h and then evaporated to dryness. The residue was then re-evaporated twice from DCM to afford the acid chloride as a dark green oily solid. A portion of the oil (0.62g) was then re-suspended in DCM (5ml) and added dropwise over 15min to a 2M solution of ethylamine in THF (7.5ml) at 0-5°C. The resulting mixture was stirred at rt for 2.0h and then loaded directly onto silica (20g cartridge) and chromatographed [silica gel, eluting with 10% NH₃ in MeOH/DCM, 0-10%]. Pooling of pure fractions afforded the title compound (E40A) as a pale brown powder (0.14g). MS electrospray (+ve ion) 387 (MH⁺). ¹H NMR δ (CDCl₃): 8.16 (1H, d, J=2.8Hz), 8.02 (1H, d, J=8.8Hz), 7.75 (1H, m), 7.21 (1H, dd, J=2.8Hz, J=8.8Hz), 3.65 (3H, m), 3.48 (3H, m), 3.11 (2H, m), 2.68 (3H, m), 1.87-1.55 (16H, m), 1.25 (3H, t).

### Example 42A

### 5-{4-[(1-Cyclobutyl-4-piperidinyl)oxy]-1-piperidinyl}-N-propyl-2-pyridinecarboxamide (E42A)

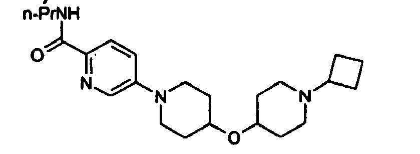

A portion of the acid chloride from Example 40A (0.62g) was suspended in DCM (5ml) and added dropwise over 15min to a solution of propylamine (1.24ml) in DCM (5ml) at 0-5°C. The resulting mixture was stirred at rt for 2.0h and then loaded directly onto silica (20g cartridge) and chromatographed [silica gel, eluting with 10%NH₃ in MeOH/DCM, 0-10%]. Pooling of pure fractions afforded the title compound (E42A) as a pale brown powder (0.25g). MS electrospray (+ve ion) 401 (MH⁺). ¹H NMR δ (CDCl₃): 8.28 (1H, d, J=2.8Hz), 8.00 (1H, d, J=8.8Hz), 7.81 (1H, m), 7.16 (1H, dd, J=2.8Hz, J=8.8Hz), 3.63 (3H, m), 3.33 (3H, m), 3.13 (2H, m), 2.68 (3H, m), 2.06-1.54 (18H, m), 0.96 (3H, t).

### Example 44A

### 5-{4-[(1-Cyclobutyl-4-piperidinyl)oxy]-1-piperidinyl}-N-isopropyl-2-pyridinecarboxamide (E44A)

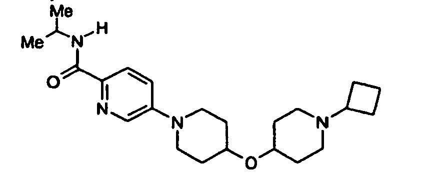

A portion of the acid chloride from Example 40 (0.62g) was suspended in DCM (5ml) and added dropwise over 15min to a solution of isopropylamine (1.24ml) in DCM (5ml) at 0-5°C. The resulting mixture was stirred at rt for 2.0h and then loaded directly onto silica (20g cartridge) and chromatographed [silica gel, eluting with 10%NH₃ in MeOH/DCM, 0-10%]. Pooling of pure fractions afforded the title compound (E44A) as a pale brown powder (0.20g). MS electrospray (+ve ion) 401 (MH⁺). ¹H NMR δ (CDCl₃): 8.15 (1H, d, J=2.8Hz), 8.02 (1H, d, J=8.8Hz), 7.63 (1H, d, J=8.1Hz), 7.21(1H, dd, J=2.8Hz, J=8.8Hz), 4.27 (1H, m), 3.62 (3H, m), 3.45 (1H, m), 3.12 (2H, m), 2.68 (3H, m), 2.07-1.53 (16H, m), 1.25 (6H, d, J=6.6Hz).

### Example 47

### 5-{4-[(1-Cyclobutyl-4-piperidinyl)oxy]-1-piperidinyl}-2-pyridinecarbonitrile hydrochloride (E47)

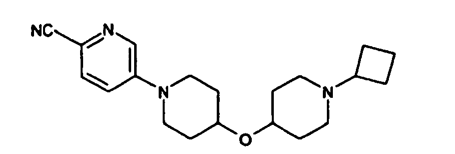

1-Cyclobutyl-4-(4-piperidinyloxy)piperidine (D6) (0.210g), 5-bromo-2-pyridinecarbonitrile (0.194g) and anhydrous potassium carbonate (0.244g) were added to a 5ml Personal Chemistry microwave vial, to which DMSO (2ml) was added. The vial was sealed and heated at 140°C for 15min in an Emrys^{™} Optimizer microwave reactor. The reaction mixture was evaporated to dryness, DCM (10ml) was added followed by Argonaut MP-NCO resin (1.0g) and the mixture allowed to stir for 16h. The crude mixture was passed through an SCX cartridge (10g, eluting with MeOH (80ml) then 2N NH₃ in MeOH (80ml). The eluted mixture was evaporated and re-evaporated from toluene (20ml). Chromatography [silica gel, eluting with (10%NH₃ in MeOH)/DCM, 0-10%] and treatment of the free base product dissolved in DCM (5ml) with HCl (1ml, 1M in diethyl ether), followed by evaporation gave the title compound (E47) (0.10g). MS electrospray (+ion) 341 (MH⁺). ¹H NMR δ (DMSO-d6): 10.38 (1H, s), 8.42 (1H, m), 7.72 (1H, d, J=9.2Hz), 7.38 (1H, m), 3.91-3.48 (6H, m), 3.31-3.10 (3H, m), 2.89-2.62 (2H, m), 2.38-1.41 (14H, m).

### Example 48

### 1-(4-{4-[(1-Cyclobutyl-4-piperidinyl)oxy]-1-piperidinyl}phenyl)ethanone hydrochloride (E48)

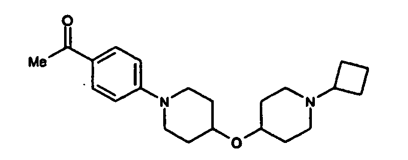

4'-Bromoacetophenone (0.102g), tris(dibenzylideneacetone)dipalladium(0) (0.025g) and 2-dicyclohexylphosphino-2'-(N,N-dimethylamino)biphenyl (0.059g) were added to degassed dioxane (5ml). After 15 min 1-cyclobutyl-4-(4-piperidinyloxy)piperidine (D6) (0.10g) and sodium *tert*-butoxide (0.061g) were added and the reaction mixture heated at 80°C for 3h, then heated at 100°C for 1h. After diluting with MeOH (5ml) the crude mixture was passed through an SCX cartridge [10g, MeOH (80ml) then 2N NH₃ in MeOH (80ml)]. Chromatography [silica gel, eluting with (10%NH₃ in MeOH)/DCM, 0-10%] and treatment of the free base product in DCM (5ml) with HCl (1ml, 1M in diethyl ether), followed by evaporation gave the title compound (E48) (0.10g). MS electrospray (+ion) 357 (MH⁺).¹H NMR δ (DMSO-d6): 7.80 (2H, dd, J=8.8, 1.2), 7.00 (2H, dd, J=8.8, 2.8), 3.78-3.46 (5H, m), 3.35-3.07 (4H, m), 2.87-2.64 (2H, m), 2.43 (3H, s), 2.41-2.39 (2H, m), 2.20-2.10 (2H, m), 2.08-1.81 (5H, m), 1.78-1.60 (3H, m), 1.57-1.42 (2H, m).

### Example 48A

### 1-(4-{4-[(1-Cyclobutyl-4-piperidinyl)oxy]-1-piperidinyl}phenyl)ethanone (E48A)

4-Fluoroacetophenone (0.21g), 1-cyclobutyl-4-(4-piperidinyloxy)piperidine (D6) (0.25g), and potassium carbonate (0.29g) in DMSO (1.5ml) were heated in a microwave reactor at 160°C for 15min. The reaction was then poured directly onto an SCX column (10g) and washed with MeOH (60ml) and then eluted with 2M ammonia in MeOH solution (60ml). After evaporation the residue was purified by chromatography [silica gel (20g cartridge), eluting with 10%NH₃ in MeOH/DCM, 0-10%]. Pure fractions were evaporated to give the title compound (E48A) as a pale yellow solid (0.38g). MS electrospray (+ve ion) 357 (MH⁺). ¹H NMR δ (CDCl₃): 7.87 (2H, d, J=9.0Hz), 6.86 (2H, d, J=9.0Hz), 3.68 (3H, m), 3.46 (1H, m), 3.14 (2H, m), 2.68 (3H, m), 2.51 (3H, s), 2.05-1.55 (16H, m, obscured by H20).

### Example 49

### 5-{4-[(1-Cyclobutyl-4-piperidinyl)oxy]-1-piperidinyl}-2-(trifluoromethyl)pyridine hydrochloride (E49)

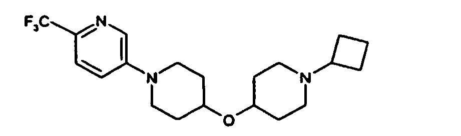

5-Bromo-2-trifluoromethylpyridine (F. Cottet and M. Schlosser, Eur. J. Org. Chem., 2002, 327) (0.187g), tris(dibenzylideneacetone)dipalladium(0) (0.034g) and 2-dicyclohexylphosphino-2'-(N,N-dimethylamino)biphenyl (0.091g) were added to degassed dioxane (4ml). After 15 min 1-cyclobutyl-4-(4-piperidinyloxy)piperidine (D6) (0.150g) and sodium *tert*-butoxide (0.095g) were added and the reaction mixture heated at 100°C for 3h, then stirred at rt for 16h. Argonaut MP-NCO resin (0.4g) was added and the mixture allowed to stir for 1h. After diluting with MeOH (5ml) the crude mixture was passed through an SCX cartridge [10g, MeOH (80ml) then 2N NH₃ in MeOH (80ml)]. Chromatography [silica gel, eluting with (10%NH₃ in MeOH)/DCM, 0-10%] and treatment of the free base product dissolved in DCM (5ml) with HCl (1ml, 1M in diethyl ether), followed by evaporation afforded the title compound (E49) (0.10g). MS electrospray (+ion) 384 (MH⁺). ¹H NMR δ (DMSO-d6): 10.75 (1H, s), 8.43 (1H, m), 7.62 (1H, d, J=8.8Hz), 7.44 (1H, d, 9.2Hz), 4.32-3.44 (5H, m, obscured by H₂O), 3.35-3.06 (4H, m), 2.88-2.62 (2H, m), 2.42-1.40 (14H, m).

### Examples 50-57 (E50-57)

Examples 50-57 were prepared in a similar manner to Description 18 step 2 from either 6-(4-{[1-(isopropyl)-4-piperidinyl]oxy}-1-piperidinyl)-3-pyridinecarboxylic acid hydrochloride (D28) or 6-(4-[(1-cyclobutyl-4-piperidinyl)oxy]-1-piperidinyl)-3-pyridinecarboxylic acid hydrochloride (D27) and the appropriate amine with EDC (1.3eq.), HOBT (1.0eq.), triethylamine (6eq.) in DMF/DCM (1:1; vol:vol). All compounds displayed ¹H NMR and mass spectral data that were consistent with structure.

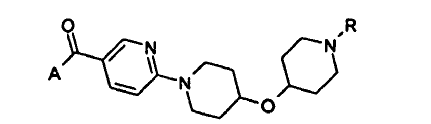

| **Example** | **A** | **R** | **Mass Spectrum** |
|---|---|---|---|
| | | | **(ES⁺)** |
| **E50** | MeNH- | i-Pr | [MH]⁺ 361 |
| **E51** | Me₂N- | i-Pr | [MH]⁺ 375 |
| **E52** | 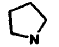 | i-Pr | [MH]⁺ 401 |
| **E53** | 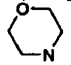 | i-Pr | [MH]⁺ 417 |
| **E54** | MeNH- | 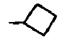 | [MH]⁺ 373 |
| **E55** | Me₂N- | 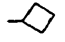 | [MH]⁺ 387 |
| **E56** | 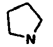 | 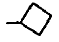 | [MH]⁺ 413 |
| **E57** | 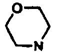 | 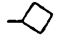 | [MH]⁺ 429 |

### Examples 58-65 (E58-65)

Examples 58-65 were prepared in a similar manner to Example 38 from either 1-isopropyl-4-(4-piperidinyloxy)piperidine (free base from D2) or 1-cyclobutyl-4-(4-piperidinyloxy) piperidine (D6) and the appropriate 6-pyridine-carboxamide (D31-34). The reactions were carried out at 210°C for 30min. All compounds displayed ¹H NMR and mass spectral data that were consistent with structure.

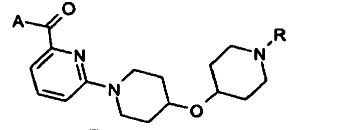

| **Example** | **A** | **R** | **Mass Spectrum** |
|---|---|---|---|
| | | | **(ES⁺)** |
| **E58** | MeNH- | i-Pr | [MH]⁺ 361 |
| **E59** | MeNH- Me₂N- | i-Pr | [MH]⁺ 375 |
| **E60** | 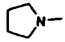 | i-Pr | [MH]⁺ 401 |
| **E61** | 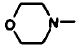 | i-Pr | [MH]⁺ 417 |
| **E62** | MeNH- | 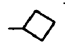 | [MH]⁺ 373 |
| **E63** | Me₂N- | 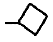 | [MH]⁺ 387 |
| **E64** | 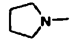 | 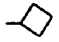 | [MH]⁺ 413 |
| **E65** | 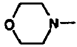 | 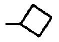 | [MH]⁺ 429 |

### Examples 66-69 (E66-69)

Examples 66-69 were prepared in a similar manner to Example 38 from either 1-isopropyl-4-(4-piperidinyloxy)piperidine (free base from D2) or 1-cyclobutyl-4-(4-piperidinyloxy)piperidine (D6) and the appropriate 2-pyridine-carboxamide (D35-D36). All compounds displayed ¹H NMR and mass spectral data that were consistent with structure.

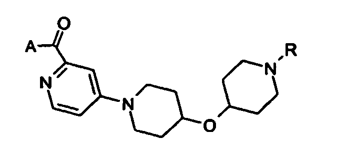

| **Example** | **A** | **R** | **Mass Spectrum** |
|---|---|---|---|
| | | | (ES⁺) |
| **E66** | i-PrNH- | i-Pr | [MH]⁺ 389 |
| **E67** | i-PrNH- | 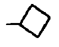 | [MH]⁺ 401 |
| **E68** | 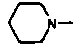 | i-Pr | [MH]⁺ 415 |
| **E69** | 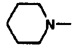 | 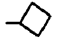 | [MH]⁺ 427 |

### Example 70

### 5-{4-[(1-Cyclobutyl-4-piperidinyl)oxy]-1-piperidinyl}-N-methyl-2-pyrazinecarboxamide butanedioate (E70)

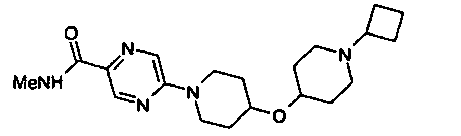

5-{4-[(1-Cyclobutyl-4-piperidinyl)oxy]-1-piperidinyl}-2-pyrazinecarbonyl chloride hydrochloride (D29) (0.1g) and methylamine hydrochloride (0.05g) were stirred in DCM (5ml). Triethylamine (0.2ml) was added and the reaction stirred at rt overnight. The reaction mixture was diluted with DCM (15ml) and washed with saturated sodium hydrogen carbonate (2 x 10m), brine (10ml) and dried (MgSO₄). The DCM solution was then loaded directly onto silica (10g cartridge) and chromatographed [silica gel, eluting with 10%NH₃ in MeOH/DCM, 0-10%]. The purified free base was evaporated from toluene then dissolved in acetone and treated with 1.0 molar equivalent of succinic acid as a solution in ethanol. The solution was concentrated and then evaporated from acetone (x 3) to give the title compound (E70) as a white solid (0.01g). MS electrospray (+ve ion) 374 (MH⁺). ¹H NMR δ (DMSO-d6): 8.57 (1H, d, J=1.2Hz), 8.32 (1H, m), 8.27 (1H, d, J=1.2Hz), 4.07 (2H, m), 3.71 (1H, m), 3.58-3.18 (5H, m), 2.84 (1H, m), 2.75 (3H, d, J=4.8Hz), 2.67 (2H, m), 2.39 (4H, s, succinate), 2.00 (2H, m), 1.85 (6H, m), 1.63 (2H, m), 1.43 (4H, m).

### Examples 71-74 (E71-74)

Examples 71-74 were prepared in a similar manner to Example 70 by reaction of 5-{4-[(1-cyclobutyl-4-piperidinyl)oxy]-1-piperidinyl}-2-pyrazinecarbonyl chloride hydrochloride (D29) with 3 molar equivalents of the appropriate amine. In the case of Example 71 the isolated free base product was converted into the butanedioate salt.

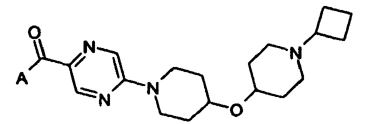

| **Example** | **A** | **Mass Spectrum** | **¹H NMR** |
|---|---|---|---|
| | | **(ES⁺)** | |
| **E71** | EtNH- | [MH]⁺ 388 | **δ (DMSO-d6)** |
| | | | 8.57 (1H, s), 8.35 (1H, t), 8.26 (1H, s), 4.07 (2H, m), 3.71 (1H, m), 3.57-3.14 (5H, m), 2.80 (1H, m), 2.67 (2H, m), 2.08-1.71 (10H, m), 1.68-1.36 (6H, m), 1.09 (3H, t). |
| **E72** | n-PrNH- | [MH]⁺ 402 | **δ (CDCl₃)** |
| | | | 8.83 (1H, s), 7.96 (1H,s), 7.48 (1H, t), 4.01 (2H, m), 3.71 (1H, m), 3.42 (5H, m), 2.66 (3H, m), 2.07-1.80 (11H, m), 1.75-1.50 (7H, m), 0.98 (3H, t). |
| **E73** | i-PrNH- | [MH]⁺ 402 | **δ (CDCl₃)** |
| | | | 8.83 (1H, s), 7.95 (1H,s), 7.27 (1H, m), 4.28 (1H, m), 4.01 (2H, m), 3.71 (1H, m), 3.45 (3H, m), 2.66 (3H, m), 2.10-1.78 (9H, m), 1.76-1.50 (7H, m), 1.26 (6H, d, J=6.8Hz). |
| **E74** | 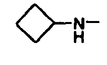 | [MH]⁺ 414 | **δ (CDCl₃)** |
| | | | 8.81 (1H, s), 7.96 (1H,s), 7.58 (1H, d, J=8.4Hz), 4.63 (1H, m), 4.02 (2H, m), 3.71 (1H, m), 3.47 (3H, m), 2.66 (3H, m), 2.41 (2H, m), 2.07-1.44 (20H, m). |

### Examples 75-79 (E75-79)

Examples 75-79 were prepared in a similar manner to Example 70 by reaction of 5-{4-[(1-isopropyl-4-piperidinyl)oxy]-1-piperidinyl}-2-pyrazinecarbonyl chloride hydrochloride (D30) with 3 molar equivalents of the appropriate amine. In the case of Examples 75, 78 and 79, the isolated free base compounds were converted into the corresponding butanedioate salts. All compounds displayed ¹H NMR and mass spectral data that were consistent with structure.

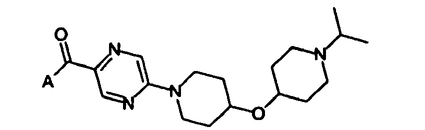

| **Example** | **A** | **Mass Spectrum** |
|---|---|---|
| | | **(ES⁺)** |
| **E75** | MeNH- | [MH]⁺ 362 |
| **E76** | EtNH- | [MH]⁺ 376 |
| **E77** | n-PrNH- | [MH]⁺ 390 |
| **E78** | i-PrNH- | [MH]⁺ 390 |
| **E79** | 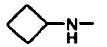 | [MH]⁺ 402 |

### Example 80

### 5-(4-{[1-(1-Methylethyl)-4-piperidinyl]oxy}-1-piperidinyl)-2-pyridinecarbonitrile hydrochloride (E80)

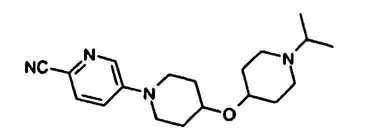

The title compound (E80) was prepared in a similar manner to Example 47 from 1-isopropyl-4-(4-piperidinyloxy)piperidine (free base from D2) and 5-bromo-2-pyridinecarbonitrile MS electrospray (+ion) 329 (MH⁺).

### Example 81

### 5-{4-[(1-Methylethyl -4-piperidinyl)oxy]-1-piperidinyl}-2-(trifluoromethyl)pyridine hydrochloride (E81)

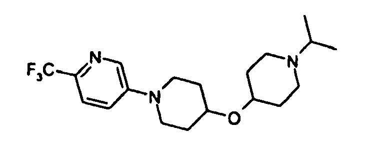

The title compound (E81) was prepared in a similar manner to Example 49 from 1-isopropyl-4-(4-piperidinyloxy)piperidine (free base from D2) and 5-bromo-2-trifluoromethylpyridine MS electrospray (+ion) 372 (MH⁺).

### Example 82

### 3-{4-[(1-Cyclobutyl-4-piperidinyl)oxy]-1-piperidinyl}-6-(trifluoromethyl)pyridazine hydrochloride (E82)

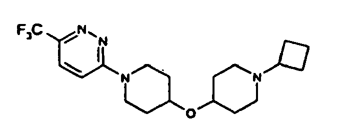

3-Chloro-6-(trifluoromethyl)pyridazine (A.J. Goodman, S.P. Stanforth and B. Tarbit, Tetrahedron, 1999, 55(52), 15067-15070) (0.09g), 1-cyclobutyl-4-(4-piperidinyloxy)piperidine (D6) (0.10g), and potassium carbonate (0.11g) in DMSO (2ml) were heated in the microwave at 120°C for 5min. The reaction was poured directly onto an SCX column (10g) and washed with MeOH (60ml) and then eluted with 2M ammonia in MeOH solution (60ml). After evaporation the residue was purified by chromatography [silica gel (10g cartridge), eluting with 10%NH₃ in MeOH/DCM, 0-10%]. Pure fractions were evaporated and the free base was dissolved in DCM and converted into the HCl salt with excess 1M HCl in diethyl ether. After evaporation of solvent the title compound (E82) was obtained as a pale brown solid (0.16g). MS electrospray (+ve ion) 385 (MH⁺). ¹H NMR δ (DMSO-d6): 11.09 (1H, s), 7.80 (1H, d, J=9.6Hz), 7.48 (1H, d, J=9.6Hz), 4.10 (2H, m), 3.95-3.38 (5H, m), 3.33-3.04 (2H, m), 2.90-2.65 (2H, m), 2.38 (2H, m), 2.10-1.57 (10H, m), 1.50 (2H, m).

### Example 83

### 3-(4-{[1-(1-Methylethyl)-4-piperidinyl]oxy}-1-piperidinyl)-6-(trifluoromethyl)pyridazine (E83)

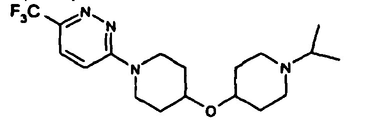

The title compound (E83) was prepared in a similar manner to Example 82 from 1-isopropyl-4-(4-piperidinyloxy)piperidine (free base from D2) and 3-chloro-6-(trifluoromethyl)pyridazine. MS electrospray (+ion) 373 (MH⁺).

### Example 84

### 2-{4-[(1-Cyclobutyl-4-piperidinyl)oxy]-1-piperidinyl}-5-(trifluoromethyl)pyrazine hydrochloride (E84)

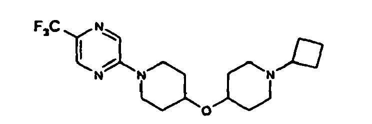

2-Chloro-5-(trifluoromethyl)pyrazine (D37) (0.09g), 1-cyclobutyl-4-(4-piperidinyloxy)piperidine (D6) (0.10g), and potassium carbonate (0.11g) in DMSO (2ml) were heated in a microwave reactor at 120°C for 5 min. The reaction was poured directly onto an SCX column (10g), washed with MeOH (60ml) and then eluted with 2M ammonia in MeOH solution (60ml). After evaporation the residue was purified by chromatography [silica gel (10g cartridge), eluting with 10%NH₃ in MeOH/DCM. 0-10%]. Pure fractions were evaporated and the free base was dissolved in DCM and converted into the HCl salt with excess 1M HCl in diethyl ether. Evaporation of solvent afforded the title compound (E84) as a pale brown solid (0.17g). MS electrospray (+ve ion) 385 (MH⁺). ¹H NMR δ (DMSO-d6): 10.94 (1H, s), 8.46 (2H, s), 4.06 (2H, m), 3.90-3.38 (5H, m), 3.35-3.06 (2H, m), 2.90-2.65 (2H, m), 2.38 (2H, m), 2.20-1.60 (10H, m), 1.48 (2H, m).

### Example 85

### 2-(4-{[1-(1-Methylethyl)-4-piperidinyl]oxy}-1-piperidinyl)-5-(trifluoromethyl)pyrazine hydrochloride (E85)

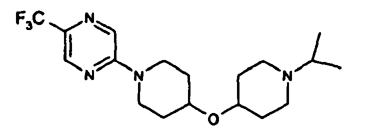

The title compound (E85) was prepared in a similar manner to Example 84 from 1-isopropyl-4-(4-piperidinyloxy)piperidine (free base from D2) and 2-chloro-5-(trifluoromethyl)pyrazine. MS electrospray (+ion) 373 (MH⁺).

### Example 86

### 2-{4-[(1-Cyclobutyl-4-piperidinyl)oxy]-1-piperidinyl}-5-(trifluoromethyl)pyridine hydrochloride (E86)

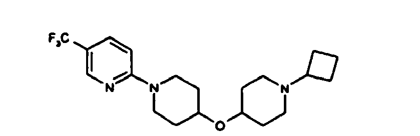

2-Bromo-5-(trifluoromethyl)pyridine (0.11g), 1-cyclobutyl-4-(4-piperidinyloxy)piperidine (D6) (0.10g), and potassium carbonate (0.11g) in DMSO (2ml) were heated in a microwave reactor at 120°C for 5min. The reaction was poured directly onto an SCX column (10g), washed with MeOH (60ml) and then eluted with 2M ammonia in MeOH solution (60ml). After evaporation the residue was purified by chromatography [silica gel (10g cartridge), eluting with 10%NH₃ in MeOH/DCM, 0-10%]. Pure fractions were evaporated and the free base was dissolved in DCM and converted into the HCI salt with excess 1M HCl in diethyl ether. Evaporation of solvent afforded the title compound (E86) as a pale brown solid (0.12g). MS electrospray (+ve ion) 384 (MH⁺). ¹H NMR δ (DMSO-d6): 10.94 (1H, s), 8.31 (1H, s), 7.80 (1H, d, J=9.2Hz),7.03 (1H, d, J=9.2Hz), 3.97 (2H, m), 3.90-3.06 (7H, m), 2.87-2.66 (2H, m), 2.38 (2H, m), 2.20-1.60 (10H, m), 1.42 (2H, m).

### Example 87

### 2-(4-{[1-(1-Methylethyl)-4-piperidinyl]oxy}-1-piperidinyl)-5-(trifluoromethyl)pyridine hydrochloride (E87)

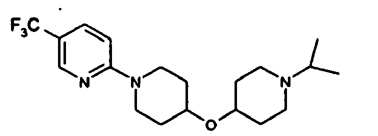

The title compound (E87) was prepared in a similar manner to Example 86 from 1-isopropyl-4-(4-piperidinyloxy)piperidine (free base from D2) and 2-bromo-5-(trifluoromethyl)pyridine. MS electrospray (+ion) 372 (MH⁺).

### Example 88

### 5-{4-[(1-Cyclobutyl-4-piperidinyl)oxy]-1-piperidinyl}-2-(trifluoromethyl)pyrimidine (E88)

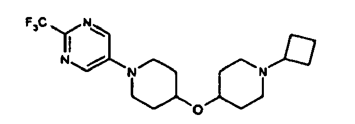

5-Bromo-2-(trifluoromethyl)pyrimidine (D38) (0.18g), 1-cyclobutyl-4-(4-piperidinyloxy)piperidine (D6) (0.15g), 2-dicyclohexylphosphino-2'-(N,N-dimethylamino)biphenyl (0.09g), tris(dibenzylidineacetone)dipalladium (0.054g), and sodium *tert*-butoxide (0.095g) in dioxane (2ml) were heated at 120°C in a microwave reactor for 5min. The reaction was poured directly onto an SCX column (10g) and washed with MeOH (60ml) and then eluted with 2M ammonia in MeOH solution (60ml). After evaporation the residue was purified by chromatography [silica gel (10g cartridge), eluting with 10%NH₃ in MeOH/DCM, 0-10%]. Fractions containing the required product were evaporated and repurified by mass directed autoprep HPLC to give the formate salt of the product. The formate salt was dissolved in MeOH (10ml) and treated with excess MP carbonate resin (2.89mmoles/g loading;1.0g) for 1h, filtered and evaporated to give the title compound (E88) as a white solid (0.068g). MS electrospray (+ve ion) 385 (MH⁺). ¹H NMR δ (CDCl₃): 8.39 (2H, s), 3.69 (1H, m), 3.62 (2H, m), 3.48 (1H, m), 3.22 (2H, m), 2.66 (3H, m), 2.08-1.80 (10H, m), 1.79-1.56 (6H, m).

### Example 89

### 5-(4-{[1-(1-Methylethyl)-4-piperidinyl]oxy}-1-piperidinyl)-2-(trifluoromethyl) pyrimidine (E89)

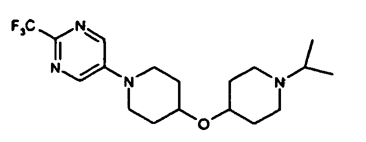

The title compound (E89) was prepared in a similar manner to Example 88 from 1-isopropyl-4-(4-piperidinyloxy)piperidine (free base from D2) and 5-bromo-2-(trifluoromethyl)pyrimidine (D38). MS electrospray (+ion) 373 (MH⁺). ¹H NMR δ (CDCl₃): 8.93 (2H, s), 3.71 (1H, m), 3.61 (2H, m), 3.43 (1H, m), 3.23 (2H, m), 2.73 (3H, m), 2.26 (2H, m), 1.90 (4H, m), 1.75 (2H, m), 1.62 (2H, m), 1.04 (6H, d, J=6.4Hz).

### Example 90

### 1-(4-{4-[(1-Cyclobutyl-4-piperidinyl)oxy]-1-piperidinyl}phenyl)-1-propanone (E90)

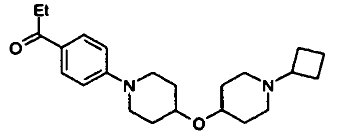

1-(4-Fluorophenyl)-1-propanone (0.24g), 1-cyclobutyl-4-(4-piperidinyloxy)piperidine (D6) (0.25g), and potassium carbonate (0.29g) in DMSO (1.5 ml) were heated in a microwave reactor at 160°C for 15min. The reaction was poured directly onto an SCX column (10g) and washed with MeOH (60 ml) and then eluted with 2M ammonia in MeOH solution (60ml). After evaporation the residue was purified by chromatography [silica gel (20g cartridge), eluting with 10% NH₃ in MeOH/DCM, 0-10%]. Pure fractions were evaporated to give the title compound (E90) as a pale yellow solid (0.32g). MS electrospray (+ve ion) 371 (MH⁺). ¹H NMR δ (CDCl₃): 7.87 (2H, d, J=8.8Hz), 6.88 (2H, d, J=9.2Hz), 3.68 (3H, m), 3.48 (1H, m), 3.13 (2H, m), 2.90 (2H, q, J=7.2Hz), 2.66 (3H, m), 2.06-1.55 (16H, m), 1.20 (3H, t, J=7.2Hz).

### Examples 91-92 (E91-92)

Examples 91-92 were prepared by reacting1-cyclobutyl-4-(4-piperidinyloxy)piperidine (D6) (1.0mmol), and the appropriate 4-fluorophenyl ketone (1.6mmol) in the presence of potassium carbonate (2.1 mmol) in DMSO (1.5ml) in a microwave reactor at 160°C for 15min using a similar procedure to that of Example 90.

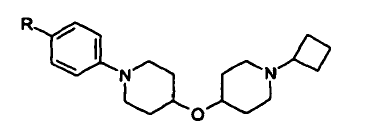

| **Example** | **R** | **Mass Spectrum** | **¹H NMR** |
|---|---|---|---|
| | | **(ES⁺)** | |
| **E91** | 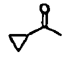 | [MH]⁺ 383 | δ (CDCl₃): 7.94 (2H, d, J=9.2Hz), 6.89 (2H, d, J=8.8Hz), 3.69 (3H ,m), 3.46 (1H, m), 3.13 (2H, m), 2.62 (4H, m), 2.07-1:80 (7H, m), 1.75-1.54 (9H, m), 1.17 (2H, m), 0.94 (2H, m). |
| **E92** | 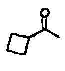 | [MH]⁺ 397 | δ (CDCl₃): 7.80 (2H, d, J=9.2Hz), 6.86 (2H, d, J=8.8Hz), 3.93 (1H, m), 3.65 (3H ,m), 3.45 (1H, m), 3.11 (2H, m), 2.67 (3H, m), 2.40 (2H, m), 2.26 (2H, m), 2.06-1.78 (11H, m), 1.73-1.52 (7H, m). |

### Example 93

### 1-(6-{4-[(1-Cyclobutyl-4-piperidinyl)oxy]-1-piperidinyl}-3-pyridinyl)ethanone (E93)

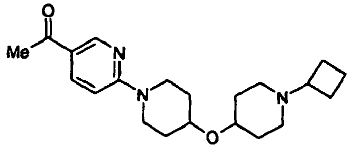

The title compound (E93) was prepared in a similar manner to Example 90 by reacting 1-cyclobutyl-4-(4-piperidinyloxy)piperidine (D6) (1mmol), 1-(6-chloro-3-pyridinyl)ethanone (1.6mmol) and potassium carbonate (2.1mmol) in DMSO (1.5ml) in a microwave at 120°C for 15min. MS electrospray (+ion) 358 (MH⁺). ¹H NMR δ (CDCl₃): 8.71 (1H, m), 8.01 (1H, dd, J=2.4Hz, J=8.8Hz), 6.64 (1H, d, J=9.2Hz), 4.07 (2H, m), 3.69 (1H, m), 3.40 (3H, m), 2.65 (3H, m), 2.49 (3H, s), 2.06-1.54 (16H, m).

### Example 94

### 6-{4-[(1-Cyclobutyl-4-piperidinyl)oxy]-1-piperidinyl}-2-methylquinoline hydrochloride (E94)

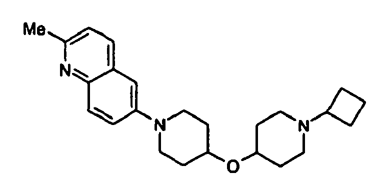

A solution of 1-cyclobutyl-4-(4-piperidinyloxy)piperidine (D6) (0.16g) in degassed dioxane (2.5ml) was treated with 6-bromo-2-methyl-quinoline (0.15g), 2-dicyclohexylphosphino-2'-(N,N-dimethylamino)biphenyl (0.06g), tris(dibenzylidineacetone)dipalladium (0.03g), and sodium *tert*-butoxide (0.13g). The mixture was heated at 120°C in a microwave reactor for 5min. The crude product was dissolved in methanol (30ml) and applied to an SCX column which was flushed with methanol and then eluted with 10%NH₃ in methanol. After evaporation the residue was purified by chromatography [silica gel, eluting with (10% NH₃ in MeOH)/DCM. 0-10%]. Pooling of pure fractions afforded the free base product which was treated with HCl in diethyl ether to give the title compound (E94) as a solid (0.19g). MS electrospray (+ve ion) 380 (MH⁺). ¹H NMR δ (methanol-d4): 8.75-8.78 (1H, d, J=8.0Hz), 8.00-8.02 (2H, m), 7.76-7.78 (1H, d, J=8.0Hz), 7.61 (1H, s), 3.97-4.0 (1H, m), 3.68-3.90 (5H, m), 3.48-3.55 (1H, m), 3.0-3.10 (2H, m), 2.9 (3H, s), 2.8-2.89 (1H, m), 1.67-2.4 (15H, m).

### Example 95

### 1,1-Dimethylethyl 5-(4-{[1-(1-methylethyl)-4-piperidinyl]oxy}-1-piperidinyl)-2-pyridinecarboxylate (E95)

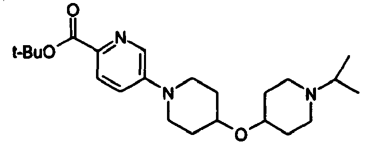

The title compound (E95) was prepared as described in Description 52. MS electrospray (+ve ion) 404 (MH⁺).

### Example 96

### 1,1-Dlmethylethyl 5-{4-[(1-cyclobutyl-4-piperidinyl)oxy]-1-piperidinyl}-2-pyridinecarboxylate (E96)

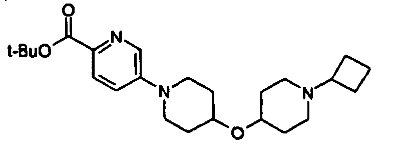

The title compound (E96) was prepared as described in Description 41.

### Example 97

### 1-[4-(4-{[1-(Methylethyl)-4-piperidinyl]oxy}-1-piperidinyl)phenyl]ethanone (E97)

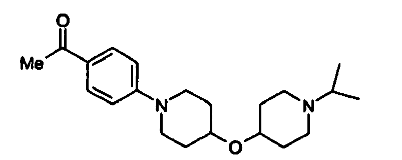

1-Isopropyl-4-(4-piperidinyloxy)piperidine (free base from D2) (0.5g), 4-fluoroacetophenone (0.81 ml) and potassium carbonate (1.22g) in acetonitrile (5ml) were heated at 120°C in a microwave reactor for 45min. The reaction mixture was then loaded onto an SCX column (10g) and washed with methanol (100ml) then eluted with 2M ammonia in methanol (100ml). After evaporation the residue was purified by chromatography [silica gel, eluting with 10%NH₃ in MeOH/DCM, 0-10%]. Pooling of pure fractions containing the faster running component afforded the title compound (E97) (0.075g). MS electrospray (+ve ion) 345 (MH⁺). ¹H NMR δ (CDCl₃): 7.85 (2H, d, J=9.0Hz), 6.86 (2H, d, J=9.0), 3.65 (3H, m), 3.51 (1H, m), 3.15 (2H, m), 2.86 (3H, m), 2.51 (3H, s), 2.42 (2H, m), 1.95 (4H, m), 1.66 (4H, m), 1.12 (6H, d, J=6.5Hz).

### Example 98

### N-(4-{4-[(1-Cyclobutyl-4-piperidinyl)oxy]-1-piperidinyl}-2-fluorophenyl)acetamide hydrochloride (E98)

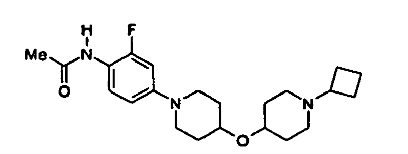

1-Cyclobutyl-4-(4-piperidinyloxy)piperidine (D6) (0.15g), *N*-(4-bromo-2-fluorophenyl)acetamide (D47) (0.14g), sodium *tert*-butoxide (0.086g), and acetato(2'-di-t-butylphosphino-1,1'-biphenyl-2-yl)palladium(II) (0.006g) were heated in toluene (3ml) at 55°C overnight. The reaction mixture was then treated with isocyanate resin (PS, Argonaut, 1.0g) and stirred at 55°C for 2h. The reaction was filtered and chromatographed [silica gel (10g cartridge), eluting with 10%NH₃ in MeOH/DCM, 0-10%]. Pure fractions were evaporated and the free base was dissolved in DCM and converted into the HCl salt with excess 1M HCl in diethyl ether and then evaporated to give the title compound (E98) as a pale yellow solid (0.018g). MS electrospray (+ve ion) 390 (MH⁺). ¹H NMR δ (DMSO-d6): 10.18 (1H, s), 9.43 (1H, s), 7.49 (1H, m), 6.88-6.77 (2H, m), 3.80-3.27 (4H, m), 3.18-2.65 (7H, m), 2.33-1.46 (17H, m).

### Examples 99-100 (E99-100)

Examples 99-100 were prepared in a similar manner to Example 86 from either 1-isopropyl-4-(4-piperidinyloxy)piperidine (free base from D2) or 1-cyclobutyl-4-(4-piperidinyloxy) piperidine (D6) and 2-chloro-4-trifluoromethylpyrimidine. Compounds displayed ¹H NMR and mass spectral data that were consistent with structure.

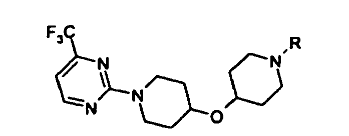

| **Example** | **R** | **Mass Spectrum** |
|---|---|---|
| | | **(ES⁺)** |
| **E99** | 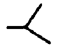 | [MH]⁺ 373 |
| **E100** | 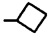 | [MH]⁺ 385 |

### Example 101

### 1-{4-[4-({1-[(1S)-1-Methylpropyl]-4-piperidinyl}oxy)-1-piperidinyl]phenyl}ethanone) (E101)

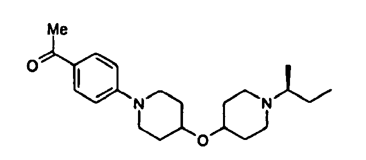

4-Fluoroacetophenone (0.15ml), 1-[(1S)-1-methylpropyl]-4-(4-piperidinyloxy)piperidine (D43) (0.20g), and potassium carbonate (0.23g) in DMSO (2ml) were heated in a microwave reactor at 160°C for 5min. The reaction was then poured directly onto an SCX column (10g) and washed with MeOH (60ml) then eluted with 2M ammonia in MeOH solution (60ml). After evaporation the residue was purified by chromatography [silica gel (20g cartridge), eluting with 10%NH₃ in MeOH/DCM, 0-10%]. Pure fractions were evaporated and the resultant oil was co-evaporated from acetone (3x) and dried under high vacuum to give the title compound (E101) as a yellow crystalline solid (0.16g). MS electrospray (+ve ion) 359 (MH⁺). ¹H NMR δ (CDCl₃): 7.86 (2H, d, J=9.2Hz), 6.86 (2H, d, J=8.8), 3.68 (3H, m), 3.40 (1H, m), 3.12 (2H, m), 2.72 (2H, m), 2.51 (3H, s), 2.46 (1H, m), 2.32 (1H, m), 2.21 (1H, m), 1.89 (4H, m), 1.72-1.48 (5H, m), 1.26 (1H, m), 0.96 (3H, d, J=6.8Hz), 0.88 (3H, t, J=7.2Hz). [α]D _{29.2°C} = +1.428 (c 0.14, methanol).

### Example 102

### 1-{4-[4-({1-[(1R)-1-Methylpropyl]-4-piperidinyl}oxy)-1-piperidinyl]phenyl}ethanone) (E102)

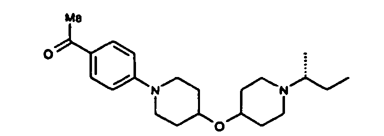

4-Fluoroacetophenone (0.15ml), 1-[(1R)-1-methylpropyl]-4-(4-piperidinyloxy)piperidine (D44) (0.20g), and potassium carbonate (0.23g) in DMSO (2ml) were heated in a microwave reactor at 160°C for 5min. The reaction mixture was then poured directly onto an SCX column (10g) and washed with MeOH (60ml) and then eluted with 2M ammonia in MeOH solution (60ml). After evaporation the residue was purified by chromatography [silica gel (20g cartridge), eluting with 10%NH₃ in MeOH/DCM, 0-10%]. Pure fractions were evaporated and the resultant oil was co-evaporated from acetone (3 x) and dried under high vacuum to give an oil which was triturated with diethyl ether, and dried under high vacuum at 30°C to give the title compound (E102) as a yellow crystalline solid (0.15g). MS electrospray (+ve ion) 359 (MH⁺). ¹H NMR δ (CDCl₃): 7.86 (2H, d, J=9.2Hz), 6.86 (2H, d, J=8.8), 3.68 (3H, m), 3.40 (1H, m), 3.12 (2H, m), 2.72 (2H, m), 2.51 (3H, s), 2.46 (1H, m), 2.32 (1H. m), 2.21 (1H, m), 1.89 (4H, m), 1.72-1.48 (5H, m), 1.26 (1H, m), 0.96 (3H, d, J=6.8Hz), 0.88 (3H, t, J=7.2Hz). [α]D _{29.2°C} = -1.818 (c 0.165, methanol).

### Example 103

### 1-[4-(4-{[1-(Cyclopropylmethyl)-4-piperidinyl]oxy}-1-piperidinyl)phenyl] ethanone (E103)

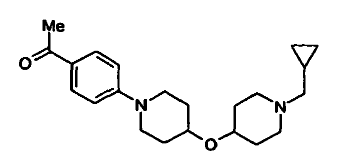

4-Fluoroacetophenone (0.15g), 1-cyclopropylmethyl-4-(4-piperidinyloxy)piperidine (D46) (0.20g), and potassium carbonate (0.23g) in DMSO (2.0ml) were heated in a microwave reactor at 160°C for 5min. The reaction was then poured directly onto an SCX column (10g) and washed with MeOH (60ml) and then eluted with 2M ammonia in MeOH solution (60ml). After evaporation the residue was purified by chromatography [silica gel (20g cartridge), eluting with 10%NH₃ in MeOH/DCM, 0-10%]. Pure fractions were evaporated to give the title compound (E103) as a pale yellow crystalline solid (0.17g). MS electrospray (+ve ion) 357 (MH⁺). ¹H NMR δ (CDCl₃): 7.86 (2H, d, J=8.8Hz), 6.86 (2H, d, J=9.2Hz), 3.70 (3H, m), 3.47 (1H, m), 3.12 (2H, m), 2.88 (2H, m), 2.51 (3H, s), 2.22 (4H, m), 1.91 (4H, m), 1.66 (4H, m), 0.87 (1H, m), 0.51 (2H, m), 0.09 (2H, m).

### Example 104

### N-Cyclobutyl-5-{4-[(1-cyclobutyl-4-piperidinyl)oxy]-1-piperidinyl}-2-pyridinecarboxamide (E104)

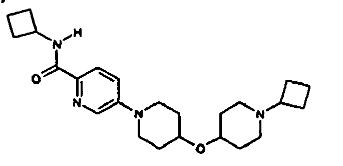

A portion of the acid chloride from Example 40 (0.62g) was suspended in DCM (5ml) and added dropwise over 15min to a solution of cyclobutylamine (1.29ml) in DCM (5ml) at 0-5°C. The resulting mixture was stirred at rt for 2.0h and then loaded directly onto silica (20g cartridge) and chromatographed [silica gel, eluting with 10%NH₃ in MeOH/DCM, 0-10%]. Pooling of pure fractions afforded the title compound (E104)as a pale brown solid (0.21g). MS electrospray (+ve ion) 413 (MH⁺). ¹H NMR δ (CDCl₃): 8.16 (1H, d, J=2.8Hz), 8.01 (1H, d, J=8.8Hz), 7.91 (1H, d, J=8.0Hz), 7.20(1H, dd, J=2.8Hz, J=8.8Hz), 4.58 (1H. m), 3.63 (3H, m), 3.45 (1H, m), 3.11 (2H, m), 2.66 (3H, m), 2.41 (2H, m), 2.10-1.53 (20H, m).

### Example 105

### N-Cyclobutyl-5-(4-{[1-(1-methylethyl)-4-piperidinyl]oxy}-1-piperidinyl)-2-pyridinecarboxamide (E105)

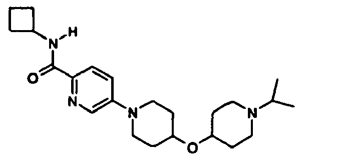

The title compound (E105) was prepared in a similar manner to Example 38 from 1-isopropyl-4-(4-piperidinyloxy)piperidine (free base from D2) and 5-bromo-N-cyclobutyl-2-pyridinecarboxamide (D54). MS electrospray (+ve ion) 401 (MH⁺).

### Example 106

### N-(3-Chloropropyl)-4-(4-{[1-(1-methylethyl)-4-piperidinyl]oxy}-1-piperidinyl)benzamide hydrochloride (E106)

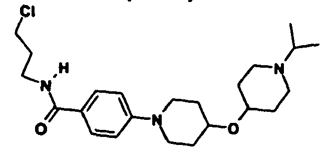

The title compound (E106) was obtained in a similar manner to Example 15 from 1-isopropyl-4-(4-piperidinyloxy)piperidine (free base from D2) and 1-(4-fluorobenzoyl)-azetidine (D48) followed by treatment of the free base azetidine product with HCl.

### Example 107

### N-(3-Chloropropyl)-5-{4-[(1-cyclobutyl-4-piperidinyl)oxy]-1-piperidinyl}-2-pyridinecarboxamide hydrochloride (E107)

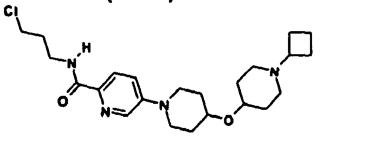

Reaction of 1-cyclobutyl-4-(4-piperidinyloxy)piperidine (D6) and 2-(1-azetidinylcarbonyl)-5-bromopyridine (D49) following the method described in Example 31 followed by treatment of the free base azetidine product with HCl afforded the title compound (E107).

### Example 108

### N-(3-Chloropropyl)-5-(4-{[1-(1-methylethyl)-4-piperidinyl]oxy}-1-piperidinyl)-2-pyridinecarboxamide hydrochloride (E108)

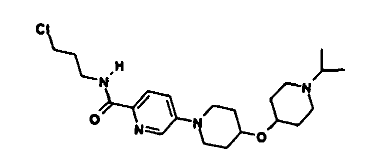

Reaction of 1-isopropyl-4-(4-piperidinyloxy)piperidine (free base from D2) and 2-(1-azetidinylcarbonyl)-5-bromopyridine (D49) following the method described in Example 31 followed by treatment of the free base azetidine product with HCl afforded the title compound (E108).

### Example 109

### 1-(4-{4-[(1-Cyclobutyl-4-piperidinyl)oxy]-1-piperidinyl}-3-fluorophenyl)ethanone (E109)

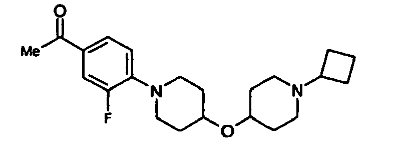

1-Cyclobutyl-4-(4-piperidinyloxy)piperidine (D6) (0.20g), 3,4-difluoroacetophenone (0.197g) and anhydrous potassium carbonate (0.116g) in DMSO (2ml) were heated at 160°C for 15min in an Emrys^{™} Optimizer microwave reactor. The crude reaction mixture was passed through an SCX cartridge [20g, eluting with MeOH (80ml) then 2N NH₃ in MeOH (80ml)]. Purification by chromatography [silica gel, eluting with (2N NH₃ in MeOH)/DCM, 0-20%] afforded the title compound (E109) as a crystalline solid (0.125g). MS electrospray (+ion) 375 (MH⁺). ¹H NMR δ (CDCl₃): 7.65 (1H, dd, J=8.4, 2.0Hz), 7.60 (1H, dd, J=14.0, 2.0Hz), 6.92 (1H, t, J=8.4Hz), 3.66-3.58 (1H, m), 3.52-3.40 (3H, m), 2.99 (2H, ddd, J=12.0, 8.8, 2.8Hz), 2.70-2.61 (3H, m), 2.52 (3H, s), 2.04-1.84 (9H, m) and 1.78-1.59 (7H, m obscured by H₂O).

### Example 110

### 1-Cyclobutyl-4-({1-[3-fluoro-4-(3-methyl-1,2,4-oxadiazol-5-yl)phenyl]-4-piperidinyl}oxy)piperidine (E110)

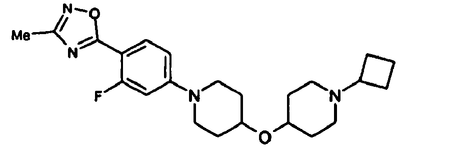

Sodium *tert*-butoxide (0.113g) was added to a solution of 1-cyclobutyl-4-(4-piperidinyloxy)piperidine (D6) (0.223g), 5-(4-bromo-2-fluorophenyl)-3-methyl-1,2,4-oxadiazole (D50) (0.20g) and acetato(2'-di-*tert*-butylphosphino-1,1'-biphenyl-2-yl)palladium(II) (0.017g) in toluene (10ml). The reaction was heated to 85°C overnight, then a further charge of acetato(2'-di-*tert*-butylphosphino-1,1'-biphenyl-2-yl)palladium(II) (0.017g) was added followed by heating at 85°C for a further 5h. The crude mixture was passed through an SCX cartridge [10g, eluting with MeOH (80ml) then 2N NH₃ in MeOH (80ml)]. Purification by chromatography [silica gel, eluting with (2N NH₃ in MeOH)/DCM, 0-20%] afforded the title compound (E110) (0.180g). MS electrospray (+ion) 415 (MH⁺). ¹H NMR δ (CDCl₃): 7.89 (1H, t, J=8.4Hz), 6.71 (1H, dd, J=8.8, 2.4Hz), 6.62 (1H, dd, J=14.8, 2.4Hz), 3.69-3.64 (3H, m), 3.49 (2H, br s), 3.12 (2H, ddd, J=12.4, 8.8, 3.6Hz), 2.78-2.63 (3H, m), 2.46 (3H, s), 2.10-2.02 (3H, m), 1.93-1.88 (6H, m) and 1.71-1.62 (6H, m, obscured by H₂O).

### Example 111

### 1-(5-{4-[(1-Cyclobutyl-4-piperidinyl)oxy]-1-piperidinyl}-2-pyridinyl)ethanone (E111)

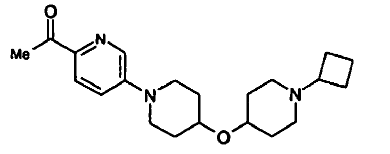

5-{4-[(1-Cyclobutyl-4-piperidinyl)oxy]-1-piperidinyl}-2-pyridinecarbonitrile (free base from E47) (0.155g) was dissolved in THF (4ml) and cooled to 0°C under argon. MeMgBr (3.1ml of a 3M solution in Et₂O, 20 equivalents) was added and the reaction mixture allowed to warm to rt and stirred until all the starting material was consumed (monitored by LC/MS). The reaction was quenched by the addition of saturated ammonium chloride solution (5ml), filtered through celite and evaporated. Purification by chromatography [silica gel, eluting with (2N NH₃ in MeOH)/DCM, 0-10%] afforded the title compound (E111) as a crystalline solid (0.089g). MS electrospray (+ion) 357 (MH⁺). ¹H NMR δ (CDCl₃): 8.27 (1H, d, J=3.0Hz), 7.94 (1H, d, J=9.0Hz), 7.15 (1H, dd, J=9.0, 3.0Hz), 3.7-3.61 (4H, m), 3.21 (2H, ddd, J=12.4, 8.4, 3.2Hz), 2.93 (1H, br s), 2.8-2.74 (2H, m), 2.64 (3H, s), 2.41 (2H, br s), 2.22 (2H, br s), 2.12-2.08 (4H, m), 1.97-1.90 (2H, m) and 1.85-1.65 (6H, m).

### Example 112

### 1-[2-Fluoro-4-(3-methyl-1,2,4-oxadiazol-5-yl)phenyl]-4-{[1-(1-methylethyl)-4-piperidinyl]oxy}piperidine hydrochloride (E112)

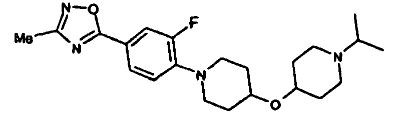

1-Isopropyl-4-(4-piperidinyloxy)piperidine (free base from D2) (0.25g) and 5-(4-bromo-3-fluorophenyl)-3-methyl-1,2,4-oxadiazole (D51) (0.31g) in dry toluene (10ml) were charged with acetato(2'-di-t-butylphosphino-1,1'-biphenyl-2-yl)palladium (II) (60mg) and sodium *tert*-butoxide (0.15g). The reaction mixture was heated at 90°C under a blanket of argon overnight. After cooling the reaction was diluted with MeOH (10ml) and then poured directly onto an SCX column (10g) and washed with MeOH (60ml) and then eluted with 2M ammonia in MeOH solution (60ml). After evaporation the residue was purified by chromatography [silica gel (20g cartridge), eluting with 10% NH₃ in MeOH/DCM, 0-10%]. Pure fractions were evaporated to give the free base compound which was dissolved in dry DCM (2ml) and treated with 1M HCl in diethyl ether (1ml).

The solvents were evaporated to dryness and the hydrochloride salt was crystallised from ethanol to give the title compound (E112) as a white crystalline solid (29mg). (MS electrospray (+ve ion) 403 (MH⁺). ¹H NMR δ (methanol-d4): 7.81-7.84 (1H, br d, J=8.4Hz), 7.70-7.74 (1H, br d, J=13.6Hz), 7.14 -7.19 (1H, m), 3.96-3.98 (1H, m), 3.65-3.80 (2H, m), 3.47-3.58 (4H, m), 3.05-3.13, (3H, m), 2.4 (3H, s), 1.9-2.32 (6H, m), 1.71-1.76 (3H, m), 1.34-1.38 (6H, m).

### Example 113

### 1-[3-Fluoro-4-(3-methyl-1 ,2,4-oxadiazol-5-yl)phenyl]-4-{[1-(1-methylethyl)-4-piperidinyl]oxy}piperidine hydrochloride (E113)

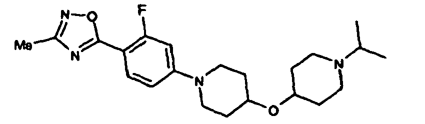

5-(4-Bromo-2-fluoro-phenyl)-3-methyl-(1,2,4)oxadiazole (D50) (0.31g) dissolved in dry and degassed dioxane (3ml) was charged with tris(dibenzylideneacetone)dipalladium(0) (40mg) and 2-dicyclohexylphosphino-2'-(N,N-dimethylamino)biphenyl (70mg). The dark solution was stirred at rt under argon for 15min, followed by the addition 1-isopropyl-4-piperidinyloxy)piperidine (free base from D2) (0.25g) in dioxane (1ml) and potassium phosphate (0.41g). The reaction mixture was stirred at 95°C for 2h and after cooling the crude reaction mixture was worked up as described for Example 112. The title compound (E113) was isolated as the hydrochloride salt (13mg). MS electrospray (+ve ion) 403 (MH⁺). ¹H NMR δ (methanol-d4): 7.87-7.92 (1H, dd, J=8.8Hz), 6.89-6.92 (1H, m), 6.80-6.85 (1H, m), 3.97-3.98 (1H, m), 3.70-3.80 (4H, m), 3.43-3.54 (2H, m), 3.05-3.20 (3H, m), 2.39 (3H, s), 1.96-2.32 (6H, m), 1.66-1.69 (3H, m), 1.35-1.38 (6H, m).

### Example 114

### 4-{4-[(1-Cyclobutyl-4-piperidinyl)oxy]-1-piperidinyl}-3-fluorobenzontrile (E114)

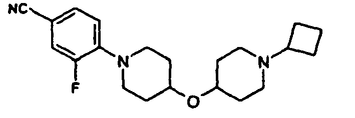

1-Cyclobutyl-4-(4-piperidinyloxy)piperidine (D6) (0.20g), 3,4-difluorobenzonitrile (0.175g) and anhydrous potassium carbonate (0.232g) in DMSO (2ml) were heated at 80°C for 5min in an Emrys^{™} Optimizer microwave reactor. The crude reaction mixture was passed through an SCX cartridge [20g, eluting with MeOH (80ml) then 2N NH₃ in MeOH (80ml)]. Evaporation of the ammonia fractions gave the title compound (E114) as a crystalline solid (0.225g). MS electrospray (+ion) 358 (MH⁺). ¹H NMR δ (CDCl₃): 7.34 (1H, dd, J=8.4, 2.0Hz), 7.25 (1H, dd, J=14.0, 2.0Hz), 6.91 (1H, t, J=8.4Hz), 3.64-3.56 (1H, m), 3.49-3.43 (4H, m), 3.18-3.12 (1H, m), 3.00 (2H, ddd, J=12.4, 8.8, 3.6Hz), 2.83-2.78 (1H, m), 2.75-2.62 (3H, m), 2.07-1.89 (7H, m obscured by H₂O) and 1.77-1.71 (6H, m).

### Example 115

### 4-{4-[(1-Cyclobutyl-4-piperidinyl)oxy]-1-piperidinyl}-2-fluorobenzonitrile (E115)

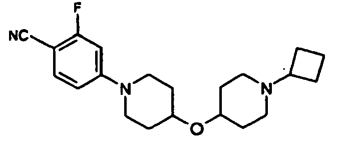

1-Cyclobutyl-4-(4-piperidinyloxy)piperidine (D6) (0.10g), 2,4-difluorobenzonitrile (0.098g) and anhydrous potassium carbonate (0.116g) in 1-methyl-2-pyrrolidinone (2ml) were heated at 60°C for 1.5min in an Emrys^{™} Optimizer microwave reactor. The crude reaction mixture was passed through an SCX cartridge [20g, eluting with MeOH (80ml) then 2N NH₃ in MeOH (80ml)]. Evaporation of the ammonia fractions gave the title compound (E115) (130mg). MS electrospray (+ion) 358 (MH⁺). ¹H NMR δ (CDCl₃): 7.55 (1H, dd, J=7.6, 1.2Hz), 6.60 (1H, dd, J= 8.8, 1.2Hz), 6.52 (1H, dd, J=13.2, 2.4Hz), 3.61-3.58 (2H, m), 3.50-3.40 (2H, m), 3.24-3.09 (2H, m), 2.81-2.58 (5H, m), 2.13-1.78 (7H, m), 1.77-1.49 (7H, m)

### Example 116

### 1-(4-{4-[(1-Cyclobutyl-4-piperidinyl)oxy]-1-piperidinyl}-2-ftuorophenyl)ethanone (E116)

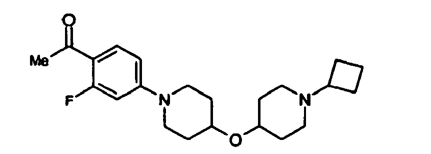

4-{4-[(1-Cyclobutyl-4-piperidinyl)oxy]-1-piperidinyl}-2-fluorobenzonitrile (E115) (0.20g), was dissolved in THF (5ml) and cooled to 0°C under argon. MeMgBr (3.7ml of a 3M solution in Et₂O, 20 equivalents) was added and the reaction mixture allowed to warm to rt and stirred until all the starting material was consumed (monitored by LC/MS). Reaction was quenched by the addition of saturated ammonium chloride solution (5ml), filtered through celite and evaporated. Purification by chromatography [silica gel, eluting with (2N NH₃ in MeOH)/DCM, 0-10%] afforded the title compound (E116) as a white solid (0.090g). MS electrospray (+ion) 375 (MH⁺). ¹H NMR δ (CDCl₃): 7.81 (1H, t, J=8.8Hz), 6.62 (1H, dd, J=10.0, 2.4Hz), 6.46 (1H, dd, J=15.2, 2.4Hz), 3.71-3.58 (4H, m), 2.99 (2H, ddd, J=12.4, 8.8, 3.2Hz), 2.88-2.77 (3H, m), 2.55 (3H, d, J=5.2Hz), 2.38-2.20 (1H, br s), 2.20-2.09 (1H, m) and 1.99-1.60 (14H, m).

### Example 117

### 4-{4-[(1-Cyclobutyl-4-piperidinyl)oxy]-1-piperidinyl}-2,5-difluorobenzonitrile (E117)

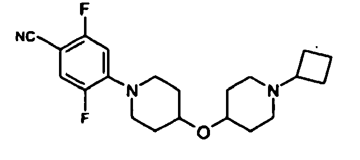

1-Cyclobutyl-4-(4-piperidinyloxy)piperidine (D6) (0.200g), 2,4,5-trifluorobenzonitrile (0.198g) and anhydrous potassium carbonate (0.232g) in DMSO (2ml) were heated at 80°C for 5min in an Emrys^{™} Optimizer microwave reactor. The crude reaction mixture was passed through an SCX cartridge [20g, eluting with MeOH (80ml) then 2N NH₃ in MeOH (80ml)]. Evaporation of the ammonia fractions gave the title compound (E117) as a crystalline solid (0.33g). MS electrospray (+ion) 376 (MH⁺). ¹H NMR δ (CDCl₃): 7.18-7.13 (1H, app q, J=6.0Hz), 6.30 (1H, dd, H=11.2, 7.2Hz), 3.64 (1H, app sept, J=4Hz), 3.51-3.45 (3H, m), 3.16 (2H, ddd, J=12.0, 8.4, 3.6Hz), 2.75-2.62 (3H, m), 2.07-1.99 (4H, m), 1.97-1.85 (6H, m) and 1.80-1.60 (6H, m).

### Example 118

### 1-(4-{4-[(1-Cyclobutyl-4-piperidinyl)oxy]-1-piperidinyl}-2,5-difluorophenyl)ethanone (E118)

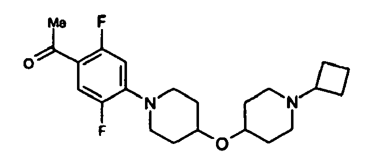

4-{4-[(1-Cyclobutyl-4-piperidinyl)oxy]-1-piperidinyl}-2,5-difluorobenzonitrile (E117) (0.315g) was dissolved in THF (10m) and cooled to 0°C under argon. MeMgBr (5.3m) of a 3M solution in Et₂O, 20 equivalents) was added and the reaction mixture allowed to warm to rt and stirred until all the starting material was consumed (monitored by LC/MS). The reaction was quenched by the addition of saturated ammonium chloride solution (10ml), filtered through celite and evaporated. Purification by chromatography [silica gel, eluting with (2N NH₃ in MeOH)/DCM, 0-10%] gave the title compound (E118) as a white solid (0.13g). MS electrospray (+ion) 393 (MH⁺). ¹H NMR δ (CDCl₃): 7.55 (1H, q, J=7.2, 13.8Hz), 6.56 (1H, q, J=7.2, 13.0Hz), 3.62 (1H, m), 3.05 (2H, m), 3.50 (3H, m), 2.87-2.61 (3H, m), 3.50 (3H, d, J=5.2Hz), 2.22-1.89 (9H, m), 1.79-1.60 (7H, m).

### Example 119

### 1-[3-Fluoro-4-(3-methyl-1,2,4-oxadiazol-5-yl)phenyl]-4-({1-[(1S)-1-methylpropyl]-4-piperidinyl}oxy)piperidine (E119)

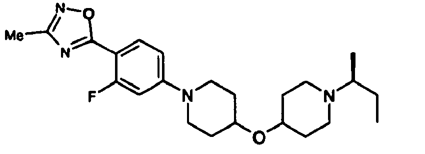

Sodium *tert*-butoxide (0.113g) was added to a solution of 1-[(1 S}-1-methylpropyl]-4-(4-piperidinyloxy)piperidine (D43) (0.225g), 5-(4-bromo-2-fluorophenyl)-3-methyl-1,2,4-oxadiazole (D50) (0.20g) and acetato(2'-di-*tert*-butylphosphino-1,1'-biphenyl-2-yl)palladium(11) (0.017g) in toluene (10ml). The reaction was heated to 85°C overnight, then a further charge of acetato(2'-di-*tert*-butylphosphino-1,1'-biphenyl-2-yl)palladium(II) (0.017g) was added followed by heating at 85°C for a further 5h. The crude mixture was passed through an SCX cartridge [10g, eluting with MeOH (80ml) then 2N NH₃ in MeOH (80ml)]. Purification by chromatography [silica gel, eluting with (2N NH₃ in MeOH)/DCM, 0-20%] afforded the title compound (E119) (0.084g). MS electrospray (+ion) 417 (MH⁺). ¹H NMR δ (CDCl₃): 7.89 (1H, t, J=8.8Hz), 6.71 (1H, dd, J=9.2, 2.4Hz), 6.62 (1H, dd, J=14.4, 2.4Hz), 3.69-3.64 (3H, m), 3.48-3.39 (1H, m), 3.18 (2H, ddd, J=12.8, 8.8, 3.6Hz), 2.80-2.69 (2H, m), 2.46 (3H, s), 2.40-2.32 (1H, m), 2.29-2.0 (1H, m), 1.96-1.86 (4H, m), 1.73-1.56 (6H, m, obscured by H₂O), 1.27 (1H, app sept, J=7.6Hz), 0.97 (3H, d, J=6.4Hz) and 0.89 (3H, t, J=7.6Hz).

### Example 120

### N-Methyl-5-(4-{[1-(1-methylethyl)-4-piperidinyl]oxy}-1-piperidinyl)-2-pyridinecarboxamide (E120)

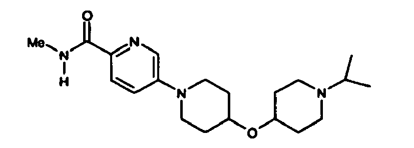

DMF (3 drops) was added to a suspension of 5-(4-{[1-(1-methylethyl)-4-piperidinyl]oxy}-1-piperidinyl)-2-pyridinecarboxylic acid (D53) (0.33g) and oxalyl chloride (1ml) in DCM (5ml) at rt. The reaction mixture was stirred for 1h, after which time the mixture was evaporated to give the crude acid chloride as a dark green solid. This was dissolved in DCM (15ml) and added dropwise to a cooled (5°C) solution of methylamine (5ml of a 2M solution in THF) over a period of 1h. After the addition was completed the reaction was allowed to stir for a further 15min and then evaporated to dryness. The crude amide was dissolved in saturated sodium hydrogen carbonate solution (5ml) and extracted into DCM (3×10ml). The organic phase was washed with saturated brine (10ml), dried (MgSO₄) and evaporated. Purification by chromatography [silica gel, eluting with (2N NH₃ in MeOH)/DCM, 0-20%] followed by evaporation afforded a mixture of free base and HCl salt. The mixture was dissolved in DCM (5ml) and washed with saturated potassium carbonate solution (15ml), dried (MgSO₄) and evaporated to give the title compound (E120) as a pale yellow crystalline solid (0.15g). MS electrospray (+ion) 361 (MH⁺). ¹H NMR δ (CDCl₃): 8.08 (1H, d, J=2.8Hz), 7.94 (1H, d, J=8.8Hz), 7.68 (1H, d, J=4.8Hz), 7.13 (1H, dd, J=8.8, 2.8Hz), 3.60-3.53 (3H, m), 3.37 (1H, ddd, J=12.4, 8.4, 3.8Hz), 3.05 (2H, ddd, J=12.4, 8.8, 3.2Hz), 2.93 (3H, d, J=4.8Hz), 2.74-2.69 (2H, m), 2.66 (1H, sep, J=6.4Hz), 2.22-2.16 (2H, m), 1.90-1.78 (4H, m), 1.67-1.49 (4H, m) and 0.97 (6H, d, J=6.4Hz).

### Abbreviations

- BINAP: 2,2'-bis(diphenylphosphino)-1,1'-binaphthyl
- DCM: dichloromethane
- DMF: N,N-dimethylformamide
- DIPEA: diisopropylethylamine
- DMSO: dimethylsulfoxide
- EDC: 1-(3-dimethylaminopropyl)-3-ethylcarbodiimide hydrochloride
- EtOAc: ethyl acetate
- HOAT: 1-hydroxy-7-azabenzotriazole
- HOBT: 1-hydroxybenzotriazole
- h: hour
- min: minutes
- rt: room temperature
- TFA: trifluoroacetic acid
- THF: tetrahydrofuran
- SCX: strong cation exchange
- MP-NCO: macroporous polystyrene isocyanate resin

### Biological Data

A membrane preparation containing histamine H3 receptors may be prepared in accordance with the following procedures:

### (i) Generation of histamine H3 cell line

DNA encoding the human histamine H3 gene (Huvar, A. *et al.* (1999) Mol. Pharmacol. **55(6)**, 1101-1107) was cloned into a holding vector, pCDNA3.1 TOPO (InVitrogen) and its cDNA was isolated from this vector by restriction digestion of plasmid DNA with the enzymes BamH1 and Not-1 and ligated into the inducible expression vector pGene (InVitrogen) digested with the same enzymes. The GeneSwitch^{™} system (a system where in transgene expression is switched off in the absence of an inducer and switched on in the presence of an inducer) was performed as described in US Patent nos: 5.364,791; 5,874,534; and 5,935,934. Ligated DNA was transformed into competent DH5α E. coli host bacterial cells and plated onto Luria Broth (LB) agar containing Zeocin^{™} (an antibiotic which allows the selection of cells expressing the sh ble gene which is present on pGene and pSwitch) at 50µg ml⁻¹. Colonies containing the re-ligated plasmid were identified by restriction analysis. DNA for transfection into mammalian cells was prepared from 250ml cultures of the host bacterium containing the pGeneH3 plasmid and isolated using a DNA preparation kit (Qiagen Midi-Prep) as per manufacturers guidelines (Qiagen).
CHO K1 cells previously transfected with the pSwitch regulatory plasmid (InVitrogen) were seeded at 2x10e6 cells per T75 flask in Complete Medium, containing Hams F12 (GIBCOBRL, Life Technologies) medium supplemented with 10% v/v dialysed foetal bovine serum, L-gtutamine, and hygromycin (100µg ml⁻¹), 24 hours prior to use. Plasmid DNA was transfected into the cells using Lipofectamine plus according to the manufacturers guidelines (In Vitrogen). 48 hours post transfection cells were placed into complete medium supplemented with 500µg ml⁻¹ Zeocin^{™}.
10-14 days post selection 10nM Mifepristone (In Vitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1x 10e7 cells were examined for receptor expression by staining with a rabbit polyclonal antibody, 4a, raised against the N-terminal domain of the histamine H3 receptor, incubated on ice for 60 minutes, followed by two washes in sorting medium. Receptor bound antibody was detected by incubation of the cells for 60 minutes on ice with a goat anti rabbit antibody, conjugated with Alexa 488 fluorescence marker (Molecular Probes). Following two further washes with Sorting Medium, cells were filtered through a 50µm Filcon^{™} (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500µg ml⁻¹ Zeocin^{™} and allowed to expand before reanalysis for receptor expression via antibody and ligand binding studies. One clone, 3H3, was selected for membrane preparation.

### (ii) Membrane preparation from cultured cells

All steps of the protocol are carried out at 4°C and with pre-cooled reagents. The cell pellet is resuspended in 10 volumes of buffer A2 containing 50mM N-2-hydroxyethylpiperazine-N'-2-ethanesulfonic acid (HEPES) (pH 7.40) supplemented with 10e-4M leupeptin (acetyl-leucyl-leucyl-arginal; Sigma L2884), 25µg/ml bacitracin (Sigma B0125). 1mM ethylenediamine tetra-acetic acid (EDTA), 1mM phenylmethylsulfonyl fluoride (PMSF) and 2x10e-6M pepstain A (Sigma). The cells are then homogenised by 2 x 15 second bursts in a 1 litre glass Waring blender, followed by centrifugation at 500g for 20 minutes. The supernatant is then spun at 48,000g for 30 minutes. The pellet is resuspended in 4 volumes of buffer A2 by vortexing for 5 seconds, followed by homogenisation in a Dounce homogeniser (10-15 strokes). At this point the preparation is aliquoted into polypropylene tubes and stored at -70°C.

Compounds of the invention may be tested for in vitro biological activity in accordance with the following assays:

### (I) Histamine H3 binding assay

For each compound being assayed, in a white walled clear bottom 96 well plate, is added:-
(a) 10µl of test compound (or 10µl of iodophenpropit (a known histamine H3 antagonist) at a final concentration of 10mM) diluted to the required concentration in 10% DMSO;
(b) 10µl ¹²⁵I 4-[3-(4-iodophenylmethoxy)propyl]-1H-imidazolium (iodoproxyfan) (Amersham; 1.85MBq/µl or 50µCi/ml; Specific Activity ~2000Ci/mmol) diluted to 200pM in assay buffer (50mM Tris(hydroxymethyl)aminomethane buffer (TRIS) pH 7.4, 0.5mM ethylenediamine tetra-acetic acid (EDTA)) to give 20pM final concentration; and
(c) 80µl bead/membrane mix prepared by suspending Scintillation Proximity Assay (SPA) bead type WGA-PVT at 100mg/ml in assay buffer followed by mixing with membrane (prepared in accordance with the methodology described above) and diluting in assay buffer to give a final volume of 80µl which contains 7.5µg protein and 0.25mg bead per well- mixture was pre-mixed at room temperature for 60 minutes on a roller. The plate is shaken for 5 minutes and then allowed to stand at room temperature for 3-4 hours prior to reading in a Wallac Microbeta counter on a 1 minute normalised tritium count protocol. Data is analysed using a 4-parameter logistic equation.

### (II) Histamine H3 functional antagonist assay

For each compound being assayed, in a white walled clear bottom 96 well plate, is added:-
(a) 10µl of test compound (or 10µl of guanosine 5'- triphosphate (GTP) (Sigma) as non-specific binding control) diluted to required concentration in assay buffer (20mM N-2-Hydroxyethylpiperazine-N'-2-ethanesulfonic acid (HEPES) + 100mM NaCl + 10mM MgCl₂, pH7.4 NaOH);
(b) 60µl bead/membrane/GDP mix prepared by suspending wheat germ agglutinin-polyvinyltoluene (WGA-PVT) scintillation proximity assay (SPA) beads at 100mg/ml in assay buffer followed by mixing with membrane (prepared in accordance with the methodology described above) and diluting in assay buffer to give a final volume of 60µl which contains 10µg protein and 0.5mg bead per well - mixture is pre-mixed at 4°C for 30 minutes on a roller and just prior to addition to the plate, 10µM final concentration of guanosine 5' diphosphate (GDP) (Sigma; diluted in assay buffer) is added;
   The plate is incubated at room temperature to equilibrate antagonist with receptor/beads by shaking for 30 minutes followed by addition of:
(c) 10µl histamine (Tocris) at a final concentration of 0.3µM; and
(d) 20µl guanosine 5' [γ35-S] thiotriphosphate, triethylamine salt (Amersham; radioactivity concentration = 37kBq/µl or 1mCi/ml; Specific Activity 1160Ci/mmol) diluted to 1.9nM in assay buffer to give 0.38nM final.
   The plate is then incubated on a shaker at room temperature for 30 minutes followed by centrifugation for 5 minutes at 1500 rpm. The plate is read between 3 and 6 hours after completion of centrifuge run in a Wallac Microbeta counter on a 1 minute normalised tritium count protocol. Data is analysed using a 4-parameter logistic equation. Basal activity used as minimum i.e. histamine not added to well.

### Results

The compounds of Examples E1-E113 were tested in the histamine H3 functional antagonist assay and exhibited pKᵢ values > 7.5. In particular, the compounds of Examples E1-E58, E60-E65, E67, E69-E98 and E101-E113 exhibited pKᵢ values > 8.0. More particularly, the compounds of E2-E13, E15-E17, E21-E49, E54-E57, E62, E70-E82, E84-E86, E88-E98, E101-E102, E104-E113 exhibited pKᵢ values ≥ 9.0. Most particularly, the compounds of E17, E38, E48, E82 and E88 exhibited pKᵢ values > 9.5.

## Claims

1. A compound of formula (I) or a pharmaceutically acceptable salt thereof: 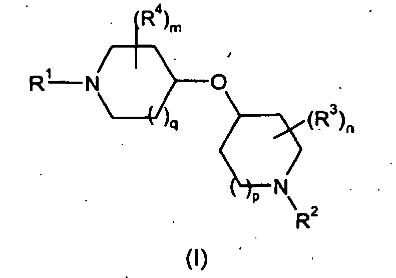 wherein:
R¹ represents aryl, heteroaryl,-aryl-X-aryl -aryl-X-heteroaryl, -aryl-X-heterocyclyl, -heteroaryl-X-heteroaryl, -heteroaryl-X-aryl or -heteroaryl-X-heterocyclyl;
wherein said aryl, heteroaryl and heterocyclyl groups of R¹ may be optionally substituted by one or more (e.g. 1, 2 or 3) substituents which may be the same or different, and which are selected from the group consisting of halogen, hydroxy, cyano, nitro, oxo, haloC₁₋₆ alkyl, polyhaloC₁₋₆ alkyl, haloC₁₋₆ alkoxy, polyhaloC₁₋₆ alkoxy, C₁₋₆ alkyl, C₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, C₁₋₆ alkylsulfonamidoC₁₋₆ alkyl, C₁₋₆ alkylamidoC₁₋₆ alkyl, aryl, arylsulfonyl, arylsulfonyloxy, aryloxy, arylsulfonamido, arylcarboxamido, aroyl, or a group -COR¹⁵, -COOR¹⁵, NR¹⁵R¹⁶, -CONR¹⁵R¹⁶, -NR¹⁵COR¹⁶, -NR¹⁵SO₂R¹⁶ or -SO₂NR¹⁵R¹⁶, wherein R¹⁵ and R¹⁶ independently represent hydrogen, C₁₋₆ alkyl, haloC₁₋₆ alkyl, polyhaloC₁₋₆ alkyl or C₃₋₆ cycloalkyl or together form a heterocyclic ring;
X represents a bond, O, CO, SO₂, OCH₂ or CH₂O;
R² represents C₃₋₈ alkyl, C₃₋₆ alkenyl, C₃₋₆ alkynyl, C₃₋₆ cycloalkyl, C₅₋₆ cycloalkenyl or -C₁₋₄alkyl-C₃₋₆ cycloalkyl;
wherein said C₃₋₆ cycloalkyl groups of R² may be optionally substituted by one or more (e.g. 1, 2 or 3) substituents which may be the same or different, and which are selected from the group consisting of halogen, C₁₋₄ alkyl or trifluoromethyl groups;
each R³ and R⁴ group independently represents C₁₋₄ alkyl;
m and n independently represent 0, 1 or 2; and
p and q independently represent 1 or 2.

2. A compound of formula (I) as defined in claim 1 wherein R¹ represents
- aryl optionally substituted by a cyano, -CONR¹⁵R¹⁶, -COR¹⁵, halogen or -NR¹⁵COR¹⁶;
- heteroaryl optionally substituted by a cyano, C₁₋₆ alkyl, polyhaloC₁₋₆ alkyl, -CONR¹⁵R¹⁶, -COR¹⁵ or -COOR¹⁵ group;
- aryl-X-heterocyclyl;
- aryl-X-heteroaryl optionally substituted by a halogen, C₁₋₆ alkyl or aryl; or
- heteroaryl-X-heterocyclyl.

3. A compound of formula (I) as defined in claim 2 wherein R¹ represents pyrid-3-yl optionally substituted by a -CONR¹⁵R¹⁶ group, -phenyl-1,2,4-oxadiazol-5-yl optionally substituted by a C₁₋₆ alkyl group, phenyl optionally substituted by a -COR¹⁵ group, pyridazin-3-yl optionally substituted by a polyhaloC₁₋₆ alkyl group, pyrazin-2-yl optionally substituted by a polyhaloC₁₋₆ alkyl group or pyrimidin-5-yl optionally substituted by a polyhaloC₁₋₆ alkyl group.

4. A compound of formula (I) as defined in claim 3 wherein R¹ represents pyrid-3-yl optionally substituted by a 6-CON(H)(Me) or 6-CON(H)(Et) group, 3-methyl-1,2,4-oxadiazol-5-yl, phenyl optionally substituted by a 4-COMe group, pyridazin-3-yl optionally substituted by a 6-CF₃ group or pyrimidin-5-yl optionally substituted by a 2-CF₃ group.

5. A compound of formula (I) as defined in any one of claims 1 to 4 wherein m and n represent 0.

6. A compound of formula (I) as defined in any one of claims 1 to 5 wherein p and q represent 1.

7. A compound of formula (I) as defined in any one of claims 1 to 6 wherein R² represents C₃₋₈ alkyl, C₃₋₆ cycloalkyl or -C₁₋₄alkyl-C₃₋₆ cycloalkyl.

8. A compound of formula (I) as defined in claim 7 wherein R² represents 1-methylpropyl, isopropyl, cyclobutyl or -CH₂-cyclopropyl.

9. A compound of formula (I) as defined in claim 8 wherein R² represents isopropyl or cyclobutyl.

10. A compound as defined in claim 1 which is:
6-(4-{[1-(1-methylethyl)-4-piperidinyl]oxy}-1-piperidinyl)-3-pyridinecarbonitrile;
6-{4-[(1-Cyclobutyl-4-piperidinyl)oxy]-1-piperidinyl}-3-pyridinecarbonitrile;
4-{4-[(1-Cyclobutyl-4-piperidinyl)oxy]-1-piperidinyl}benzonitrile;
4-(4-{[1-(1-Methylethyl)-4-piperidinyl]oxy}-1-piperidinyl)benzonitrile;
4-[(4-{4-[(1-Cyclobutyl-4-piperidinyl)oxy]-1-piperidinyl}phenyl)carbonyl]morpholine;
4-{4-[(1-Cyclobutyl-4-piperidinyl)oxy]-1-piperidinyl}-N-methylbenzamide;
4-{[4-(4-{[1-(1-Methylethyl)-4-piperidinyl]oxy}-1-piperidinyl)phenyl]carbonyl}morpholine;
1-(1-Methylethyl)-4-({1-[4-(1-piperidinylcarbonyl)phenyl]-4-piperidinyl}oxy)piperidine;
1-Cyclobutyl-4-({1-[4-(1-piperidinylcarbonyl)phenyl]-4-piperidinyl}oxy)piperidine;
1-(1-Methylethyl)-4-({1-[4-(1-pyrrolidinylcarbonyl)phenyl]-4-piperidinyl}oxy)piperidine;
1-Cyclobutyl-4-({1-[4-(1-pyrrolidinylcarbonyl)phenyl]-4-piperidinyl}oxy)piperidine;
N,N-Dimethyl-4-(4-{[1-(1-methylethyl)-4-piperidinyl]oxy}-1-piperidinyl)benzamide;
4-{4-[(1-Cyclobutyl-4-piperidinyl)oxy]-1-piperidinyl}-N,N-dimethylbenzamide;
N-Methyl-4-(4-{[1-(1-methylethyl)-4-piperidinyl]oxy}-1-piperidinyl)benzamide;
N-(3-Chloropropyl)-4-{4-[(1-cyclobutyl-4-piperidinyl)oxy]-1-piperidinyl}benzamide;
1-Cyclobutyl-4-({1-[4-(2-methyl-1,3-oxazol-4-yl)phenyl]-4-piperidinyl}oxy)piperidine;
1-(1-Methylethyl)-4-({1-[4-(3-methyl-1,2,4-oxadiazol-5-yl)phenyl]-4-piperidinyl}oxy)piperidine;
1-(1-Methylethyl)-4-({1-[4-(2-methyl-1,3-oxazol-4-yl)phenyl]-4-piperidinyl}oxy)piperidine;
1-(1-Methylethyl)-4-({1-[4-(1,3-oxazol-2-yl)phenyl]-4-piperidinyl}oxy)piperidine;
1-(1-Methylethyl)-4-({1-[4-(2-methyl-1,3-oxazol-5-yl)phenyl]-4-piperidinyl}oxy)piperidine;
1-Cyclobutyl-4-({1-[4-(1,3-oxazol-2-yl)phenyl]-4-piperidinyl}oxy)piperidine;
1-Cyclobutyl-4-({1-[4-(2-methyl-1,3-oxazol-5-yl)phenyl]-4-piperidinyl}oxy)piperidine;
1-(1-Methylethyl)-4-({1-[4-(3-methyl-5-isoxazolyl)phenyl]-4-piperidinyl}oxy)piperidine;
1-Cyclobutyl-4-({1-[4-(3-methyl-5-isoxazolyl)phenyl]-4-piperidinyl}oxy)piperidine;
1-Cyclobutyl-4-({1-[4-(5-methyl-1,2,4-oxadiazol-3-yl)phenyl]-4-piperidinyl}oxy)piperidine;
1-(1-Methylethyl)-4-({1-[4-(5-phenyl-1,3,4-oxadiazol-2-yl)phenyl]-4-piperidinyl}oxy)piperidine;
1-Cyclobutyl-4-({1-[4-(5-phenyl-1,3,4-oxadiazol-2-yl)phenyl]-4-piperidinyl}oxy)piperidine;
1-Cyclobutyl-4-({1-[4-(3-methyl-1,2,4-oxadiazol-5-yl)phenyl]-4-piperidinyl}oxy)piperidine;
1-Cyclobutyl-4-({1-[4-(1,3-oxazol-5-yl)phenyl]-4-piperidinyl}oxy)piperidine;
1-(1-Methylethyl)-4-({1-[4-(1,3-oxazol-5-yl)phenyl]-4-piperidinyl}oxy)piperidine;
5-{4-[(1-Cyclobutyl-4-piperidinyl)oxy]-1-piperidinyl}-N,N-dimethyl-2-pyridinecarboxamide;
N,N-Dimethyl-5-(4-{[1-(1-methylethyl)-4-piperidinyl]oxy}-1-piperidinyl)-2-pyridinecarboxamide;
5-(4-{[1-(1-Methylethyl)-4-piperidinyl]oxy}-1-piperidinyl)-2-(1-pyrrolidinylcarbonyl)pyridine;
5-(4-{[1-(1-Methylethyl)-4-piperidinyl]oxy}-1-piperidinyl)-2-(1-piperidinylcarbonyl)pyridine;
5-{4-[(1-Cyclobutyl-4-piperidinyl)oxy]-1-piperidinyl}-2-(1-piperidinylcarbonyl)pyridine;
4-{[5-(4-{[1-(1-Methylethyl)-4-piperidinyl]oxy}-1-piperidinyl)-2-pyridinyl]carbonyl}morpholine;
4-[(5-{4-[(1-Cyclobutyl-4-piperidinyl)oxy]-1-piperidinyl}-2-pyridinyl)carbonyl]morpholine;
5-{4-[(1-Cyclobutyl-4-piperidinyl)oxy]-1-piperidinyl}-N-methyl-2-pyridinecarboxamide;
N-Ethyl-5-(4-{[1-(1-methylethyl)-4-piperidinyl]oxy}-1-piperidinyl)-2-pyridinecarboxamide;
5-{4-[(1-Cyclobutyl-4-piperidinyl)oxy]-1-piperidinyl}-N-ethyl-2-pyridinecarboxamide;
5-(4-{[1-(1-Methylethyl)-4-piperidinyl]oxy}-1-piperidinyl)-N-propyl-2-pyridinecarboxamide;
5-{4-[(1-Cyclobutyl-4-piperidinyl)oxy]-1-piperidinyl}-N-propyl-2-pyridinecarboxamide;
N-(1-Methylethyl)-5-(4-{[1-(1-methylethyl)-4-piperidinyl]oxy}-1-piperidinyl)-2-pyridinecarboxamide;
5-{4-[(1-Cyclobutyl-4-piperidinyl)oxy]-1-piperidinyl}-N-(1-methylethyl)-2-pyridinecarboxamide;
N-Cyclopentyl-5-(4-{[1-(1-methylethyl)-4-piperidinyl]oxy}-1-piperidinyl)-2-pyridinecarboxamide;
5-{4-[(1-Cyclobutyl-4-piperidinyl)oxy]-1-piperidinyl}-N-cyclopentyl-2-pyridinecarboxamide;
5-{4-[(1-Cyclobutyl-4-piperidinyl)oxy]-1-piperidinyl}-2-pyridinecarbonitrile;
1-(4-{4-[(1-Cyclobutyl-4-piperidinyl)oxy]-1-piperidinyl}phenyl)ethanone;
5-{4-[(1-Cyclobutyl-4-piperidinyl)oxy]-1-piperidinyl}-2-(trifluoromethyl)pyridine;
N-Methyl-6-(4-{[1-(1-methylethyl)-4-piperidinyl]oxy}-1-piperidinyl)-3-pyridinecarboxamide;
N, N-Dimethyl-6-(4-{[1-(1-methylethy)-4-piperidinyl]oxy}-1-piperidinyl)-3-pyridinecarboxamide;
2-(4-{[1-(1-Methylethyl)-4-piperidinyl]oxy}-1-piperidinyl)-5-(1-pyrrolidinylcarbonyl)pyridine;
4-{[6-(4-{[1-(1-Methylethyl)-4-piperidinyl]oxy}-1-piperidinyl)-3-pyridinyl]carbonyl}morpholine;
6-{4-[(1-Cyclobutyl-4-piperidinyl)oxy]-1-piperidinyl}-N-methyl-3-pyridinecarboxamide;
6-{4-[(1-Cyclobutyl-4-piperidinyl)oxy]-1-piperidinyl}-N,N-dimethyl-3-pyridinecarboxamide;
2-{4-[(1-Cyclobutyl-4-piperidinyl)oxy]-1-piperidinyl}-5-(1-pyrrolidinylcarbonyl)pyridine;
4-[(6-{4-[(1-Cyclobutyl-4-piperidinyl)oxy]-1-piperidinyl}-3-pyridinyl)carbonyl]morpholine;
N-Methyl-6-(4-{[1-(1-methylethyl)-4-piperidinyl]oxy}-1-piperidinyl)-2-pyridinecarboxamide;
N,N-Dimethyl-6-(4-{[1-(1-methylethyl)-4-piperidinyl]oxy}-1-piperidinyl)-2-pyridinecarboxamide;
2-(4-{[1-(1-Methylethyl)-4-piperidinyl]oxy}-1-piperidinyl)-6-(1-pyrrolidinylcarbonyl)pyridine;
4-{[6-(4-{[1-(1-Methylethyl)-4-piperidinyl]oxy}-1-piperidinyl)-2-pyridinyl]carbonyl}morpholine;
6-{4-[(1-Cyclobutyl-4-piperidinyl)oxy]-1-piperidinyl}-N-methyl-2-pyridinecarboxamide;
6-{4-[(1-Cyclobutyl-4-piperidinyl)oxy]-1-piperidinyl}-N,N-dimethyl-2-pyridinecarboxamide;
2-{4-[(1-Cyclobutyl-4-piperidinyl)oxy]-1-piperidinyl}-6-(1-pyrrolidinylcarbonyl)pyridine;
4-[(6-{4-[(1-Cyclobutyl-4-piperidinyl)oxy]-1-piperidinyl}-2-pyridinyl)carbonyl]morpholine;
N-(1-Methylethyl)-4-(4-{[1-(1-methylethyl)-4-piperidinyl]oxy}-1-piperidinyl)-2-pyridinecarboxamide;
4-{4-[(1-Cyclobutyl-4-piperidinyl)oxy]-1-piperidinyl}-N-(1-methylethyl)-2-pyridinecarboxamide;
4-(4-{[1-(1-Methylethyl)-4-piperidinyl]oxy}-1-piperidinyl)-2-(1-piperidinylcarbonyl)pyridine;
4-{4-[(1-Cyclobutyl-4-piperidinyl)oxy]-1-piperidinyl}-2-(1-piperidinylcarbonyl)pyridine;
5-{4-[(1-Cyclobutyl-4-piperidinyl)oxy]-1-piperidinyl}-N-methyl-2-pyrazinecarboxamide;
5-{4-[(1-Cyclobutyl-4-piperidinyl)oxy]-1-piperidinyl}-N-ethyl-2-pyrazinecarboxamide;
5-{4-[(1-Cyclobutyl-4-piperidinyl)oxy]-1-piperidinyl}-N-propyl-2-pyrazinecarboxamide;
5-{4-[(1-Cyclobutyl-4-piperidinyl)oxy]-1-piperidinyl}-N-(1-methylethyl)-2-pyrazinecarboxamide;
N-Cyclobutyl-5-{4-[(1-cyclobutyl-4-piperidinyl)oxy]-1-piperidinyl}-2-pyrazinecarboxamide;
N-Methyl-5-(4-{[1-(1-methylethyl)-4-piperidinyl]oxy}-1-piperidinyl)-2-pyrazinecarboxamide;
N-Ethyl-5-(4-{[1-(1-methylethyl)-4-piperidinyl]oxy}-1-piperidinyl)-2-pyrazinecarboxamide;
5-(4-{[1-(1-Methylethyl)-4-piperidinyl]oxy}-1-piperidinyl)-N-propyl-2-pyrazinecarboxamide;
N-(1-Methylethyl)-5-(4-{[1-(1-methylethyl)-4-piperidinyl]oxy}-1-piperidinyl)-2-pyrazinecarboxamide;
N-Cyclobutyl-5-(4-{[1-(1-methylethyl)-4-piperidinyl]oxy}-1-piperidinyl)-2-pyrazinecarboxamide;
5-(4-{[1-(1-Methylethyl)-4-piperidinyl]oxy}-1-piperidinyl)-2-pyridinecarbonitrile;
5-(4-{[1-(1-Methylethyl)-4-piperidinyl]oxy}-1-piperidinyl)-2-(trifluoromethyl)pyridine;
3-{4-[(1-Cyclobutyl-4-piperidinyl)oxy]-1-piperidinyl}-6-(trifluoromethyl)pyridazine;
3-(4-{[1-(1-Methylethyl)-4-piperidinyl]oxy}-1-piperidinyl)-6-(trifluoromethyl)pyridazine;
2-{4-[(1-Cyclobutyl-4-piperidinyl)oxy]-1-piperidinyl}-5-(trifluoromethyl)pyrazine;
2-(4-{[1-(1-Methylethyl)-4-piperidinyl]oxy}-1-piperidinyl)-5-(trifluoromethyl)pyrazine;
2-{4-[(1-Cyclobutyl-4-piperidinyl)oxy]-1-piperidinyl}-5-(trifluoromethyl)pyridine;
2-(4-{[1-(1-Methylethyl)-4-piperidinyl]oxy}-1-piperidinyl)-5-(trifluoromethyl)pyridine;
5-{4-[(1-Cyclobutyl-4-piperidinyl)oxy]-1-piperidinyl}-2-(trifluoromethyl)pyrimidine;
5-(4-{[1-(1-Methylethyl)-4-piperidinyl]oxy}-1-piperidinyl)-2-(trifluoromethyl)pyrimidine;
1-(4-{4-[(1-Cycloputyl-4-piperidinyl)oxy]-1-piperidinyl}phenyl)-1-propanone;
(4-{4-[(1-Cyclobutyl-4-piperidinyl)oxy]-1-piperidinyl}phenyl)(cyclopropyl)methanone;
Cyclobutyl-(4-{4-[(1-cyclobutyl-4-piperidinyl)oxy]-1-piperidinyl}phenyl)methanone;
1-(6-{4-[(1-Cyclobutyl-4-piperidinyl)oxy]-1-piperidinyl}-3-pyridinyl)ethanone;
6-{4-[(1-Cyclobutyl-4-piperidinyl)oxy]-1-piperidinyl}-2-methylquinoline;
1,1-Dimethylethyl-5-(4-{[1-(1-methylethyl)-4-piperidinyl]oxy}-1-piperidinyl)-2-pyridinecarboxylate;
1,1-Dimethylethyl-5-{4-[(1-cyclobutyl-4-piperidinyl)oxy]-1-piperidinyl}-2-pyridinecarboxylate;
1-[4-(4-{[1-(1-Methylethyl)-4-piperidinyl]oxy}-1-piperidinyl)phenyl]ethanorie;
N-(4-{4-[(1-Cyclobutyl-4-piperidinyl)oxy]-1-piperidinyl}-2-fluorophenyl)acetamide;
2-(4-{[1-(1-Methylethyl)-4-piperidinyl]oxy}-1-piperidinyl)-4-(trifluoromethyl)pyrimidine;
2-{4-[(1-Cyclobutyl-4-piperidinyl)oxy]-1-piperidinyl}-4-(trifluoromethyl)pyrimidine;
1-{4-[4-({1-[(1S)-1-Methylpropyl]-4-piperidinyl}oxy)-1-piperidinyl]phenyl}ethanone;
1-{4-[4-({1-[(1R)-1-Methylpropyl]-4-piperidinyl}oxy)-1-piperidinyl]phenyl}ethanone;
1-[4-(4-{[1-(Cyclopropylmethyl)-4-piperidinyl]oxy}-1-piperidinyl)phenyl]ethanone;
N-Cyctobutyl-5-{4-[(1-cyclobutyl-4-piperidinyl)oxy]-1-piperidinyl}-2-pyridinecarboxamide;
N-Cyclobutyl-5-(4-{[1-(1-methylethyl)-4-piperidinyl]oxy}-1-piperidinyl)-2-pyridinecarboxamide;
N-(3-Chloropropyl)-4-(4-{[1-(1-methylethyl)-4-piperidinyl]oxy}-1-piperidinyl)benzamide;
N-(3-Chloropropyl)-5-{4-[(1-cyclobutyl-4-piperidinyl)oxy]-1-piperidinyl}-2-pyridinecarboxamide;
N-(3-Chloropropyl)-5-(4-{[1-(1-methylethyl)-4-piperidinyl]oxy}-1-piperidinyl)-2-pyridinecarboxamide;
1-(4-{4-[(1-Cyclobutyl-4-piperidinyl)oxy]-1-piperidinyl}-3-fluorophenyl)ethanone;
1-Cyclobutyl-4-({1-[3-fluoro-4-(3-methyl-1,2,4-oxadiazol-5-yl)phenyl]-4-piperidinyl}oxy)piperidine;
1-(5-{4-[(1-Cyclobutyl-4-piperidinyl)oxy]-1-piperidinyl}-2-pyridinyl)ethanone;
1-[2-Fluoro-4-(3-methyl-1,2,4-oxadiazol-5-yl)phenyl]-4-{[1-(1-methylethyl)-4-piperidinyl]oxy}piperidine;
1-[3-Fluoro-4-(3-methyl-1,2,4-oxadiazol-5-yl)phenyl]-4-{[1-(1-methylethyl)-4-piperidinyl]oxy}piperidine;
4-{4-[(1-Cyclobutyl-4-piperidinyl)oxy]-1-piperidinyl}-3-fluorobenzonitrile;
4-{4-[(1-Cyclobutyl-4-piperidinyl)oxy]-1-piperidinyl}-2-fluorobenzonitrile;
1-(4-{4-[(1-Cyclobutyl-4-piperidinyl)oxy]-1-piperidinyl}-2-fluorophenyl)ethanone;
4-{4-[(1-Cyclobutyl-4-piperidinyl)oxy]-1-piperidinyl}-2,5-difluorobenzonitrile;
1-(4-{4-[(1-Cyclobutyl-4-piperidinyl)oxy]-1-piperidinyl}-2,5-difluorophenyl)ethanone;
1-[3-Fluoro-4-(3-methyl-1,2,4-oxadiazol-5-yl)phenyl]-4-({1-[(1S)-1-methylpropyl]-4-piperidinyl}oxy)piperidine; or
N-Methyl-5-(4-{[1-(1-methylethyl)-4-piperidinyl]oxy}-1-piperidinyl)-2-pyridinecarboxamide;
or a pharmaceutically acceptable salt thereof.

11. A compound as defined in claim 1 which is
1-(1-Methylethyl)-4-({1-[4-(3-methyl-1,2,4-oxadiazol-5-yl)phenyl]-4-piperidinyl}oxy)piperidine;
5-{4-[(1-Cyclobutyl-4-piperidinyl)oxy]-1-piperidinyl}-*N*-methyl-2-pyridinecarboxamide;
1-(4-{4-[(1-Cyclobutyl-4-piperidinyl)oxy]-1-piperidinyl}phenyl)ethanone;
3-{4-[(1-Cyclobutyl-4-piperidinyl)oxy]-1-piperidinyl}-6-(trifluoromethyl)pyridazine; or
5-{4-[(1-Cyclobutyl-4-piperidinyl)oxy]-1-piperidinyl}-2-(trifluoromethyl)pyrimidine;
or a pharmaceutically acceptable salt thereof.

12. A pharmaceutical composition which comprises the compound of formula (I) as defined in any one of claims 1 to 11 or a pharmaceutically acceptable salt thereof and a pharmaceutically acceptable carrier or excipient.

13. A compound as defined in any one of claims 1 to 11 for use in therapy.

14. A compound as defined in any one of claims 1 to 11 for use in the treatment of neurological diseases.

15. Use of a compound as defined in any one of claims 1 to 11 in the manufacture of a medicament for the treatment of neurological diseases.

16. A process for the preparation of a compound of formula (I) or a pharmaceutically acceptable salt thereof, which process comprises:
(a) reacting a compound of formula (II) 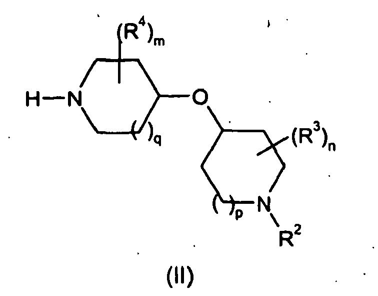 wherein R², R³, R⁴, m, n, p and q are as defined in claim 1, with a compound of formula R¹-L¹, wherein R¹ is as defined in claim 1 and L¹ represents a suitable leaving group, such as a halogen atom; or
(b) reacting a compound of formula (III) 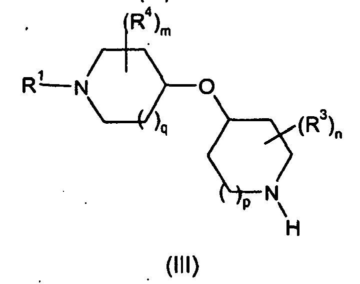 wherein R¹, R³, R⁴, m, n, p and q are as defined in claim 1, with a compound of formula R²-L²where R² is as defined in claim 1 and L² represents a suitable leaving group, such as a halogen atom or a sulfonate such as methanesulfonate; or
(c) reacting a compound of formula (III) as defined above with a compound of formula H-R^{2'}=O under reductive conditions, wherein R^{2'} is as defined in claim 1 for R² or a group convertible thereto; or
(d) preparing a compound of formula (I) wherein p represents 1 which comprises reduction of a compound of formula (IV) 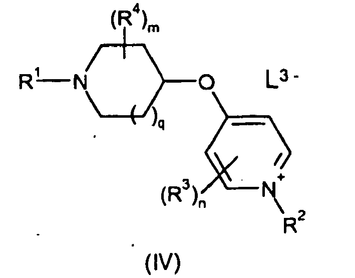 wherein R¹, R², R³, R⁴, m, n and q are as defined in claim 1 and L³⁻ represents a suitable counter ion such as a halogen atom; or
(e) deprotecting a compound of formula (I) or converting groups which are protected; and optionally thereafter
(f) interconversion to other compounds of formula (I).

## Patentansprüche

1. Verbindung der Formel (I) oder ein pharmazeutisch verträgliches Salz davon: 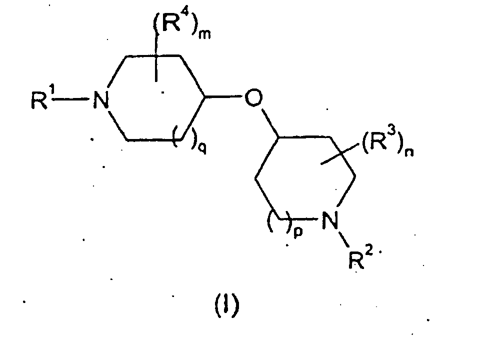 wobei:
R¹ Aryl, Heteroaryl, -Aryl-X-aryl, -Aryl-X-heteroaryl, -Aryl-X-heterocyclyl, -Heteroaryl-X-heteroaryl, -Heteroaryl-X-aryl oder -Heteroaryl-X-heterocyclyl darstellt;
wobei die Aryl-, Heteroaryl- und Heterocyclylreste von R¹ gegebenenfalls mit einem oder mehreren (z.B. 1, 2 oder 3) Substituenten substituiert sein können, welche gleich oder verschieden sein können und welche ausgewählt sind aus Halogen, Hydroxy, Cyano, Nitro, Oxo, Halogen-C₁₋₆-alkyl, Polyhalogen-C₁₋₆-alkyl, Halogen-C₁₋₆-alkoxy, Polyhalogen-C₁₋₆-alkoxy, C₁₋₆-Alkyl, C₁₋₆-Alkoxy, C₁₋₆-Alkylthio, C₁₋₆-Alkoxy-C₁₋₆-alkyl, C₃₋₇-Cycloalkyl-C₁₋₆-alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, C₁₋₆-Alkylsulfonamido-C₁₋₆-alkyl, C₁₋₆-Alkylamido-C₁₋₆-alkyl, Aryl, Arylsulfonyl, Arylsulfonyloxy, Aryloxy, Arylsulfonamido, Arylcarboxamido, Aroyl oder einem Rest -COR¹⁵, -COOR¹⁵, NR¹⁵R¹⁶, -CONR¹⁵R¹⁶, -NR¹⁵COR¹⁶, -NR¹⁵SO₂R¹⁶ oder -SO₂NR¹⁵R¹⁶, wobei R¹⁵ und R¹⁶ unabhängig voneinander Wasserstoff, C₁₋₆-Alkyl, Halogen-C₁₋₆-alkyl, Polyhalogen-C₁₋₆-alkyl oder C₃₋₆-Cycloalkyl darstellen oder zusammen einen heterocyclischen Ring bilden;
X eine Bindung, O, CO, SO₂, OCH₂ oder CH₂O darstellt;
R² C₃₋₈-Alkyl, C₃₋₆-Alkenyl, C₃₋₆-Alkinyl, C₃₋₆-Cycloalkyl, C₅₋₆-Cycloalkenyl oder -C₁₋₄-Alkyl-C₃₋₆-cycloalkyl darstellt;
wobei die C₃₋₆-Cycloalkylreste von R² gegebenenfalls mit einem oder mehreren (z.B. 1, 2 oder 3) Substituenten substituiert sein können, welche gleich oder verschieden sein können und welche ausgewählt sind aus Halogenatomen, C₁₋₄-Alkyl- oder Trifluormethylresten;
jeder R³- und R⁴-Rest unabhängig voneinander C₁₋₄-Alkyl darstellt;
m und n unabhängig voneinander 0, 1 oder 2 darstellen; und
p und q unabhängig voneinander 1 oder 2 darstellen.

2. Verbindung der Formel (I) wie in Anspruch 1 definiert, wobei R¹
einen -Arylrest, gegebenenfalls substituiert mit einem Cyano, -CONR¹⁵R¹⁶, -COR¹⁵, Halogen oder -NR¹⁵COR¹⁶;
- Heteroaryl, gegebenenfalls substituiert mit einer Cyanogruppe, einem C₁₋₆-Alkyl-, Polyhalogen-C₁₋₆-alkyl-, -CONR¹⁵R¹⁶-, -COR¹⁵- oder -COOR¹⁵-Rest; .
- Aryl-X-heterocyclyl;
- Aryl-X-heteroaryl, gegebenenfalls substituiert mit einem Halogenatom, C₁₋₆-Alkyl oder Aryl; oder
- Heteroaryl-X-heterocyclyl
darstellt.

3. Verbindung der Formel (I) wie in Anspruch 2 definiert, wobei R¹ Pyrid-3-yl, gegebenenfalls substituiert mit einem -CONR¹⁵R¹⁶-Rest, -Phenyl-1,2,4-oxadiazol-5-yl, gegebenenfalls substituiert mit einem C₁₋₆-Alkylrest, Phenyl, gegebenenfalls substituiert mit einem -COR¹⁵-Rest, Pyridazin-3-yl, gegebenenfalls substituiert mit einem Polyhalogen-C₁₋₆-alkylrest, Pyrazin-2-yl, gegebenenfalls substituiert mit einem Polyhalogen-C₁₋₆-alkylrest, oder Pyrimidin-5-yl, gegebenenfalls substituiert mit einem Polyhalogen-C₁₋₆-alkylrest, darstellt.

4. Verbindung der Formel (I) wie in Anspruch 3 definiert, wobei R¹ Pyrid-3-yl, gegebenenfalls substituiert mit einem 6-CON(H)(Me)- oder 6-CON(H)(Et)-Rest, 3-Methyl-1,2,4-oxadiazol-5-yl, Phenyl, gegebenenfalls substituiert mit einem 4-COMe-Rest, Pyridazin-3-yl, gegebenenfalls substituiert mit einem 6-CF₃-Rest, oder Pyrimidin-5-yl, gegebenenfalls substituiert mit einem 2-CF₃-Rest, darstellt.

5. Verbindung der Formel (I) wie in einem der Ansprüche 1 bis 4 definiert, wobei m und n 0 darstellen.

6. Verbindung der Formel (I) wie in einem der Ansprüche 1 bis 5 definiert, wobei p und q 1 darstellen.

7. Verbindung der Formel (I) wie in einem der Ansprüche 1 bis 6 definiert, wobei R² C₃₋₈-Alkyl, C₃₋₆-Cycloalkyl oder -C₁₋₄-Alkyl-C₃₋₆-cycloalkyl darstellt.

8. Verbindung der Formel (I) wie in Anspruch 7 definiert, wobei R² 1-Methylpropyl, Isopropyl, Cyclobutyl oder -CH₂-Cyclopropyl darstellt.

9. Verbindung der Formel (I) wie in Anspruch 8 definiert, wobei R² Isopropyl oder Cyclobutyl darstellt.

10. Verbindung wie in Anspruch 1 definiert, nämlich:
6-(4-{[1-(1-Methylethyl)-4-piperidinyl]oxy}-1-piperidinyl)-3-pyridincarbonitril;
6-{4-[(1-Cyclobutyl-4-piperidinyl)oxy]-1-piperidinyl}-3-pyridincarbonitril;
4-{4-[(1-Cyclobutyl-4-piperidinyl)oxy]-1-piperidinyl}benzonitril;
4-(4-{[1-(1-Methylethyl)-4-piperidinyl]oxy}-1-piperidinyl)benzonitril;
4-[(4-{4-[(1-Cyclobutyl-4-piperidinyl)oxy]-1-piperidinyl}phenyl)carbonyl]morpholin;
4-{4-[(1-Cyclobutyl-4-piperidinyl)oxy]-1-piperidinyl}-N-methylbenzamid;
4-{[4-(4-{[1-(1-Methylethyl)-4-piperidinyl]oxy}-1-piperidinyl)phenyl]carbonyl}morpholin;
1-(1-Methylethyl)-4-({1-[4-(1-piperidinylcarbonyl)phenyl]-4-piperidinyl}oxy)piperidin;
1-Cyclobutyl-4-({1-[4-(1-piperidinylcarbonyl)phenyl]-4-piperidinyl}oxy)piperidin;
1-(1-Methylethyl)-4-({1-[4-(1-pyrrolidinylcarbonyl)phenyl]-4-piperidinyl}oxy)piperidin;
1-Cyclobutyl-4-({1-[4-(1-pyrrolidinylcarbonyl)phenyl]-4-piperidinyl}oxy)piperidin;
N,N-Dimethyl-4-(4-{[1-(1-methylethyl)-4-piperidinyl]oxy}-1-piperidinyl)benzamid;
4-{4-[(1-Cyclobutyl-4-piperidinyl)oxy]-1-piperidinyl}-N,N-dimethylbenzamid;
N-Methyl-4-(4-{[1-(1-methylethyl)-4-piperidinyl]oxy}-1-piperidinyl)benzamid;
N-(3-Chlorpropyl)-4-{4-[(1-cyclobutyl-4-piperidinyl)oxy]-1-piperidinyl}benzamid;
1-Cyclobutyl-4-({1-[4-(2-methyl-1,3-oxazol-4-yl)phenyl]-4-piperidinyl}oxy)piperidin;
1-(1-Methylethyl)-4-({1-[4-(3-methyl-1,2,4-oxadiazol-5-yl)phenyl]-4-piperidinyl}oxy)piperidin;
1-(1-Methylethyl)-4-({1-[4-(2-methyl-1,3-oxazol-4-yl)phenyl]-4-piperidinyl}oxy)piperidin;
1-(1-Methylethyl)-4-({1-[4-(1,3-oxazol-2-yl)phenyl]-4-piperidinyl}oxy)piperidin;
1-(1-Methylethyl)-4-({1-[4-(2-methyl-1,3-oxazol-5-yl)phenyl]-4-piperidinyl}oxy)piperidin;
1-Cyclobutyl-4-({1-[4-(1,3-oxazol-2-yl)phenyl]-4-piperidinyl}oxy)piperidin;
1-Cyclobutyl-4-({1-[4-(2-methyl-1,3-oxazol-5-yl)phenyl]-4-piperidinyl}oxy)piperidin;
1-(1-Methylethyl)-4-({1-[4-(3-methyl-5-isoxazolyl)phenyl]-4-piperidinyl}oxy)piperidin;
1-Cyclobutyl-4-({1-[4-(3-methyl-5-isoxazolyl)phenyl]-4-piperidinyl}oxy)piperidin;
1-Cyclobutyl-4-({1-[4-(5-methyl-1,2,4-oxadiazol-3-yl)phenyl]-4-piperidinyl}oxy)piperidin;
1-(1-Methylethyl)-4-({1-[4-(5-phenyl-1,3,4-oxadiazol-2-yl)phenyl]-4-piperidinyl}oxy)piperidin;
1-Cyclobutyl-4-({1-[4-(5-phenyl-1,3,4-oxadiazol-2-yl)phenyl]-4-piperidinyl}oxy)piperidin;
1-Cyclobutyl-4-({1-[4-(3-methyl-1,2,4-oxadiazol-5-yl)phenyl]-4-piperidinyl}oxy)piperidin;
1-Cyclobutyl-4-({1-[4-(1,3-oxazol-5-yl)phenyl]-4-piperidinyl}oxy)piperidin;
1-(1-Methylethyl)-4-({1-[4-(1,3-oxazol-5-yl)phenyl]-4-piperidinyl}oxy)piperidin;
5-{4-[(1-Cyclobutyl-4-piperidinyl)oxy]-1-piperidinyl}-N,N-dimethyl-2-pyridincarboxamid;
N,N-Dimethyl-5-(4-{[1-(1-methylethyl)-4-piperidinyl]oxy}-1-piperidinyl)-2-pyridincarboxamid;
5-(4-{[1-(1-Methylethyl)-4-piperidinyl]oxy}-1-piperidinyl)-2-(1-pyrrolidinylcarbonyl)pyridin;
5-(4-{[1-(1-Methylethyl)-4-piperidinyl]oxy}-1-piperidinyl)-2-(1-piperidinylcarbonyl)pyridin;
5-{4-[(1-Cyclobutyl-4-piperidinyl)oxy]-1-piperidinyl}-2-(1-piperidinylcarbonyl)pyridin;
4-{[5-(4-{[1-(1-Methylethyl)-4-piperidinyl]oxy}-1-piperidinyl)-2-pyridinyl]carbonyl)morpholin;
4-[(5-{4-[(1-Cyclobutyl-4-piperidinyl)oxy]-1-piperidinyl}-2-pyridinyl)carbonyl]morpholin;
5- {4-[(1-Cyclobutyl-4-piperidinyl)oxy]-1-piperidinyl}-N-methyl-2-pyridincarboxamid;
N-Ethyl-5-(4-{[1-(1-methylethyl)-4-piperidinyl]oxy}-1-piperidinyl)-2-pyridincarboxamid;
5-{4-[(1-Cyclobutyl-4-piperidinyl)oxy]-1-piperidinyl}-N-ethyl-2-pyridincarboxamid;
5-(4-{[1-(1-Methylethyl)-4-piperidinyl]oxy}-1-piperidinyl)-N-propyl-2-pyridincarboxamid;
5-{4-[(1-Cyclobutyl-4-piperidinyl)oxy]-1-piperidinyl}-N-propyl-2-pyridincarboxamid;
N-(1-Methylethyl)-5-(4-{[1-(1-methylethyl)-4-piperidinyl]oxy}-1-piperidinyl)-2-pyridincarboxamid;
5-{4-[(1-Cyclobutyl-4-piperidinyl)oxy]-1-piperidinyl}-N-(1-methylethyl)-2-pyridincarboxamid;
N-Cyclopentyl-5-(4-{[1-(1-methylethyl)-4-piperidinyl]oxy}-1-piperidinyl)-2-pyridincarboxamid;
5-{4-[(1-Cyclobutyl-4-piperidinyl)oxy]-1-piperidinyl}-N-cyclopentyl-2-pyridincarboxamid;
5-{4-[(1-Cyclobutyl-4-piperidinyl)oxy]-1-piperidinyl}-2-pyridincarbonitril;
1-(4-{4-[(1-Cyclobutyl-4-piperidinyl)oxy]-1-piperidinyl}phenyl)ethanon;
5-{4-[(1-Cyclobutyl-4-piperidinyl)oxy]-1-piperidinyl}-2-(trifluormethyl)pyridin;
N-Methyl-6-(4-{[1-(1-methylethyl)-4-piperidinyl]oxy}-1-piperidinyl)-3-pyridincarboxamid;
N,N-Dimethyl-6-(4-{[1-(1-methylethyl)-4-piperidinyl]oxy}-1-piperidinyl)-3-pyridincarboxamid;
2-(4-{[1-(1-Methylethyl)-4-piperidinyl]oxy}-1-piperidinyl)-5-(1-pyrrolidinylcarbonyl)pyridin;
4-{[6-(4-{[1-(1-Methylethyl)-4-piperidinyl]oxy}-1-piperidinyl)-3-pyridinyl]carbonyl}morpholin;
6 {4-[(1-Cyclobutyl-4-piperidinyl)oxy]-1-piperidinyl}-N-methyl-3-pyridincarboxamid;
6-{4-[(1-Cyclobutyl-4-piperidinyl)oxy]-1-piperidinyl}-N,N-dimethyl-3-pyridincarboxamid;
2-{4-[(1-Cyclobutyl-4-piperidinyl)oxy]-1-piperidinyl}-5-(1-pyrrolidinylcarbonyl)pyridin;
4-[(6-{4-[(1-Cyclobutyl-4-piperidinyl)oxy]-1-piperidinyl}-3-pyridinyl)carbonyl]morpholin;
N-Methyl-6-(4-{[1-(1-methylethyl)-4-piperidinyl]oxy}-1-piperidinyl)-2-pyridincarboxamid;
N,N-Dimethyl-6-(4-{[1-(1-methylethyl)-4-piperidinyl]oxy}-1-piperidinyl)-2-pyridincarboxamid;
2-(4-{[1-(1-Methylethyl)-4-piperidinyl]oxy}-1-piperidinyl)-6-(1-pyrrolidinylcarbonyl)pyridin;
4-{[6-(4-{[1-(1-Methylethyl)-4-piperidinyl]oxy}-1-piperidinyl)-2-pyridinyl]carbonyl}morpholin;
6-{4-[(1-Cyclobutyl-4-piperidinyl)oxy]-1-piperidinyl}-N-methyl-2-pyridincarboxamid;.
6-{4-[(1-Cyclobutyl-4-piperidinyl)oxy]-1-piperidinyl}-N,N-dimethyl-2-pyridincarboxamid;
2-{4-[(1-Cyclobutyl-4-piperidinyl)oxy]-1-piperidinyl}-6-(1-pyrrolidinylcarbonyl)pyridin;
4-[(6-{4-[(1-Cyclobutyl-4-piperidinyl)oxy]-1-piperidinyl}-2-pyridinyl)carbonyl]morpholin;
N-(1-Methylethyl)-4-(4-{[1-(1-methylethyl)-4-piperidinyl]oxy}-1-piperidinyl)-2-pyridincarboxamid;
4-{4-[(1-Cyclobutyl-4-piperidinyl)oxy]-1-piperidinyl}-N-(1-methylethyl)-2-pyridincarboxamid;
4-(4-{[1-(1-Methylethyl)-4-piperidinyl]oxy}-1-piperidinyl)-2-(1-piperidinylcarbonyl)pyridin;
4-{4-[(1-Cyclobutyl-4-piperidinyl)oxy]-1-piperidinyl}-2-(1-piperidinylcarbonyl)pyridin;
5-{4-[(1-Cyclobutyl-4-piperidinyl)oxy]-1-piperidinyl}-N-methyl-2-pyrazincarboxamid;
5-{4-[(1-Cyclobutyl-4-piperidinyl)oxy]-1-piperidinyl}-N-ethyl-2-pyrazincarboxamid;
5-{4-[(-Cyclobutyl-4-piperidinyl)oxy]-1-piperidinyl}-N-propyl-2-pyrazincarboxamid;
5-{4-[(1-Cyclobutyl-4-piperidinyl)oxy]-1-piperidinyl}-N-(1-methylethyl)-2-pyrazincarboxamid;
N-Cyclobutyl-5-{4-[(1-cyclobutyl-4-piperidinyl)oxy]-1-piperidinyl}-2-pyrazincarboxamid;
N-Methyl-5-(4-{[1-(1-methylethyl)-4-piperidinyl]oxy}-1-piperidinyl)-2-pyrazincarboxamid;
N-Ethyl-5-(4-{[1-(1-methylethyl)-4-piperidinyl]oxy}-1-piperidinyl)-2-pyrazincarboxamid;
5-(4-{[1-(1-Methylethyl)-4-piperidinyl]oxy}-1-piperidinyl)-N-propyl-2-pyrazincarboxamid;
N-(1-Methylethyl)-5-(4-{[1-(1-methylethyl)-4-piperidinyl]oxy}-1-piperidinyl)-2-pyrazincarboxamid;
N-Cyclobutyl-5-(4-{[1-(1-methylethyl)-4-piperidinyl]oxy}-1-piperidinyl)-2-pyrazincarboxamid;
5-(4-{[1-(1-Methylethyl)-4-piperidinyl]oxy}-1-piperidinyl)-2-pyridincarbonitril;
5-(4-{[1-(1-Methylethyl)-4-piperidinyl]oxy}-1-piperidinyl)-2-(trifluormethyl)pyridin;
3-{4-[(1-Cyclobutyl-4-piperidinyl)oxy]-1-piperidinyl}-6-(trifluormethyl)pyridazin;
3-(4-{[1-(1-Methylethyl)-4-piperidinyl]oxy}-1-piperidinyl)-6-(trifluormethyl)pyridazin;
2-{4-[(1-Cyclobutyl-4-piperidinyl)oxy]-1-piperidinyl}-5-(trifluormethyl)pyrazin;
2-(4-{[1-(1-Methylethyl)-4-piperidinyl]oxy}-1-piperidinyl)-5-(trifluormethyl)pyrazin;
2-{4-[(1-Cyclobutyl-4-piperidinyl)oxy]-1-piperidinyl}-5-(trifluormethyl)pyridin;
2-(4-{[1-(1-Methylethyl)-4-piperidinyl]oxy}-1-piperidinyl)-5-(trifluormethyl)pyridin;
5-{4-[(1-Cyclobutyl-4-piperidinyl)oxy]-1-piperidinyl}-2-(trifluormethyl)pyrimidin;
5-(4-{[1-(1-Methylethyl)-4-piperidinyl]oxy}-1-piperidinyl)-2-(trifluormethyl)pyrimidin;
1-(4-{4-[(1-Cyclobutyl-4-piperidinyl)oxy]-1-piperidinyl}phenyl)-1-propanon;
(4-{4-[(1-Cyclobutyl-4-piperidinyl)oxy]-1-piperidinyl}phenyl)(cyclopropyl)methanon;
Cyclobutyl-(4-{4-[(1-cyclobutyl-4-piperidinyl)oxy]-1-piperidinyl}phenyl)methanon;
1-(6-{4-[(1-Cyclobutyl-4-piperidinyl)oxy]-1-piperidinyl}-3-pyridinyl)ethanon;
6-{4-[(1-Cyclobutyl-4-piperidinyl)oxy]-1-piperidinyl}-2-methylchinolin;
1,1-Dimethylethyl-5-(4-{[1-(1-methylethyl)-4-piperidinyl]oxy}-1-piperidinyl)-2-pyridincarboxylat;
1,1-Dimethylethyl-5-{4-[(1-cyclobutyl-4-piperidinyl)oxy]-1-piperidinyl}-2-pyridincarboxylat;
1-[4-(4-{[1-(1-Methylethyl)-4-piperidinyl]oxy}-1-piperidinyl)phenyl]ethanon;
N-(4-(4-[(1-Cyclobutyl-4-piperidinyl)oxy]-1-piperidinyl)-2-fluorphenyl)acetamid;
2-(4-{[1-(1-Methylethyl)-4-piperidinyl]oxy}-1-piperidinyl)-4-(trifluormethyl)pyrimidin;
2-{4-[(1-Cyclobutyl-4-piperidinyl)oxy]-1-piperidinyl}-4-(trifluormethyl)pyrimidin;
1-{4-[4-({1-[(1S)-1-Methylpropyl]-4-piperidinyl}oxy)-1-piperidinyl]phenyl}ethanon;
1-{4-[4-({1-[(1R)-1-Methylpropyl]-4-piperidinyl}oxy)-1-piperidinyl]phenyl}ethanon;
1-[4-(4-{[1-(Cyclopropylmethyl)-4-piperidinyl]oxy}-1-piperidinyl)phenyl]ethanon;
N-Cyclobutyl-5-{4-[(1-cyclobutyl-4-piperidinyl)oxy]-1-piperidinyl}-2-pyridincarboxamid;
N-Cyclobutyl-5-(4-{[1-(1-methylethyl)-4-piperidinyl]oxy}-1-piperidinyl)-2-pyridincarboxamid;
N-(3-Chlorpropyl)-4-(4-{[1-(1-methylethyl)-4-piperidinyl]oxy}-1-piperidinyl)benzamid;
N-(3-Chlorpropyl)-5-{4-[(1-cyclobutyl-4-piperidinyl)oxy]-1-piperidinyl}1-2-pyridincarboxamid;
N-(3-Chlorpropyl)-5-(4-{[1-(1-methylethyl)-4-piperidinyl]oxy}-1-piperidinyl)-2-pyridincarboxamid;
1-(4-{4-[(1-Cyclobutyl-4-piperidinyl)oxy]-1-piperidinyl}-3-fluorphenyl)ethanon;
1-Cyclobutyl-4-({1-[3-fluor-4-(3-methyl-1,2,4-oxadiazol-5-yl)phenyl]-4-piperidinyl}oxy)piperidin;
1-(5-{4-[(1-Cyclobutyl-4-piperidinyl)oxy]-1-piperidinyl}-2-pyridinyl)ethanon;
1-[2-Fluor-4-(3-methyl-1,2,4-oxadiazol-5-yl)phenyl]-4-{[1-(1-methylethyl)-4-piperidinyl]oxy}piperidin;
1-[3-Fluor-4-(3-methyl-1,2,4-oxadiazol-5-yl)phenyl]-4-{[1-(1-methylethyl)-4-piperidinyl]oxy}piperidin;
4-{4-[(1-Cyclobutyl-4-piperidinyl)oxy]-1-piperidinyl}-3-fluorbenzonitril;
4-{4-[(1-Cyclobutyl-4-piperidinyl)oxy]-1-piperidinyl}-2-fluorbenzonitril;
1-(4-{4-[(1-Cyclobutyl-4-piperidinyl)oxy]-1-piperidinyl}-2-fluorophenyl)ethanon;
4-{4-[(1-Cyclobutyl-4-piperidinyl)oxy]-1-piperidinyl }-2,5-difluorbenzonitril;
1-(4-{4-[(1-Cyclobutyl-4-piperidinyl)oxy]-1-piperidinyl}-2,5-difluorphenyl)ethanon;
1-[3-Fluor-4-(3-methyl-1,2,4-oxadiazol-5-yl)phenyl]-4-({1-[(1S)-1-methylpropyl]-4-piperidinyl}oxy)piperidin; oder
N-Methyl-5-(4-{[1-(1-methylethyl)-4-piperidinyl]oxy}-1-piperidinyl)-2-pyridincarboxamid;
oder ein pharmazeutisch verträgliches Salz davon.

11. Verbindung wie in Anspruch 1 definiert, nämlich
1-(1-Methylethyl)-4-({1-[4-(3-methyl-1,2,4-oxadiazol-5-yl)phenyl]-4-piperidinyl}oxy)piperidin;
5-{4-[(1-Cyclobutyl-4-piperidinyl)oxy]-1-piperidinyl}-*N*-methyl-2-pyridincarboxamid;
1-(4-{4-[(1-Cyclobutyl-4-piperidinyl)oxy]-1-piperidinyl}phenyl)ethanon;
3-{4-[(1-Cyclobutyl-4-piperidinyl)oxy]-1-piperidinyl}-6-(trifluormethyl)pyridazin; oder
5-{4-[(1-Cyclobutyl-4-piperidinyl)oxy]-1-piperidinyl}-2-(trifluormethyl)pyrimidin;
oder ein pharmazeutisch verträgliches Salz davon.

12. Arzneimittel, welches die Verbindung der Formel (I) wie in einem der Ansprüche 1 bis 11 definiert oder ein pharmazeutisch verträgliches Salz davon und einen pharmazeutisch verträglichen Träger oder Exzipienten umfasst.

13. Verbindung wie in einem der Ansprüche 1 bis 11 definiert zur Verwendung in der Therapie.

14. Verbindung wie in einem der Ansprüche 1 bis 11 definiert zur Verwendung bei der Behandlung von neurologischen Erkrankungen.

15. Verwendung einer Verbindung wie in einem der Ansprüche 1 bis 11 definiert zur Herstellung eines Medikaments zur Behandlung von neurologischen Erkrankungen.

16. Verfahren zur Herstellung einer Verbindung der Formel (I) oder eines pharmazeutisch verträglichen Salzes davon, wobei das Verfahren umfasst:
(a) Umsetzen einer Verbindung der Formel (II) 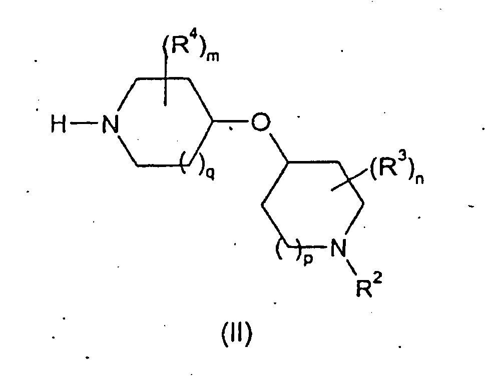 wobei R², R³, R⁴, m, n, p und q wie in Anspruch 1 definiert sind, mit einer Verbindung der Formel R¹-L¹, wobei R¹ wie in Anspruch 1 definiert ist und L¹ eine geeignete Abgangsgruppe, wie ein Halogenatom, darstellt; oder
(b) Umsetzen einer Verbindung der Formel (III) 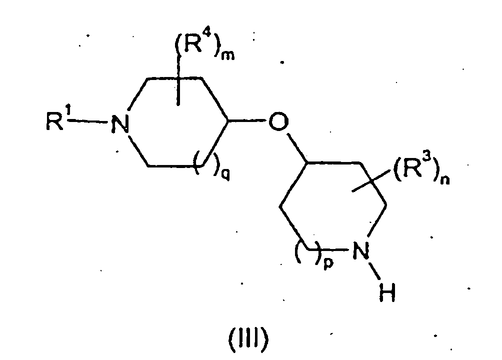 wobei R¹, R³, R⁴, m, n, p und q wie in Anspruch 1 definiert sind, mit einer Verbindung der Formel R²-L², wobei R² wie in Anspruch 1 definiert ist und L² eine geeignete Abgangsgruppe, wie ein Halogenatom, oder ein Sulfonat, wie Methansulfonat, darstellt; oder
c) Umsetzen einer Verbindung der Formel (III) wie vorstehend definiert mit einer Verbindung der Formel H-R^{2'}=O unter reduzierenden Bedingungen, wobei R^{2'} wie in Anspruch 1 für R² definiert ist oder ein Rest, der darin umgewandelt werden kann, ist; oder
d) Herstellen einer Verbindung der Formel (I), wobei p 1 darstellt, welches die Reduktion einer Verbindung der Formel (IV) umfasst, 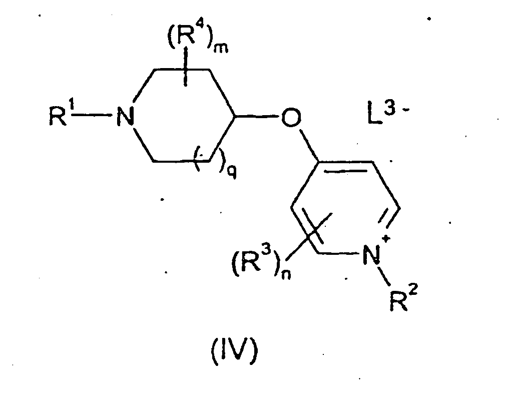 wobei R¹, R², R³, R⁴, m, n und q wie in Anspruch 1 definiert sind und L³⁻ ein geeignetes Gegenion, wie ein Halogenatom, darstellt; oder
(e) Entschützen einer Verbindung der Formel (I) oder Umwandeln von Resten, welche geschützt sind; und gegebenenfalls anschließend
(f) gegenseitiges Umwandeln in andere Verbindungen der Formel (I).

## Revendications

1. Composé de formule (I) ou un de ses sels pharmaceutiquement acceptables : 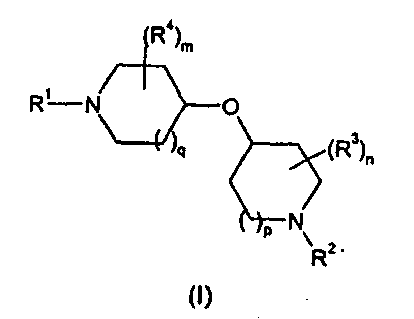 formule dans laquelle :
R¹ représente un groupe aryle, hétéroaryle, -aryl-X-aryle, -aryl-X-hétéroaryle, -aryl-X-hétérocyclyle, -hétéroaryl-X-hétéroaryle, -hétéroaryl-X-aryle ou -hétéroaryl-X-hétérocyclyle ;
lesdits groupes aryle, hétéroaryle et hétérocyclyle de R¹ pouvant être facultativement substitués avec un ou plusieurs (par exemple 1, 2 ou 3) substituants qui peuvent être identiques ou différents et qui sont choisis dans le groupe consistant en des substituants halogéno, hydroxy, cyano, nitro, oxo, halogénalkyle en C₁ à C₆, polyhalogénalkyle en C₁ à C₆, halogénalkoxy en C₁ à C₆, polyhalogénalkoxy en C₁ à C₆, alkyle en C₁ à C₆, alkoxy en C₁ à C₆, alkylthio en C₁ à C₆, (alkoxy en C₁ à C₆) (alkyle en C₁ à C₆), (cycloalkyle en C₃ à C₇) (alkoxy en C₁ à C₆), alcanoyle en C₁ à C₆, (alkoxy en C₁ à C₆)carbonyle, alkylsulfonyle en C₁ à C₆, alkylsulfinyle en C₁ à C₆, alkylsulfonyloxy en C₁ à C₆, (alkyle en C₁ à C₆) sulfonyl (alkyle en C₁ à C₆), (alkyle en C₁ à C₆) sulfonamido (alkyle en C₁ à C₆), (alkyle en C₁ à C₆) amido (alkyle en C₁ à C₆), aryle, arylsulfonyle, arylsulfonyloxy, aryloxy, arylsulfonamido, arylcarboxamido, aroyle, ou un groupe -COR¹⁵, -COOR¹⁵, NR¹⁵R¹⁶, -CONR¹⁵R¹⁶, -NR¹⁵COR¹⁶, -NR¹⁵SO₂R¹⁶ ou -SO₂NR¹⁵R¹⁶, dans lequel R¹⁵ et R¹⁶ représentent indépendamment un atome d'hydrogène, un groupe alkyle en C₁ à C₆, halogénalkyle en C₁ à C₆, polyhalogénalkyle en C₁ à C₆ ou cycloalkyle en C₃ à C₆, ou bien forment conjointement un noyau hétérocyclique ;
X représente une liaison, un groupe O, CO, SO₂, OCH₂ ou CH₂O ;
R² représente un groupe alkyle en C₃ à C₈, alcényle en C₃ à C₆, alcynyle en C₃ à C₆, cycloalkyle en C₃ à C₆, cycloalcényle en C₅ ou C₆ ou - (alkyle en C₁ à C₄) - (cycloalkyle en C₃ à C₆) ;
lesdits groupes cycloalkyle en C₃ à C₆ de R² pouvant être facultativement substitués avec un ou plusieurs (par exemple 1, 2 ou 3) substituants qui peuvent être identiques ou différents et qui sont choisis dans le groupe consistant en des groupes halogéno, alkyle en C₁ à C₄ et trifluorométhyle ;
chacun des groupes R³ et R⁴ représente indépendamment un groupe alkyle en C₁ à C₄ ;
m et n sont égaux indépendamment à 0, 1 ou 2 ; et
p et q sont égaux indépendamment à 1 ou 2.

2. Composé de formule (I) suivant la revendication 1, dans lequel R¹ représente un groupe
- aryle facultativement substitué avec un substituant cyano, -CONR¹⁵R¹⁶, -COR¹⁵, halogéno ou -NR¹⁵COR¹⁶ ;
- hétéroaryle facultativement substitué avec un groupe cyano, alkyle en C₁ à C₆, polyhalogénalkyle en C₁ à C₆, -CONR¹⁵R¹⁶, -COR¹⁵ ou -COOR¹⁵;
- aryl-X-hétérocyclyle ;
- aryl-X-hétéroaryle facultativement substitué avec un substituant halogéno, alkyle en C₁ à C₆ ou aryle ; ou
- hétéroaryl-X-hétérocyclyle.

3. Composé de formule (I) suivant la revendication 2, dans lequel R¹ représente un groupe pyrid-3-yle facultativement substitué avec un groupe -CONR¹⁵R¹⁶, un groupe -phényl-1,2,4-oxadiazole-5-yle facultativement substitué avec un groupe alkyle en C₁ à C₆, un groupe phényle facultativement substitué avec un groupe -COR¹⁵, un groupe pyridazine-3-yle facultativement substitué avec un groupe polyhalogénalkyle en C₁ à C₆, un groupe pyrazine-2-yle facultativement substitué avec un groupe polyhalogénalkyle en C₁ à C₆ ou un groupe pyrimidine-5-yle facultativement substitué avec un groupe polyhalogénalkyle en C₁ à C₆.

4. Composé de formule (I) suivant la revendication 3, dans lequel R¹ représente un groupe pyrid-3-yle facultativement substitué avec un groupe 6-CON(H)(Me) ou 6-CON(H)(Et), un groupe 3-méthyl-1,2,4-oxadiazole-5-yle, un groupe phényle facultativement substitué avec un groupe 4-COMe, un groupe pyridazine-3-yle facultativement substitué avec un groupe 6-CF₃ ou un groupe pyrimidine-5-yle facultativement substitué avec un groupe 2-CF₃.

5. Composé de formule (I) suivant l'une quelconque des revendications 1 à 4, dans lequel m et n sont égaux à 0.

6. Composé de formule (I) suivant l'une quelconque des revendications 1 à 5, dans lequel p et q sont égaux à 1.

7. Composé de formule (I) suivant l'une quelconque des revendications 1 à 6, dans lequel R² représente un groupe alkyle en C₃ à C₈, cycloalkyle en C₃ à C₆ ou -(alkyle en C₁ à C₄)-(cycloalkyle en C₃ à C₆).

8. Composé de formule (I) suivant la revendication 7, dans lequel R² représente un groupe 1-méthylpropyle, isopropyle, cyclobutyle ou -CH₂-cyclopropyle.

9. Composé de formule (I) suivant la revendication 8, dans lequel R² représente un groupe isopropyle ou cyclobutyle.

10. Composé suivant la revendication 1, qui est :
le 6-(4-{[1-(1-méthyléthyl)-4-pipéridinyl]oxy}-1-pipéridinyl)-3-pyridinecarbonitrile ;
le 6-{4-[(1-cyclobutyl-4-pipéridinyl)oxy]-1-pipéridinyl}-3-pyridinecarbonitrile ;
le 4-{4-[(1-cyclobutyl-4-pipéridinyl)oxy]-1-pipéridinyl}benzonitrile ;
le 4-(4-{[1-(1-méthyléthyl)-4-pipéridinyl]oxy}-1-pipéridinyl)benzonitrile ;
la 4-[(4-{4-[(1-cyclobutyl-4-pipéridinyl)oxy]-1-pipéridinyl}phényl)carbonyl]morpholine ;
le 4-{4-[(1-cyclobutyl-4-pipéridinyl)oxy]-1-pipéridinyl}-N-méthylbenzamide ;
la 4-{[4-(4-{[1-(1-méthyléthyl)-4-pipéridinyl]oxy}-1-pipéridinyl)phényl]carbonyl}morpholine ;
la 1-(1-méthyléthyl)-4-({1-[4-(1-pipéridinylcarbonyl)phényl]-4-pipéridinyl}oxy)pipéridine ;
la 1-cyclobutyl-4-({1-[4-(1-pipéridinylcarbonyl)phényl]-4-pipéridinyl}oxy)pipéridine ;
la 1-(1-méthyléthyl)-4-({1-[4-(1-pyrrolidinylcarbonyl)phényl]-4-pipéridinyl}oxy)pipéridine ;
la 1-cyclobutyl-4-({1-[4-(1-pyrrolidinylcarbonyl)phényl]-4-pipéridinyl}oxy)pipéridine ;
le N,N-diméthyl-4-(4-{[1-(1-méthyléthyl)-4-pipéridinyl]oxy}-1-pipéridinyl)benzamide ;
le 4-{4-[(1-cyclobutyl-4-pipéridinyl)oxy]-1-pipéridinyl}-N,N-diméthylbenzamide ;
le N-méthyl-4-(4-{[1-(1-méthyléthyl)-4-pipéridinyl]oxy}-1-pipéridinyl)benzamide ;
le N-(3-chloropropyl)-4-{4-[(1-cyclobutyl-4-pipéridinyl)oxy]-1-pipéridinyl}benzamide ;
la 1-cyclobutyl-4-({1-[4-(2-méthyl-1,3-oxazole-4-yl)phényl]-4-pipéridinyl}oxy)pipéridine ;
la 1-(1-méthyléthyl)-4-({1-[4-(3-méthyl-1,2,4-oxadiazole-5-yl)phényl]-4-pipéridinyl}oxy)pipéridine ;
la 1-(1-méthyléthyl)-4-({1-[4-(2-méthyl-1,3-oxazole-4-yl)phényl]-4-pipéridinyl}oxy)pipéridine ;
la 1-(1-méthyléthyl)-4-({1-[4-(1,3-oxazole-2-yl)phényl]-4-pipéridinyl}oxy)pipéridine ;
la 1-(1-méthyléthyl)-4-({1-[4-(2-méthyl-1,3-oxazole-5-yl)phényl]-4-pipéridinyl}oxy)pipéridine ;
la 1-cyclobutyl-4-({1-[4-(1,3-oxazole-2-yl)phényl]-4-pipéridinyl}oxy)pipéridine ;
la 1-cyclobutyl-4-({1-[4-(2-méthyl-1,3-oxazole-5-yl)phényl]-4-pipéridinyl}oxy)pipéridine ;
la 1-(1-méthyléthyl)-4-({1-[4-(3-méthyl-5-isoxazolyl)phényl]-4-pipéridinyl}oxy)pipéridine ;
la 1-cyclobutyl-4-({1-[4-(3-méthyl-5-isoxazolyl)phényl]-4-pipéridinyl}oxy)pipéridine ;
la 1-cyclobutyl-4-({1-[4-(5-méthyl-1,2,4-oxadiazole-3-yl)phényl]-4-pipéridinyl}oxy)pipéridine ;
la 1-(1-méthyléthyl)-4-({1-[4-(5-phényl-1,3,4-oxadiazole-2-yl)phényl]-4-pipéridinyl}oxy)pipéridine ;
la 1-cyclobutyl-4-({1-[4-(5-phényl-1,3,4-oxadiazole-2-yl)phényl]-4-pipéridinyl}oxy)pipéridine ;
la 1-cyclobutyl-4-({1-[4-(3-méthyl-1,2,4-oxadiazole-5-yl)phényl]-4-pipéridinyl}oxy)pipéridine ;
la 1-cyclobutyl-4-({1-[4-(1,3-oxazole-5-yl)phényl]-4-pipéridinyl}oxy)pipéridine ;
la 1-(1-méthyléthyl)-4-({1-[4-(1,3-oxazole-5-yl)phényl]-4-pipéridinyl}oxy)pipéridine ;
le 5-{4-[(1-cyclobutyl-4-pipéridinyl)oxy]-1-pipéridinyl}-N,N-diméthyl-2-pyridinecarboxamide ;
le N,N-diméthyl-5-(4-{[1-(1-méthyléthyl)-4-pipéridinyl]oxy}-1-pipéridinyl)-2-pyridinecarboxamide ;
la 5-(4-{[1-(1-méthyléthyl)-4-pipéridinyl]oxy}-1-pipéridinyl)-2-(1-pyrrolidinylcarbonyl)pyridine ;
la 5-(4-{[1-(1-méthyléthyl)-4-pipéridinyl]oxy}-1-pipéridinyl)-2-(1-pipéridinylcarbonyl)pyridine ;
la 5-{4-[(1-cyclobutyl-4-pipéridinyl)oxy]-1-pipéridinyl}-2-(1-pipéridinylcarbonyl)pyridine ;
la 4-{[5-(4-{[1-(1-méthyléthyl)-4-pipéridinyl]oxy}-1-pipéridinyl)-2-pyridinyl]carbonyl}morpholine ;
la 4-[(5-{4-[(1-cyclobutyl-4-pipéridinyl)oxy]-1-pipéridinyl}-2-pyridinyl)carbonyl]morpholine ;
le 5-{4-[(1-cyclobutyl-4-pipéridinyl)oxy]-1-pipéridinyl}-N-méthyl-2-pyridinecarboxamide ;
le N-éthyl-5-(4-{[1-(1-méthyléthyl)-4-pipéridinyl]oxy}-1-pipéridinyl)-2-pyridinecarboxamide ;
le 5-{4-[(1-cyclobutyl-4-pipéridinyl)oxy]-1-pipéridinyl}-N-éthyl-2-pyridinecarboxamide ;
le 5-(4-{[1-(1-méthyléthyl)-4-pipéridinyl]oxy}-1-pipéridinyl)-N-propyl-2-pyridinecarboxamide ;
le 5-{4-[(1-cyclobutyl-4-pipéridinyl)oxy]-1-pipéridinyl}-N-propyl-2-pyridinecarboxamide ;
le N-(1-méthyléthyl)-5-(4-{[1-(1-méthyléthyl)-4-pipéridinyl]oxy}-1-pipéridinyl)-2-pyridinecarboxamide ;
le 5-{4-[(1-cyclobutyl-4-pipéridinyl)oxy]-1-pipéridinyl}-N-(1-méthyléthyl)-2-pyridinecarboxamide ;
le N-cyclopentyl-5-(4-{[1-(1-méthyléthyl)-4-pipéridinyl]oxy}-1-pipéridinyl)-2-pyridinecarboxamide ;
le 5-{4-[(1-cyclobutyl-4-pipéridinyl)oxy]-1-pipéridinyl}-N-cyclopentyl-2-pyridinecarboxamide ;
le 5-{4-[(1-cyclobutyl-4-pipéridinyl)oxy]-1-pipéridinyl}-2-pyridinecarbonitrile ;
la 1-(4-{4-[(1-cyclobutyl-4-pipéridinyl)oxy]-1-pipéridinyl}phényl)éthanone ;
la 5-{4-[(1-cyclobutyl-4-pipéridinyl)oxy]-1-pipéridinyl}-2-(trifluorométhyl)pyridine ;
le N-méthyl-6*-*(4-{[1-(1-méthyléthyl)-4-pipéridinyl]oxy}-1-pipéridinyl)-3-pyridinecarboxamide ;
le N,N-diméthyl-6-(4-{[1-(1-méthyléthyl)-4-pipéridinyl]oxy}-1-pipéridinyl)-3-pyridinecarboxamide ;
la 2-(4-{[1-(1-méthyléthyl)-4-pipéridinyl]oxy}-1-pipéridinyl)-5-(1-pyrrolidinylcarbonyl)pyridine ;
la 4-{[6-(4-{[1-(1-méthyléthyl)-4-pipéridinyl]oxy}-1-pipéridinyl)-3-pyridinyl]carbonyl}morpholine ;
le 6-{4-[(1-cyclobutyl-4-pipéridinyl)oxy]-1-pipéridinyl}-N-méthyl-3-pyridinecarboxamide ;
le 6-{4-[(1-cyclobutyl-4-pipéridinyl)oxy]-1-pipéridinyl}-N,N-diméthyl-3-pyridinecarboxamide ;
la 2-{4-[(1-cyclobutyl-4-pipéridinyl)oxy]-1-pipéridinyl}-5-(1-pyrrolidinylcarbonyl)pyridine ;
la 4-[(6-{4-[(1-cyclobutyl-4-pipéridinyl)oxy]-1-pipéridinyl}-3-pyridinyl)carbonyl]morpholine ;
le N-méthyl-6-(4-{[1-(1-méthyléthyl)-4-pipéridinyl]oxy}-1-pipéridinyl)-2-pyridinecarboxamide ;
le N,N-diméthyl-6-(4-{[1-(1-méthyléthyl)-4-pipéridinyl]oxy}-1-pipéridinyl)-2-pyridinecarboxamide ;
la 2-(4-{[1-(1-méthyléthyl)-4-pipéridinyl]oxy}-1-pipéridinyl)-6-(1-pyrrolidinylcarbonyl)pyridine ;
la 4-{[6-(4-{[1-(1-méthyléthyl)-4-pipéridinyl]oxy}-1-pipéridinyl)-2-pyridinyl]carbonyl}morpholine;
le 6-{4-[(1-cyclobutyl-4-pipéridinyl)oxy] -1-pipéridinyl}-N-méthyl-2-pyridinecarboxamide ;
le 6-{4-[(1-cyclobutyl-4-pipéridinyl)oxy]-1-pipéridinyl}-N,N-diméthyl-2-pyridinecarboxamide ;
la 2-{4-[(1-cyclobutyl-4-pipéridinyl)oxy]-1-pipéridinyl}-6-(1-pyrrolidinylcarbonyl)pyridine ;
la 4-[(6-{4-[(1-cyclobutyl-4-pipéridinyl)oxy]-1-pipéridinyl}-2-pyridinyl)carbonyl]morpholine ;
le N-(1-méthyléthyl)-4-(4-{[1-(1-méthyléthyl)-4-pipéridinyl]oxy}-1-pipéridinyl)-2-pyridinecarboxamide ;
le 4-{4-[(1-cyclobutyl-4-pipéridinyl)oxy]-1-pipéridinyl}-N-(1-méthyléthyl)-2-pyridinecarboxamide ;
la 4-(4-{[1-(1-méthyléthyl)-4-pipéridinyl]oxy}-1-pipéridinyl)-2-(1-pipéridinylcarbonyl)pyridine ;
la 4-{4-[(1-cyclobutyl-4-pipéridinyl)oxy]-1-pipéridinyl}-2-(1-pipéridinylcarbonyl)pyridine ;
le 5-{4-[(1-cyclobutyl-4-pipéridinyl)oxy]-1-pipéridinyl}-N-méthyl-2-pyrazinecarboxamide ;
le 5-{4-[(1-cyclobutyl-4-pipéridinyl)oxy]-1-pipéridinyl}-N-éthyl-2-pyrazinecarboxamide ;
le 5-{4-[(1-cyclobutyl-4-pipéridinyl)oxy]-1-pipéridinyl}-N-propyl-2-pyrazinecarboxamide ;
le 5-{4-[(1-cyclobutyl-4-pipéridinyl)oxy]-1-pipéridinyl}-N-(1-méthyléthyl)-2-pyrazinecarboxamide ;
le N-cyclobutyl-5-{4-[(1-cyclobutyl-4-pipéridinyl)oxy]-1-pipéridinyl}-2-pyrazinecarboxamide ;
le N-méthyl-5-(4-{[1-(1-méthyléthyl)-4-pipéridinyl]oxy}-1-pipéridinyl)-2-pyrazinecarboxamide ;
le N-éthyl-5-(4-{[1-(1-méthyléthyl)-4-pipéridinyl]oxy}-1-pipéridinyl)-2-pyrazinecarboxamide ;
le 5-(4-{[1-(1-méthyléthyl)-4-pipéridinyl]oxy}-1-pipéridinyl)-N-propyl-2-pyrazinecarboxamide ;
le N-(1-méthyléthyl)-5-(4-{[1-(1-méthyléthyl)-4-pipéridinyl]oxy}-1-pipéridinyl)-2-pyrazinecarboxamide ;
le N-cyclobutyl-5-(4-{[1-(1-méthyléthyl)-4-pipéridinyl]-oxy}-1-pipéridinyl)-2-pyrazinecarboxamide ;
le 5- (4-{[1-(1-méthyléthyl)-4-pipéridinyl]oxy}-1-pipéridinyl)-2-pyridinecarbonitrile ;
la 5-(4-{[1-(1-méthyléthyl)-4-pipéridinyl]oxy}-1-pipéridinyl)-2-(trifluorométhyl)pyridine ;
la 3-{4-[(1-cyclobutyl-4-pipéridinyl)oxy]-1-pipéridinyl}-6-(trifluorométhyl)pyridazine ;
la 3-(4-{[1-(1-méthyléthyl)-4-pipéridinyl]oxy}-1-pipéridinyl)-6-(trifluorométhyl)pyridazine ;
la 2-{4-[(1-cyclobutyl-4-pipéridinyl)oxy]-1-pipéridinyl}-5-(trifluorométhyl)pyrazine ;
la 2-(4-{[1-(1-méthyléthyl)-4-pipéridinyl]oxy}-1-pipéridinyl)-5-(trifluorométhyl)pyrazine ;
la 2-{4-[(1-cyclobutyl-4-pipéridinyl)oxy]-1-pipéridinyl}-5-(trifluorométhyl)pyridine ;
la 2-(4-{[1-(1-méthyléthyl)-4-pipéridinyl]oxy}-1-pipéridinyl)-5-(trifluorométhyl)pyridine ;
la 5-{4-[(1-cyclobutyl-4-pipéridinyl)oxy]-1-pipéridinyl}-2-(trifluorométhyl)pyrimidine ;
la 5-(4-{[1-(1-méthyléthyl)-4-pipéridinyl]oxy}-1-pipéridinyl)-2-(trifluorométhyl)pyrimidine;
la 1-(4-{4-[(1-cyclobutyl-4-pipéridinyl)oxy]-1-pipéridinyl}phényl)-1-propanone ;
la (4-{4-[(1-cyclobutyl-4-pipéridinyl)oxy]-1-pipéridinyl}phényl)(cyclopropyl)méthanone;
la cyclobutyl-(4-{4-[(1-cyclobutyl-4-pipéridinyl)oxy]-1-pipéridinyl}phényl)méthanone ;
la 1-(6-{4-[(1-cyclobutyl-4-pipéridinyl)oxy]-1-pipéridinyl}-3-pyridinyl)éthanone ;
la 6-{4-[(1-cyclobutyl-4-pipéridinyl)oxy]-1-pipéridinyl}-2-méthylquinoléine ;
le 5-(4-{[1-(1-méthyléthyl)-4-pipéridinyl]oxy}-1-pipéridinyl)-2-pyridinecarboxylate de 1,1-diméthyléthyle ;
le 5-{4-[(1-cyclobutyl-4-pipéridinyl)oxy]-1-pipéridinyl}-2-pyridinecarboxylate de 1,1-diméthyléthyle ;
la 1-[4-(4-{[1-(1-méthyléthyl)-4-pipéridinyl]oxy}-1-pipéridinyl)phényl]éthanone ;
le N-(4-{4-[(1-cyclobutyl-4-pipéridinyl)oxy]-1-pipéridinyl}-2-fluorophényl)acétamide ;
la 2-(4-{[1-(1-méthyléthyl)-4-pipéridinyl]oxy}-1-pipéridinyl)-4-(trifluorométhyl)pyrimidine ;
la 2-{4-[(1-cyclobutyl-4-pipéridinyl)oxy]-1-pipéridinyl}-4-(trifluorométhyl)pyrimidine ;
la 1-{4-[4-({1-[(1S)-1-méthylpropyl]-4-pipéridinyl}oxy)-1-pipéridinyl]phényl}éthanone ;
la 1-{4-[4-({1-[(1R)-1-méthylpropyl]-4-pipéridinyl}oxy)-1-pipéridinyl]phényl}éthanone ;
la 1-[4-(4-{[1-(cyclopropylméthyl)-4-pipéridinyl]oxy}-1-pipéridinyl)phényl]éthanone ;
le N-cyclobutyl-5-{4-[(1-cyclobutyl-4-pipéridinyl)oxy]-1-pipéridinyl}-2-pyridinecarboxamide ;
le N-cyclobutyl-5-(4-{[1-(1-méthyléthyl)-4-pipéridinyl]oxy}-1-pipéridinyl)-2-pyridinecarboxamide ;
le N-(3-chloropropyl)-4-(4-{[1-(1-méthyléthyl)-4-pipéridinyl]oxy}-1-pipéridinyl)benzamide ;
le N-(3-chloropropyl)-5-{4-[(1-cyclobutyl-4-pipéridinyl)oxy]-1-pipéridinyl}-2-pyridinecarboxamide ;
le N-(3-chloropropyl)-5-(4-{[1-(1-méthyléthyl)-4-pipéridinyl]oxy}-1-pipéridinyl)-2-pyridinecarboxamide ;
la 1-(4-{4-[(1-cyclobutyl-4-pipéridinyl)oxy]-1-pipéridinyl}-3-fluorophényl)éthanone ;
la 1-cyclobutyl-4-({1-[3-fluoro-4-(3-méthyl-1,2,4-oxadiazole-5-yl)phényl]-4-pipéridinyl}oxy)pipéridine ;
la 1-(5-{4-[(1-cyclobutyl-4-pipéridinyl)oxy]-1-pipéridinyl}-2-pyridinyl)éthanone ;
la 1-[2-fluoro-4-(3-méthyl-1,2,4-oxadiazole-5-yl)phényl]-4-{[1-(1-méthyléthyl)-4-pipéridinyl]oxy}pipéridine ;
la 1-[3-fluoro-4-(3-méthyl-1,2,4-oxadiazole-5-yl)phényl]-4-{[1-(1-méthyléthyl)-4-pipéridinyl]oxy}pipéridine ;
le 4-{4-[(1-cyclobutyl-4-pipéridinyl)oxy]-1-pipéridinyl}-3-fluorobenzonitrile ;
le 4-{4-[(1-cyclobutyl-4-pipéridinyl)oxy]-1-pipéridinyl}-2-fluorobenzonitrile ;
la 1-(4-{4-[(1-cyclobutyl-4-pipéridinyl)oxy]-1-pipéridinyl}-2-fluorophényl)éthanone ;
le 4-{4-[(1-cyclobutyl-4-pipéridinyl)oxy]-1-pipéridinyl}-2,5-difluorobenzonitrile ;
la 1-(4-{4-[(1-cyclobutyl-4-pipéridinyl)oxy]-1-pipéridinyl}-2,5-difluorophényl)éthanone ;
la 1-[3-fluoro-4-(3-méthyl-1,2,4-oxadiazole-5-yl)phényl]-4-({1-[(1S)-1-méthylpropyl]-4-pipéridinyl}oxy)pipéridine ; ou
le N-méthyl-5-(4-{[1-(1-méthyléthyl)-4-pipéridinyl]oxy}-1-pipéridinyl)-2-pyridinecarboxamide ; ou un de ses sels pharmaceutiquement acceptables.

11. Composé suivant la revendication 1, qui est
la 1-(1-méthyléthyl)-4-({1-[4-(3-méthyl-1,2,4-oxadiazole-5-yl)phényl]-4-pipéridinyl}oxy)pipéridine ;
le 5-{4-[(1-cyclobutyl-4-pipéridinyl)oxy]-1-pipéridinyl}-*N*-méthyl-2-pyridinecarboxamide ;
la 1-(4-{4-[(1-cyclobutyl-4-pipéridinyl)oxy]-1-pipéridinyl}phényl)éthanone ;
la 3-{4-[(1-cyclobutyl-4-pipéridinyl)oxy]-1-pipéridinyl}-6-(trifluorométhyl)pyridazine ; ou
la 5-{4-[(1-cyclobutyl-4-pipéridinyl)oxy]-1-pipéridinyl}-2-(trifluorométhyl)pyrimidine ;
ou un de ses sels pharmaceutiquement acceptables.

12. Composition pharmaceutique qui comprend le composé de formule (I) suivant l'une quelconque des revendications 1 à 11, ou un de ses sels pharmaceutiquement acceptables et un support ou excipient pharmaceutiquement acceptable.

13. Composé suivant l'une quelconque des revendications 1 à 11, destiné à être utilisé en thérapie.

14. Composé suivant l'une quelconque des revendications 1 à 11, destiné à être utilisé dans le traitement de maladies neurologiques.

15. Utilisation d'un composé suivant l'une quelconque des revendications 1 à 11, dans la production d'un médicament destiné au traitement de maladies neurologiques.

16. Procédé pour la préparation d'un composé de formule (I) ou d'un de ses sels pharmaceutiquement acceptables, procédé qui comprend :
(a) la réaction d'un composé de formule (II) 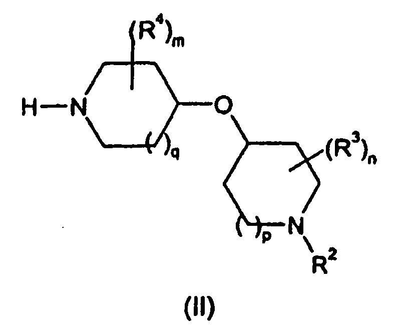 dans laquelle R², R³, R⁴, m, n, p et q sont tels que définis dans la revendication 1, avec un composé de formule R¹-L¹, dans laquelle R¹ est tel que défini dans la revendication 1 et L¹ représente un groupe partant convenable, tel qu'un atome d'halogène; ou
(b) la réaction de formule (III) 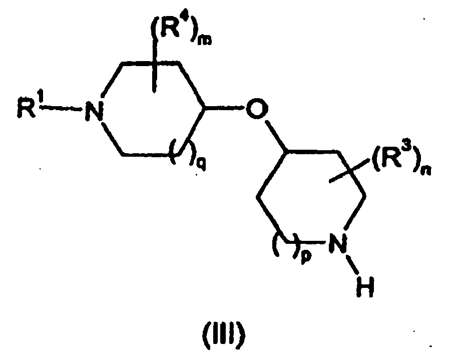 dans laquelle R¹, R³, R⁴, m, n, p et q sont tels que définis dans la revendication 1, avec un composé de formule R²-L² dans laquelle R² est tel que défini dans la revendication 1 et L² représente un groupe partant convenable, tel qu'un atome d'halogène ou un groupe sulfonate tel que le groupe méthanesulfonate ; ou
(c) la réaction d'un composé de formule (III) telle que définie ci-dessus, avec un composé de formule H-R^{2'}=O dans des conditions réductrices, formule dans laquelle R^{2'} est tel que défini dans la revendication 1 pour le groupe R² ou représente un groupe convertible en celui-ci ; ou
(d) la préparation d'un composé de formule (I) dans laquelle p est égal à 1, qui comprend la réduction d'un composé de formule (IV) 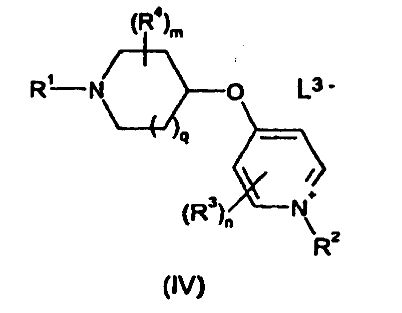 dans laquelle R¹, R², R³, R⁴, m, n et q sont tels que définis dans la revendication 1 et L³⁻ représente un ion complémentaire convenable tel qu'un atome d'halogène ; ou
(e) la suppression de la protection d'un composé de formule (I) ou la conversion de groupes qui sont protégés ; puis, facultativement
(f) l'interconversion en d'autres composés de formule (I).
